# EUROPEAN PATENT SPECIFICATION

(11) **EP 3 102 221 B1**
(45) Date of publication and mention of the grant of the patent: **29.01.2020**
(21) Application number: 15746473.6
(22) Date of filing: 06.02.2015
(51) Int. Cl.: A61K 38/00, C12P 19/34

(54) **OLIGOMERIZATION DOMAIN OF P53 TO BYPASS THE DOMINANT-NEGATIVE EFFECT OF MUTANT**
OLIGOMERISATIONSDOMÄNE VON P53 ZUR UMGEHUNG DES DOMINANT-NEGATIVEN EFFEKTS VON MUTANTEN
DOMAINE D'OLIGOMÉRISATION DE P53 SERVANT À CONTOURNER L'EFFET DOMINANT NÉGATIF D'UN MUTANT

(30) Priority: 06.02.2014 US 201461936790 P; 13.05.2014 US 201461992678 P
(43) Date of publication of application: 14.12.2016
(73) Proprietor: University of Utah Research Foundation, Salt Lake City, UT 84108 (US)
(72) Inventor: LIM, Carol, Salt Lake City, UT 84124 (US); OKAL, Abood, Kaysville, UT 84037 (US)
(74) Representative: CH Kilger Anwaltspartnerschaft mbB
(86) International application number: PCT/US2015/014855
(87) International publication number: WO 2015/120298

(56) References cited:
- WO-A2-03/006486
- US-A1- 2004 022 772
- US-A1- 2011 212 898
- WATERMAN M J F ET AL: "AN ENGINEERED FOUR-STRANDED COILED COIL SUBSTITUTES FOR THE TETRAMERIZATION DOMAIN OF WILD-TYPE P53 AND ALLEVIATES TRANSDOMINANT INHIBITION BY TUMOR-DERIVED P53 MUTANTS", CANCER RESEARCH, AMERICAN ASSOCIATION FOR CANCER RESEARCH, US, vol. 56, no. 1, 1 January 1996 (1996-01-01), pages 158-163, XP000608177, ISSN: 0008-5472
- ABOOD OKAL ET AL: "Re-Engineered p53 Chimera with Enhanced Homo-Oligomerization That Maintains Tumor Suppressor Activity", MOLECULAR PHARMACEUTICS, vol. 11, no. 7, 7 July 2014 (2014-07-07), pages 2442-2452, XP055384907, US ISSN: 1543-8384, DOI: 10.1021/mp500202p
- A OKAL ET AL: "Re-engineered p53 activates apoptosis in vivo and causes primary tumor regression in a dominant negative breast cancer xenograft model", GENE THERAPY, vol. 21, no. 10, 31 July 2014 (2014-07-31) , pages 903-912, XP055384909, GB ISSN: 0969-7128, DOI: 10.1038/gt.2014.70
- Abood, Okal: "BYPASSING TRANSDOMINANT INHIBITION WITHCHIMERIC p53 FOR CANCER GENE THERAPY", 7 June 2017 (2017-06-07), A dissertation submitted to the faculty ofThe University of Utahin partial fulfillment of the requirements for the degree of Doctor of Philosophy, Department of Pharmaceutics and Pharmaceutical ChemistryThe University of Utah, XP002771375, * Chapers 3-5 *
- OKAL, A ET AL.: 'A Chimeric p53 Evades Mutant p53 Transdominant Inhibition In Cancer Cells.' MOLECULAR PHARMACEUTICALS. vol. 10, no. 10, 07 October 2013, pages 3922 - 3933, XP055219564
- DIXON, A ET AL.: 'Disruption Of Bcr-Abl Coiled Coil Oligomerization By Design.' JOUMAL OF BIOLOGICAL CHEMISTRY. vol. 286, no. 31, 04 August 2011, pages 27751 - 27760, XP055219566
- DATABASE GENBANK [Online] 'Human mRNA For p53 Cellular Tumor Antigen: Human mRNA For p53 Cellular Tumor Antigen', XP055219569 Database accession no. X02469.1 & EMBO J., [Online] vol. 4, no. 5, 12 September 1993, pages 1251 - 1255 Retrieved from the Internet: <URL:http://www.ncbi.nlm.nih.gov/nucleotide /35209?report=genbank&log $=nuclalign&blast_rank=9&RID=KK07X4XT01R> [retrieved on 2015-04-23]

## Description

### CROSS-REFERENCE TO RELATED APPLICATIONS

This application claims benefit of U.S. Provisional Application No. 61/992,678, filed May 13, 2014, and U.S. Provisional Application No. 61/936,790, filed February 6, 2014.

### STATEMENT REGARDING FEDERALLY SPONSORED RESEARCH

This invention was made with government support under RO 1-CA 15184 7 awarded by the National Institutes of Health. The government has certain rights in the invention.

### BACKGROUND

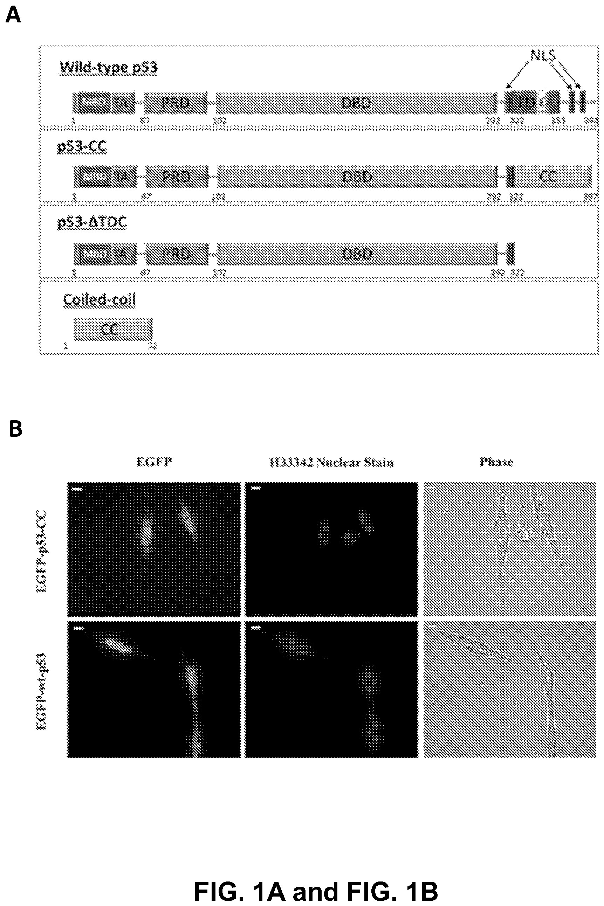

The ability ofp53 to achieve tumor suppressor function depends on formation of p53 tetramers to act as a transcription factor of several target genes. Once activated, p53 stimulates a wide network of signals including DNA repair, cell cycle arrest, and apoptosis. The significance of p53 function is highlighted by the correlation of its inactivity and malignant development. Inactivation ofp53 pathway is reported in more than half of all human tumors and can be achieved via several mechanisms including nuclear exclusion and hyperactivation of MDM2, the main regulator ofp53 function. However, acquisition of missense mutations in one or both alleles of the TP53 gene remains the most common mechanism ofp53 inactivation. The majority of these mutations take place in the DNA binding domain (DBD) which is responsible for p53 interaction with DNA. Although mutant p53 in cancer cells may have impaired tumor suppressor function and transcriptional activity, it retains its ability to oligomerize with other mutant or wild-type (wt) p53 via the tetramerization domain (TD). When mutant p53 oligomerizes with wt-p53 through hetero-oligomerization, the resulting tetramer has impaired function in most cases due to transdominant inhibition by mutant p53 (Figure 1). The outcome of this transdominant inhibition varies significantly based on the type of mutant p53 present in cells (Peng, Human gene therapy 16, 1016-1027 (2005)). This phenomenon is known as the dominant negative effect of mutant p53 and gives rise to a critical barrier to utilizing wt-p53 for cancer gene therapy.

### BRIEF SUMMARY

Disclosed are peptides comprising a partial p53 peptide and a mutated Bcr coiled-coil domain, wherein the partial p53 peptide does not have a functional tetramerization domain but retains tumor suppressor activity and the mutated Bcr coiled-coil domain comprises amino acid substitutions and deletions but retains tetramerization activity, wherein the mutated Bcr coiled-coil domain comprises mutations at residues 34 and 55 of SEQ ID NO:4 and has a lysine at position 34 and a glutamic acid at position 55 of SEQ ID NO:4 or comprises the sequence of SEQ ID NO:5, wherein the mutated Bcr coiled-coil domains comprise at least 50% sequence identity to SEQ ID NO:4.

Disclosed are peptides comprising a partial p53 peptide and a mutated Bcr coiled-coil domain, wherein the mutated Bcr coiled-coil domain is linked to the C-terminus of the DNA binding domain of the partial p53 peptide. The mutated Bcr coiled-coil domain can be located between the DNA binding domain of the partial p53 peptide and the C-terminus of the peptide.

Also disclosed are nucleic acid sequences capable of encoding the peptides disclosed herein.

Disclosed are nucleic acid sequences capable of encoding a peptide comprising a partial p53 peptide and a mutated Bcr coiled-coil domain, wherein the mutated Bcr coiled-coil domain is linked to the C-terminus of the DNA binding domain of a partial p53 peptide.

Disclosed are nucleic acid sequences capable of encoding a peptide comprising a partial p53 peptide and a mutated Bcr coiled-coil domain, wherein the mutated Bcr coiled-coil domain is located between the DNA binding domain of the partial p53 peptide and the C-terminus of the peptide.

Disclosed are nucleic acid sequences capable of encoding a peptide comprising a partial p53 peptide and a mutated Bcr coiled-coil domain, wherein the mutated Bcr coiled-coil domain comprises mutations at residues 34 and 55 of SEQ ID N0:4.

Disclosed are nucleic acid sequences capable of encoding a peptide comprising a partial p53 peptide and a mutated Bcr coiled-coil domain, wherein the mutated Bcr coiled-coil domain comprises the sequence of SEQ ID N0:5, wherein the mutated Bcr coiled-coil domains comprise at least 50% sequence identity to SEQ ID NO:4.

Also disclosed are vectors comprising the nucleic acid sequences disclosed herein.

Disclosed are vectors comprising a nucleic acid sequence, wherein the nucleic acid sequence is capable of encoding a peptide comprising a partial p53 peptide and a mutated Bcr coiled-coil domain, wherein the mutated Bcr coiled-coil domain is linked to the C-terminus of the DNA binding domain of the partial p53 peptide.

Disclosed are vectors comprising a nucleic acid sequence, wherein the nucleic acid sequence is capable of encoding a peptide comprising a partial p53 peptide and a mutated Bcr coiled-coil domain, wherein the mutated Bcr coiled-coil domain is located between the DNA binding domain of the partial p53 peptide and the C-terminus of the peptide.

Disclosed are vectors comprising a nucleic acid sequence, wherein the nucleic acid sequence is capable of encoding a peptide comprising a partial p53 peptide and a mutated Bcr coiled-coil domain, wherein the mutated Bcr coiled-coil domain comprises mutations at residues 34 and 55 of SEQ ID N0:4.

Disclosed are vectors comprising a nucleic acid sequence, wherein the nucleic acid sequence is capable of encoding a peptide comprising a partial p53 peptide and a mutated Bcr coiled-coil domain, wherein the mutated Bcr coiled-coil domain consists of the sequence of SEQ ID N0:5, or active fragments thereof.

Disclosed are vectors comprising a nucleic acid sequence, wherein the nucleic acid sequence is capable of encoding a peptide comprising a partial p53 peptide and a mutated Bcr coiled-coil domain, wherein the vector is a viral vector. The viral vector can be an adenoviral vector.

Also disclosed are compositions comprising a peptide comprising a partial p53 peptide and a mutated Bcr coiled-coil domain for use in inducing apoptosis in the treatment of a patient [0034] Disclosed are compositions comprising a peptide comprising a partial p53 peptide and a mutated Bcr coiled-coil domain for use in inducing apoptosis in the treatment of a patient wherein the mutated Bcr coiled-coil domain is linked to the C-terminus of the DNA binding domain of the partial p53 peptide.

Disclosed are compositions comprising a peptide comprising a partial p53 peptide and a mutated Bcr coiled-coil domain for use in inducing apoptosis in the treatment of a patient, wherein the mutated Bcr coiled-coil domain is located between the DNA binding domain of the partial p53 peptide and the C-terminus of the peptide.

Disclosed are compositions comprising a peptide comprising a partial p53 peptide and a mutated Bcr coiled-coil domain for use in inducing apoptosis in the treatment of a patient, wherein the mutated Bcr coiled-coil domain comprises the sequence of SEQ ID N0:5, wherein the mutated Bcr coiled-coil domains comprise at least 50% sequence identity to SEQ ID NO:4.

Also disclosed are compositions comprising a peptide comprising a partial p53 peptide and a mutated Bcr coiled-coil domain for use in suppressing a tumor in a patient
[wherein the mutated Bcr coiled-coil domain is linked to the C-terminus of the DNA binding domain of the partial p53 peptide or
wherein the mutated Bcr coiled-coil domain is located between the DNA binding domain of the partial p53 peptide and the C-terminus of the peptide.

Disclosed are compositions comprising a peptide comprising a partial p53 peptide and a mutated Bcr coiled-coil domain for use in suppressing a tumor in a patient wherein the peptide comprises a partial p53 peptide and a mutated Bcr coiled-coil domain, wherein the mutated Bcr coiled-coil comprises the sequence of SEQ ID N0:5, or active fragments thereof or wherein the peptide comprises the sequence of SEQ ID N0:3.

Disclosed are compositions comprising a peptide comprising a partial p53 peptide and a mutated Bcr coiled-coil domain for use in inducing apoptosis in the treatment of a patient or for use in suppressing a tumor in a patient, further comprising a pharmaceutically acceptable carrier..

Disclosed is a peptidewherein the peptide comprises a partial p53 peptide and a mutated Bcr coiled-coil domain for use in the treatment of cancer, wherein the cancer comprises breast cancer, triple negative breast cancer, ovarian cancer or any blood cancer.

Disclosed is a peptide for treating a hyperproliferative disorder, wherein the peptide comprises a partial p53 peptide and a mutated Bcr coiled-coil domain, wherein the mutated Bcr coiled-coil domain is linked to the C-terminus of the DNA binding domain of the partial p53 peptide.

Disclosed is a peptide for treating a hyperproliferative disorder, wherein the peptide comprises a partial p53 peptide and a mutated Bcr coiled-coil domain, wherein the mutated Bcr coiled-coil domain is located between the DNA binding domain of the partial p53 peptide and the C-terminus of the peptide.

Disclosed is a peptide for treating a hyperproliferative disorder, wherein the peptide comprises a partial p53 peptide and a mutated Bcr coiled-coil domain, wherein the mutated Bcr coiled-coil domain comprises the sequence of SEQ ID N0:5, or active fragments thereof, or comprises the sequence of SEQ ID N0:3.

Disclosed is a peptide for treating a hyperproliferative disorder in a patient , wherein the peptide comprises a partial p53 peptide and a mutated Bcr coiled-coil domain, wherein the hyperproliferative disorder comprises cancer, wherein cancer comprises breast cancer, triple negative breast cancer, ovarian cancer, or any blood cancer.

Disclosed is a peptide for treating cancer wherein the peptide comprises a partial p53 peptide and a mutated Bcr coiled-coil domain, wherein the mutated Bcr coiled-coil domain is linked to the C-terminus of the DNA binding domain of the partial p53 peptide.

Disclosed is a peptide for treating cancer wherein the peptide comprises a partial p53 peptide and a mutated Bcr coiled-coil domain, wherein the mutated coiled-coil domain is located between the DNA binding domain of the partial p53 peptide and the C-terminus of the peptide.

Disclosed is a peptide for treating cancer wherein the peptide comprises a partial p53 peptide and a mutated Bcr coiled-coil domain, wherein the mutated Bcr coiled-coil domain comprises the sequence of SEQ ID N0:5, or wherein the peptide comprises the sequence of SEQ ID N0:3.

Also disclosed are recombinant cells comprising the nucleic acid sequences described herein. The recombinant cells can comprise nucleic acid sequences capable of producing any of the peptides described herein.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

Figures 1A and 1B show p53 constructs and nuclear localization of the constructs. (A) Schematic representation of the experimental constructs and controls. Full length p53 (wt-p53) contains a MDM2 binding domain (MBD), a transactivation domain (TA) in the amino terminus, a proline-rich domain (PRD), a DNA binding domain (DBD), a strong nuclear localization signal (NLS), a tetramerization domain (TD) that also contains a nuclear export signal (E), and a carboxy terminus (C-terminus) that includes two weak NLSs. For p53-CC, the TD and C-terminus were replaced by the coiled-coil (CC) from Bcr. p53-ΔTDC lacks both the TD and the C-terminus. All constructs were fused to EGFP on the N-terminus (not shown in diagram). (B) Representative fluorescence microscopy images of 1471.1 cells confirm exclusive nuclear accumulation ofEGFP-p53-CC similar to EGFP-wt-p53. EGFP Fluorescence, nuclear staining with H33342, and phase contrast images are shown, left to right. 1471.1 breast cancer cells were chosen for this study due to their optimal microscopy characteristics (elongated morphology and distinguishable subcellular compartments) 4. White scale bars on top left corners are 10 11m.

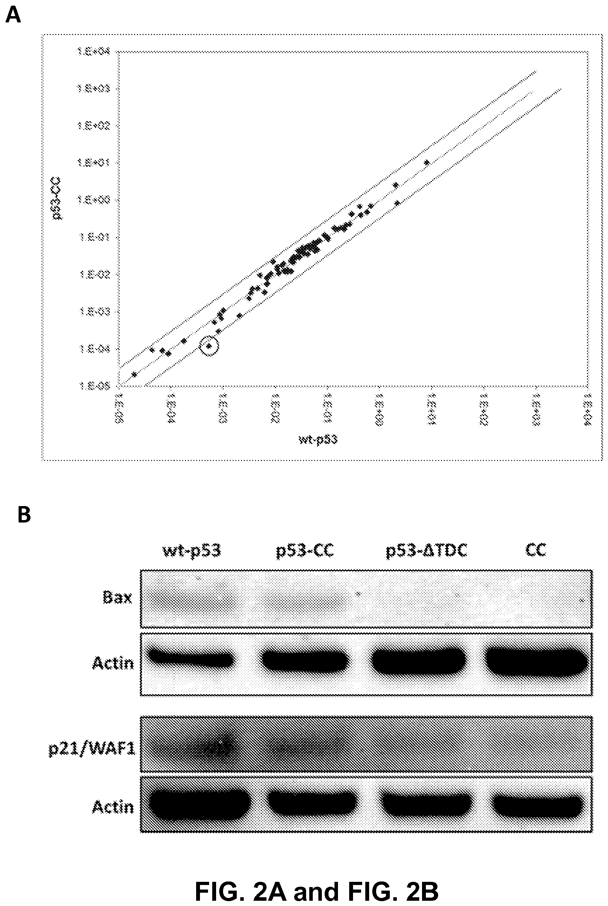

Figures 2A and 2B show p53-CC is capable of transactivating several p53 target genes. (A) Scatter plot representation of mRNA levels of 84 p53 target genes in T47D cells transfected with wt-p53 or p53-CC. Each dot represents one of the 84 genes assayed in this PCR array. The two magenta lines represent a boundary of two fold upregulation or downregulation in mRNA levels. Cells treated with wt-p53 or p53-CC showed similar levels of mRNA for all 84 genes except for one, p53AIP1, which is circled on the scatter plot. (B) Representative cropped western blots of T47D cell lysates 24 h post transfection with wt-p53, p53-CC, p53-ΔTDC, or CC. Similar levels of Bax and p21/WAF1 protein expression were detected from cells treated with wt-p53 or p53-CC.

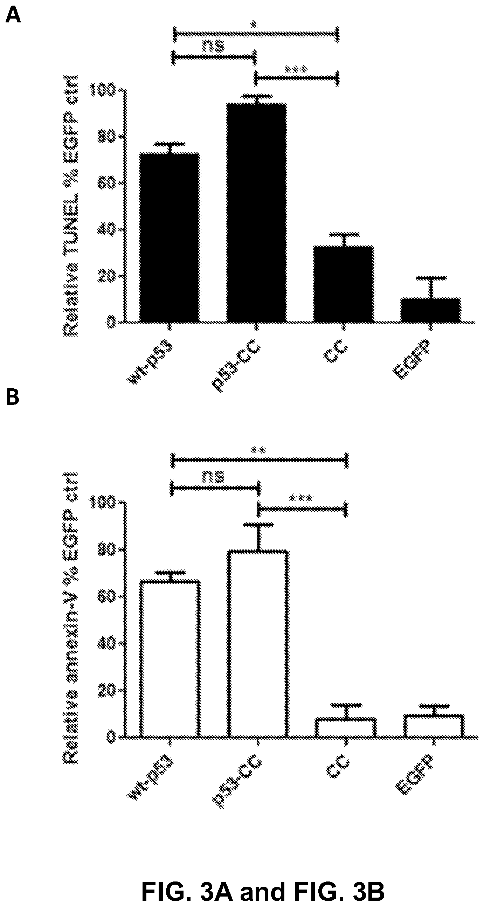

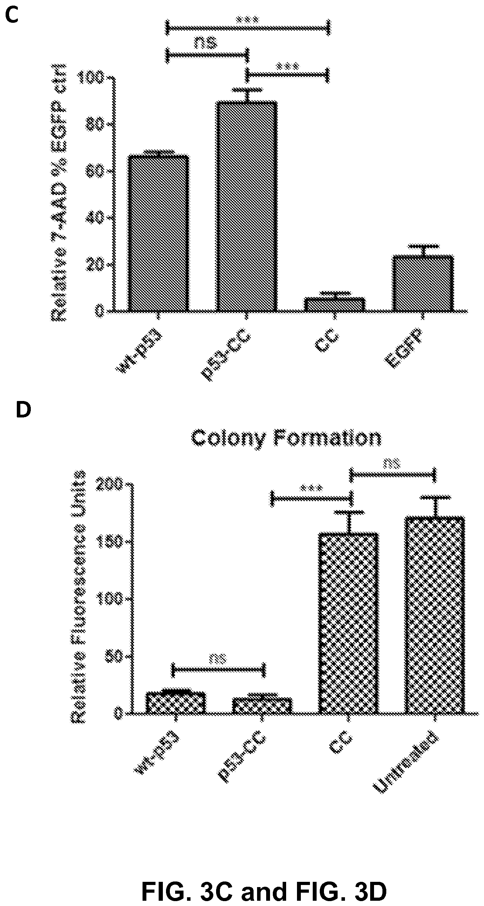

Figures 3A, 3B, 3C and 3D show apoptotic and cell proliferation assays performed in T47D cells 48 h after transfection. (A) TUNEL assay shows similar apoptotic activity ofp53-CC compared to wt-p53. Both p53-CC and wt-p53 demonstrate a significantly higher activity compared to CC negative control. Similar results were obtained from (B) annexin V staining and (C) 7-AAD staining. (D) The colony forming assay shows the transformative ability of T47D cells post treatment with wt-p53, p53-CC, and CC. Cells treated with wt-p53 and p53-CC show significant reduction in transformative ability (oncogenic potential) of T47D cells compared to untreated cells or cells treated with CC. Mean values were analyzed using one-way ANOV A with Bonferroni's post test; * p < 0.05, ** p < 0.01, and *** p < 0.001. Error bars represent standard deviations from at least three independent experiments (n=3).

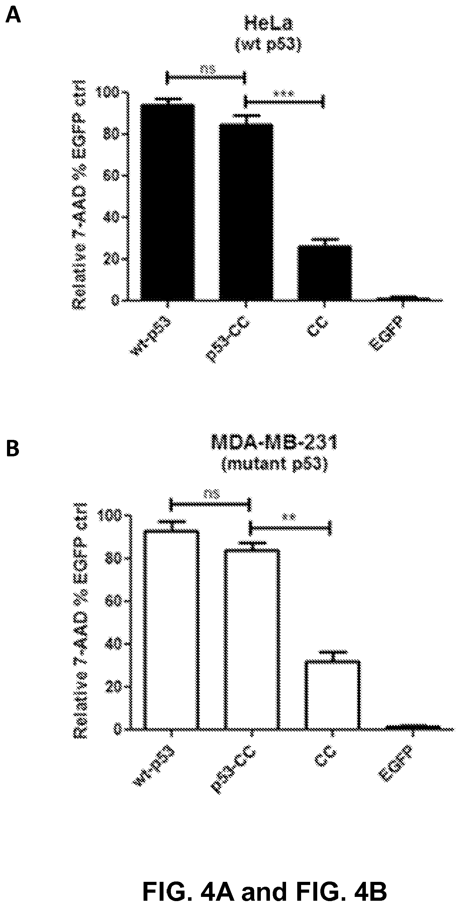

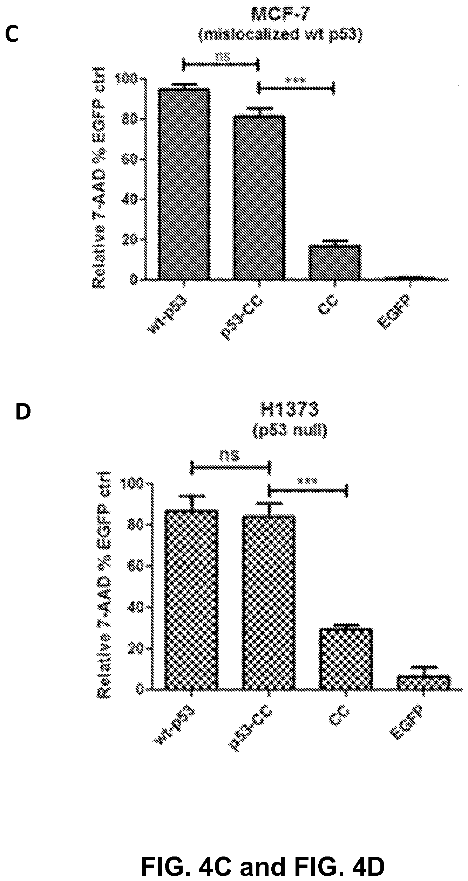

Figures 4A, 4B, 4C and 4D show 7-AAD assay conducted in four different cell lines with varying p53 status (A) Hela, (B) MDA-MB-231, (C) MCF-7, and (D) H1373. In all four cases, p53-CC is capable of inducing cell death in a similar fashion compared to wt-p53, regardless of the endogenous p53 status or the cancer cell line used. Statistical analysis was performed using one-way ANOVA with Bonferroni's post test; ** p < 0.01 and *** p < 0.001.

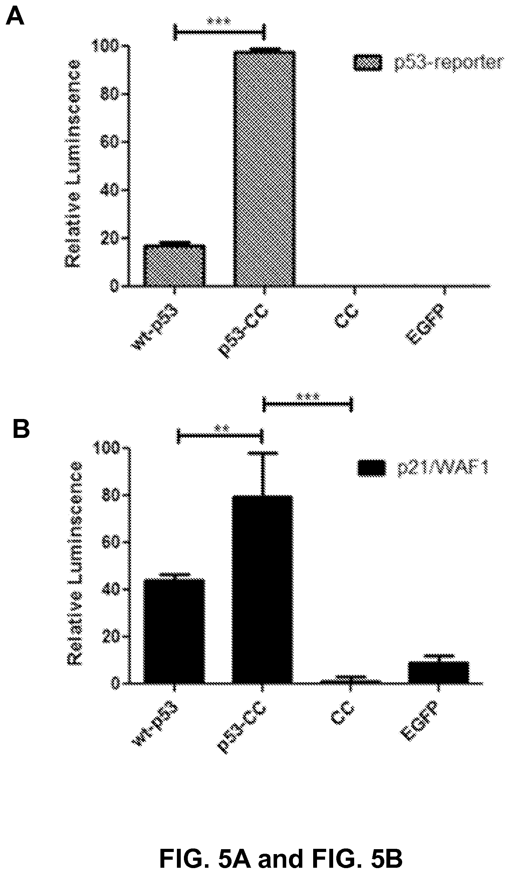

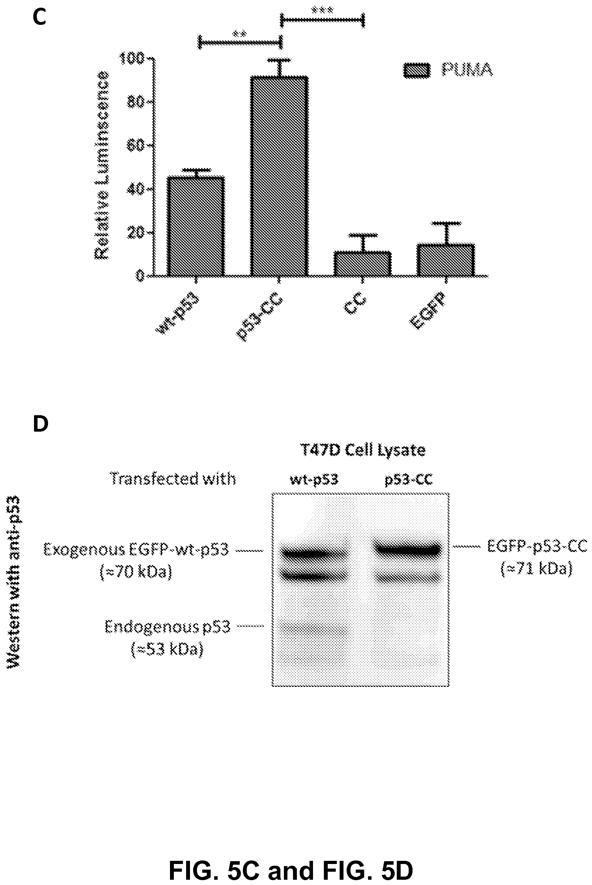

Figures 5A, 5B, and 5C show relative luminescence representing the activation of (A) the p53-cis reporter, (B) the p21/WAF1 reporter, and (C) the PUMA reporter in T47D cells. The ability ofp53-CC to transactivate these promoters is higher than wt-p53. In all three cases, 3.5 11g of construct (wt-p53, p53-CC, CC, or EGFP) was co-transfected with 0.35 11g of pRL-SV40 plasmid encoding for Renilla luciferase to normalize for transfection efficiency. In addition to Renilla luciferase, constructs were co-transfected with 3.5 11g ofp53-Luc Cis-Reporter, p21/WAF1 reporter, or PUMA reporter encoding for firefly luciferase. Mean values were analyzed using one-way ANOVA with Bonferroni's post test; ** p < 0.01, and *** p < 0.001. Error bars represent standard deviations from three independent experiments (n=3). (D) Interaction of endogenous p53 with exogenous wt-p53 or p53-CC was investigated in T47D via co-IP. A representative cropped western blot of protein complexes co-immunoprecipitated using anti-GFP antibody is shown. Left lane, endogenous p53 (53 kDa) co-immunoprecipitates with exogenous EGFP-wt-p53 (70 kDa). Right lane, endogenous p53 fails to co-immunoprecipitate with exogenous EGFP-p53-CC (71 kDa).

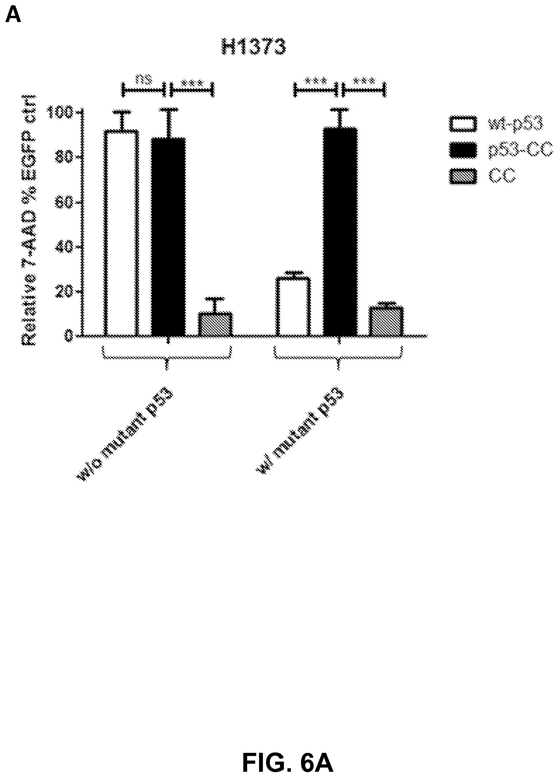

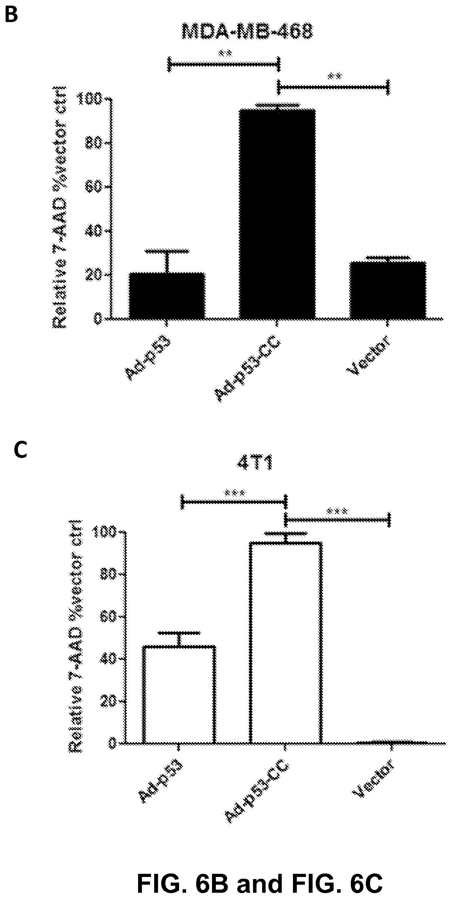

Figures 6A, 6B and 6C show p53-CC circumvents transdominant inhibition by mutant p53. (A) Overexpression of mutant p53 reduces the activity of exogenous wt-p53 but has no influence on exogenous p53-CC activity. H1373 cells were chosen for this experiment since they are p53 null and hence there will be no additional p53 activity from the cells due to lack of endogenous p53. (B) 7-AAD assay was conducted 48 h post transducing MDA-MB-468 cells, which harbor a potent transdominant mutant p53 (R273H), with adenoviral vectors expressing either wt-p53 or p53-CC with a multiplicity of infection (MOI) of 200. As expected, exogenous wt-p53 (Ad-p53) activity is limited in this cell line due to the presence of endogenous transdominant tumor derived p53. (C) 7-AAD assay was also performed 48 h post transducing 4TI cells (MOI 250). Interestingly, p53-CC is more active than wt-p53 in this particular cell line. The adenoviral vector alone was used as a negative control. Mean values were analyzed using one-way ANOVA with Bonferroni's post test; **< p 0.01, and *** p < 0.001. Error bars represent standard deviations from three independent experiments (n=3).

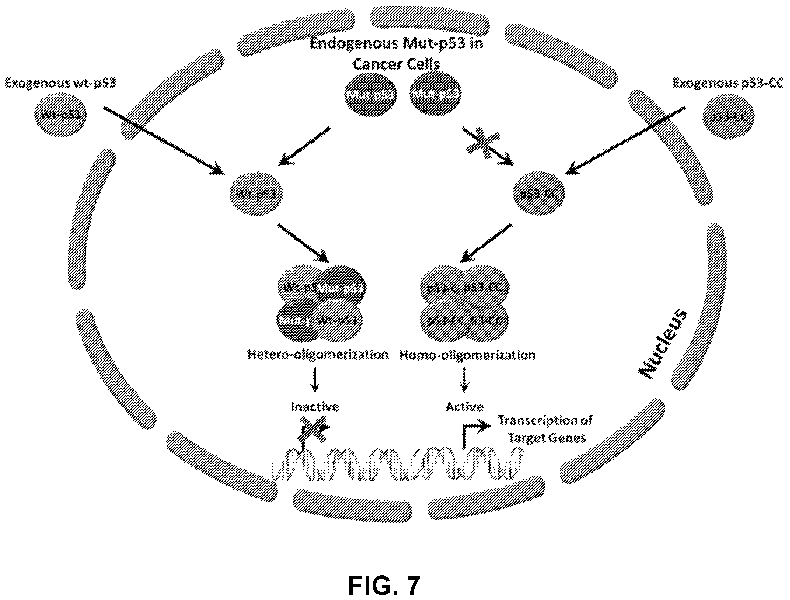

Figure 7 is a schematic of the proposed mechanism ofp53-CC activity. Left side of figure: exogenously added wt-p53 can still form hetero-tetramers with mutant p53 due to the presence of the TD, and becomes inactivated. Right side of figure: p53-CC can bypass transdominant inhibition by mutant p53 in cancer cells, and still exhibit tumor suppressor activity.

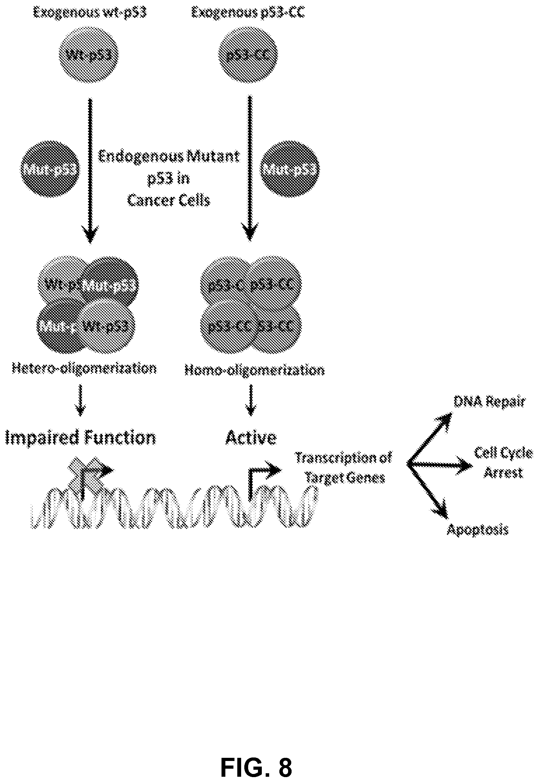

Figure 8 is a schematic representation of the fates ofwt-p53 (left) and p53-CC (right) in the presence of endogenous mutant p53 in cancer cells. Wt-p53 (left) is sequestered into hetero-oligomers that have an impaired transcription function, while p53-CC (right) can exclusively form homo-oligomers that retain full tumor suppressor activity.

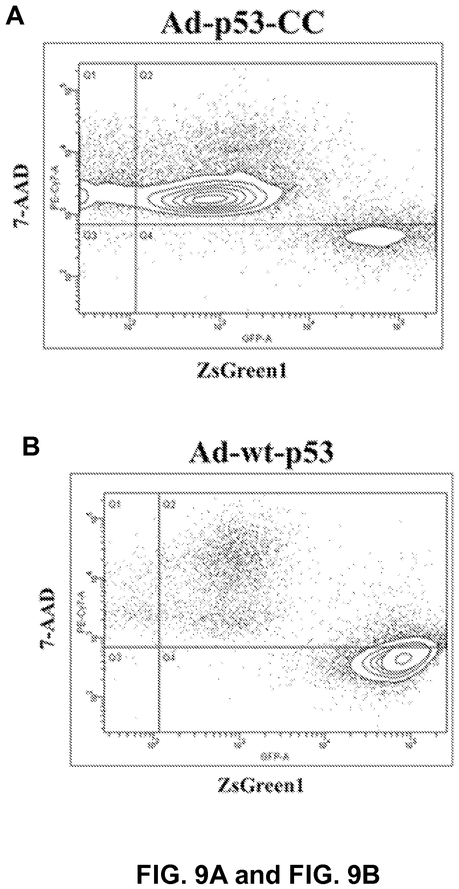

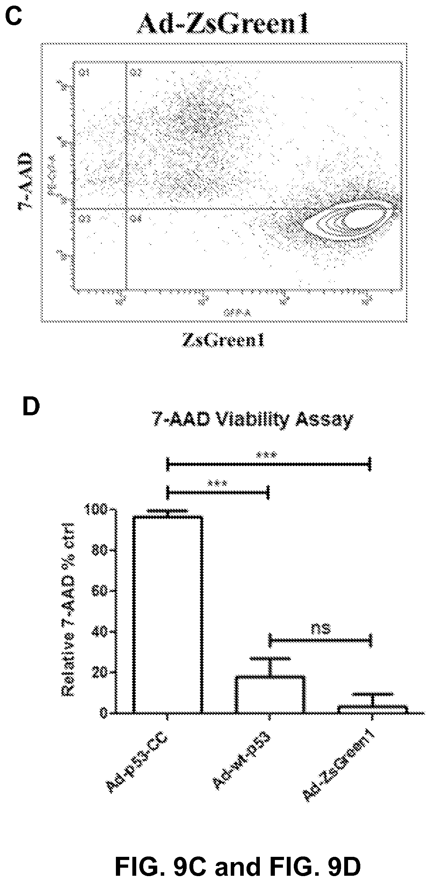

Figures 9A, 9B, 9C, and 9D show 7-AAD staining of apoptotic and necrotic samples. 48 h after viral transfection, cells were analyzed and gated for ZsGreenl. (A-C) Representative individual contour plots from each transfection and treatment group showing only ZsGreenlgated cells. QI&Q2= 7-AAD positive cells; Q3&Q4= 7-AAD negative cells. (D) Percentage of cell death induced by each transfection and treatment group. Mean values were analyzed using one-way ANOVA with Bonferroni's post test; ns= non-significant, *** p < 0.001. Error bars represent standard deviations from at least three independent experiments (n = 3).

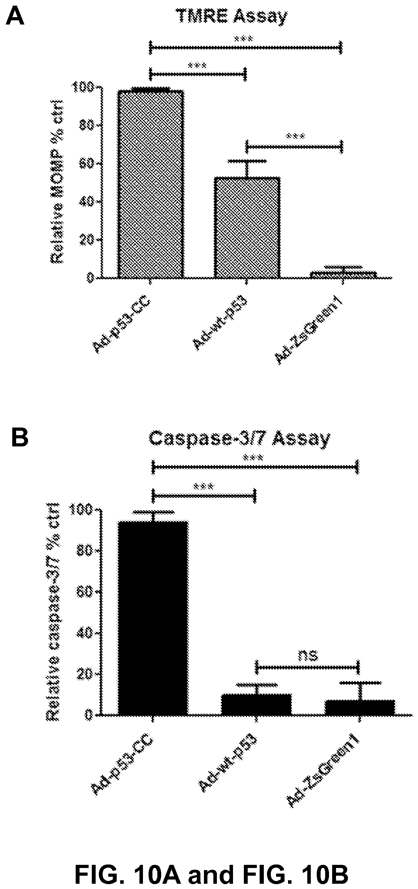

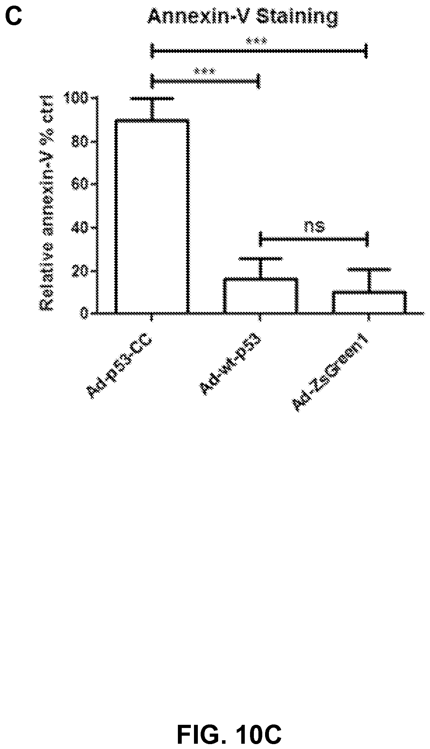

Figures 10A, 10B, and 10C show the induction of apoptosis is measured by (A) TMRE, (B) Caspas-317, and (C) Annexin-V. In all three assays, MDA-MB-468 cells trated with Ad-p53-CC undergo higher levels of apoptosis compared to cells infected with Ad-wt-p53 or the negative control Ad-ZsGreenl. Mean values were analyzed using one-way ANOVA with Bonferroni's post test; ns= non-significant, *** p < 0.001. Error bars represent standard deviations from at least three independent experiments (n = 3).

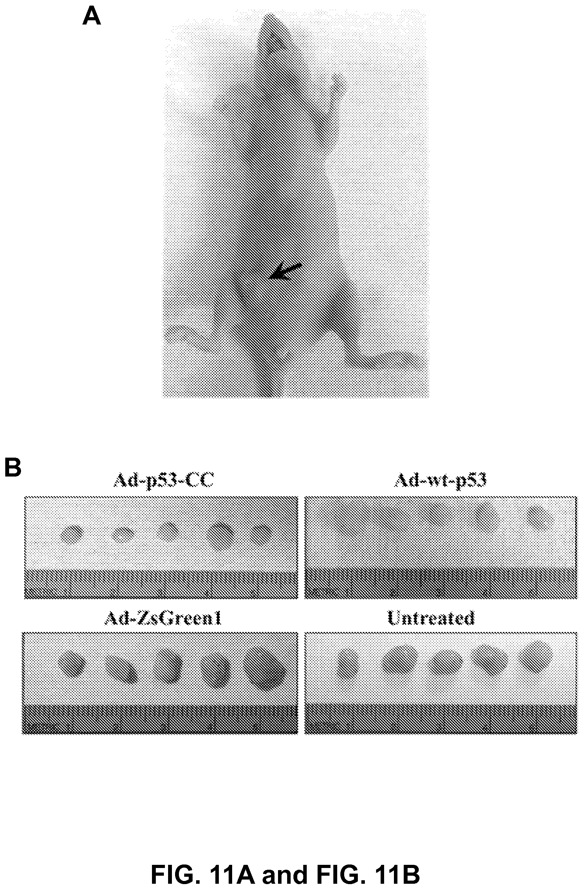

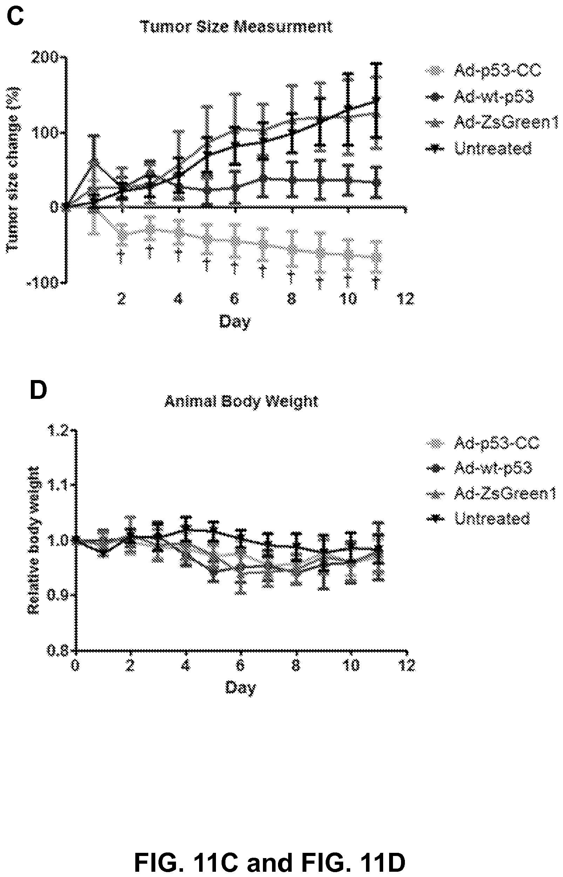

Figures 11A, 11B, 11C, and 11D show the effects of viral gene therapy using p53-CC and wt-p53 on induced the aggressive p53-dominant negative MDA-MB-468 human breast adenocarcinoma in female athymic nu/nu mice. (A) A representative image of a mouse in the study. For tumor inductions, MDA-MB-468 cells were injected in the right mammary fat pad of the inguinal area (highlighted by the black arrow). (B) Representative images of the excised tumors from each treatment group scaled to the same ratios. (C) Tumor size measured with calipers daily and normalized to Day 0. (D) Animal weights as measured daily and normalized relative to weights from Day 0. Six mice per group were used for this study. Mean values were analyzed using one-way ANOVA with Bonferroni's post test; tP < 0.001. Error bars represent standard deviations.

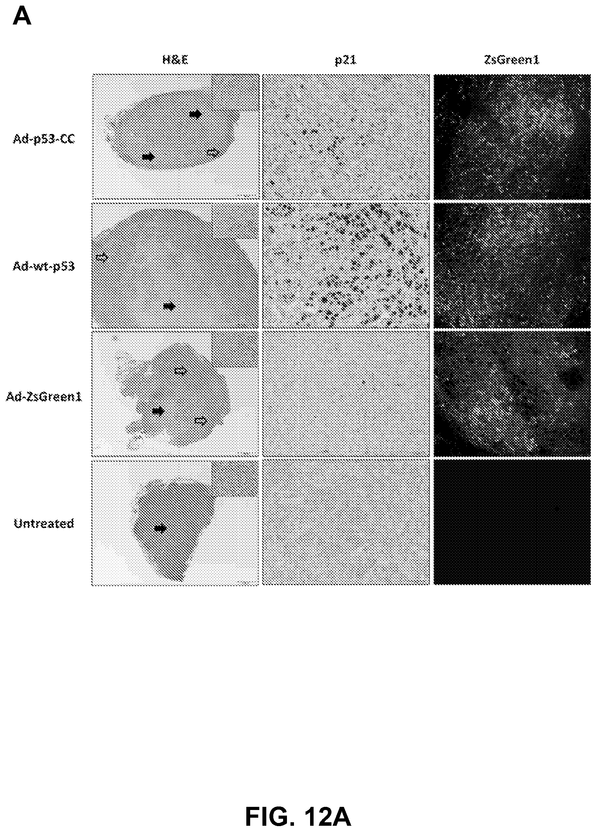

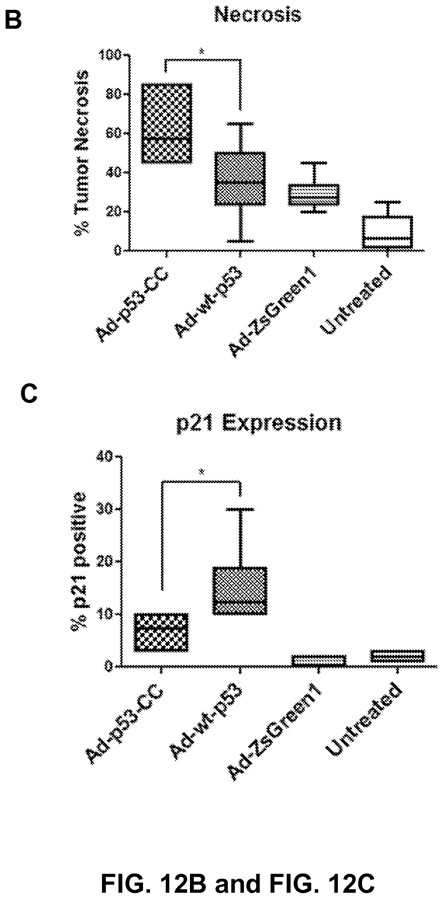

Figures 12A, 12B and 12C show representative photomicrographs showing the effects of the different treatment groups on tumor tissues visualized via H&E staining (A) left column, p21 immunohistochemistry staining (A) middle column, and ZsGreen1 fluorescence (A) right column. (A)Solid black arrows (left column) indicate necrotic cells, while open arrows indicate non-necrotic areas. 3,3' diaminobenzidine (DAPI) stains the nuclei ofp21-positive cells brown (middle column). Examination of the H&E staining microscopically revealed higher levels of necrosis in all tumor tissues from mice injected with Ad-p53-CC compared to the Adwt-p53, Ad-ZsGreen1, or untreated groups. p21 immunohistochemistry staining revealed higher levels ofp21 induction in the Ad-wt-p53 treatment group compared to the Ad-p53-CC treatment group. Semi-quantitative histoscore analyses of (B) tumor necrosis and (C) p21 up-regulation in the excised tumors from all groups is shown. Mean values were analyzed using one-way ANOVA with Bonferroni's post test; * p < 0.05. Error bars represent standard deviations (n=6).

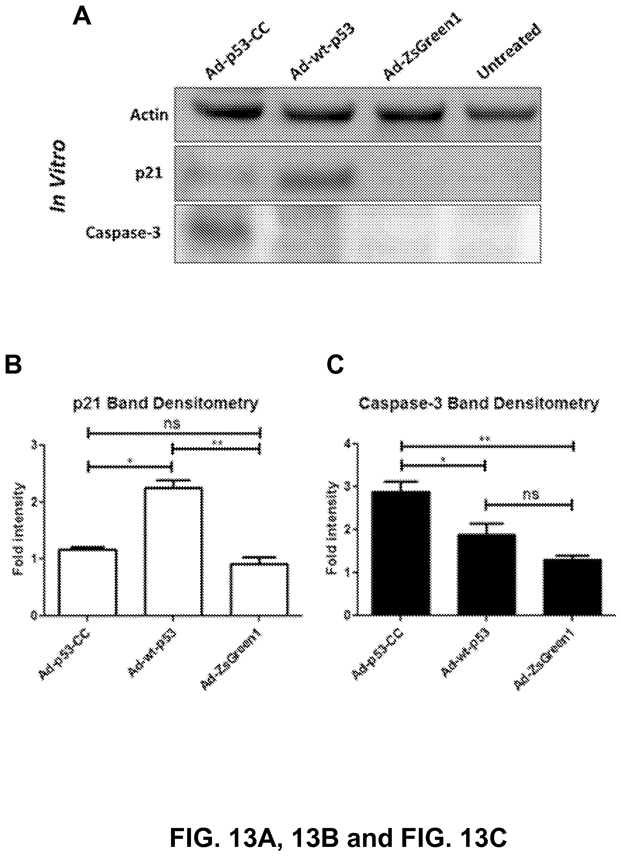

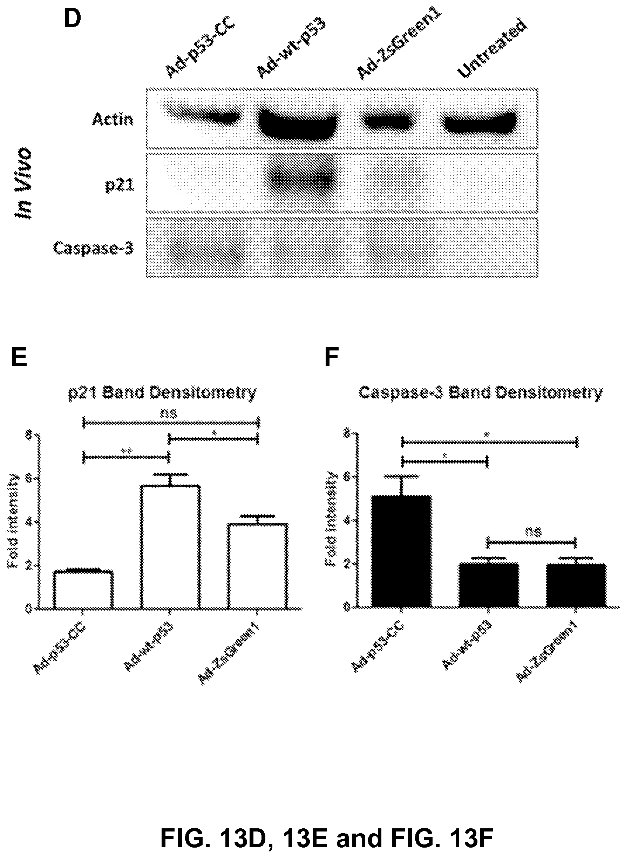

Figures 13A, 13B, 13C, 13D, 13E and 13F show representative cropped western blots ofMDA-MB-468 (A-C) in vitro cell lysates and (D-F) homogenized tumors from the in vivo study treated with Ad-p53-CC, Ad-wt-p53, Ad-ZsGreen1, or untreated. Western analyses show that MDA-MB-468 cells (A) and tumor tissues (D) treated with Ad-p53-CC both express lower levels of p21, but higher levels of activated caspase-3 compared to cells treated with Ad-wt-p53. No significant levels of p21 or caspase-3 induction were observed in cells (A) or tumors (D) injected with Ad-ZsGreen1 or untreated. Semi-quantitative densitometric analyses was carried out as described before47 to evaluate p21 (B) and caspase-3 (C) expression in vitro as well as expression ofp21 (E) and caspase-3 (F) expression in vivo. Mean values were analyzed using one-way ANOVA with Bonferroni's post test; * p < 0.05, ** p < 0.01, and *** p < 0.001. Error bars represent standard deviations (n=3).

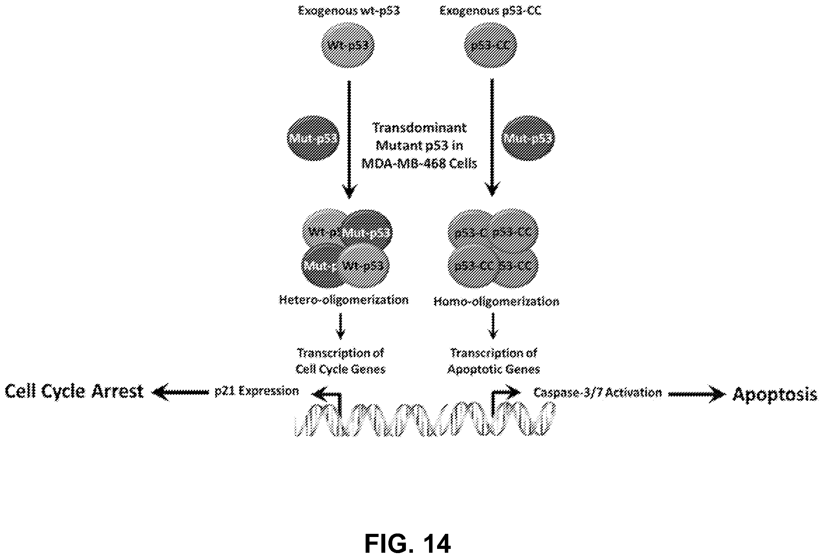

Figure 14 shows a schematic representation of the outcomes ofwt-p53 (left) and p53-CC (right) activation. Wt-p53 induces cell cycle arrest via p21 expression (left), while p53-CC induces cell death via the apoptotic pathway (right).

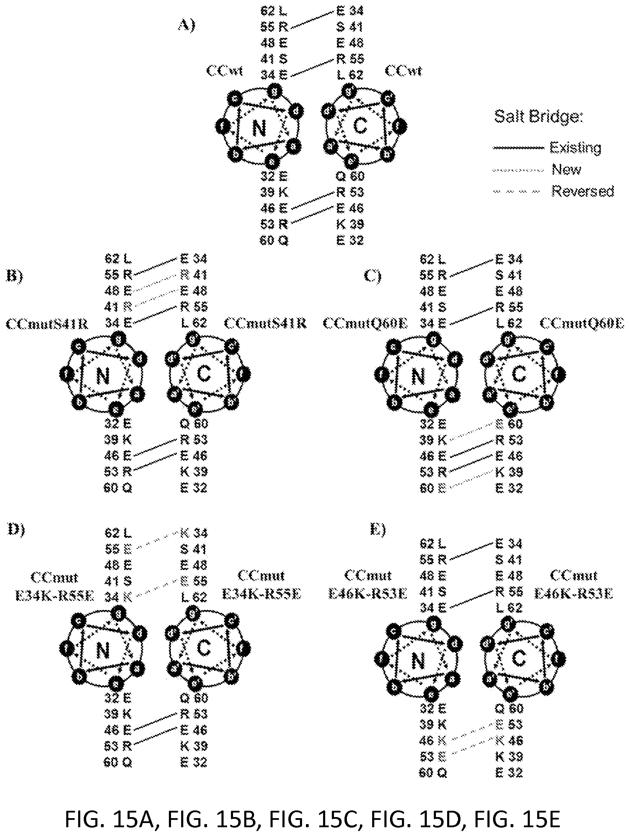

Figures 15A, 15B, 15C, 15D, and 15E show helical wheel diagrams of wild-type CC homo-dimers (CCwt) (A), CCmutS41R homo-dimers (B), CCmutQ60E homo-dimers (C), CCmutE34K-R55E homo-dimers (D), and CCmutE46K-R53E homo-dimers (E). Solid lines indicate possible ionic interactions already existing in the wild-type coiled-coil. Dotted lines represent newly formed ionic interactions. Dashed lines indicate reversed ionic interactions existing in the wild-type coiled-coil.

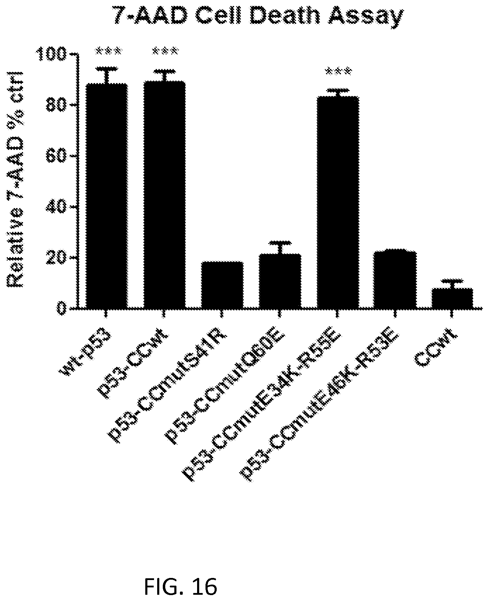

Figure 16 is a bar graph showing tumor suppressor activity screening using the 7-AAD assay was conducted in T47D cells 48 h post transfection. p53-CCmutE34K-R55E is the only candidate that retains the ability to induce cell death in a similar to p53-CCwt and the wtp53 control. CCwt was used as a negative control. Statistical analysis was performed using one way ANOVA with Bonferroni's post test; *** p < 0.001 compared to CCwt. Error bars represent standard deviations (n=3).

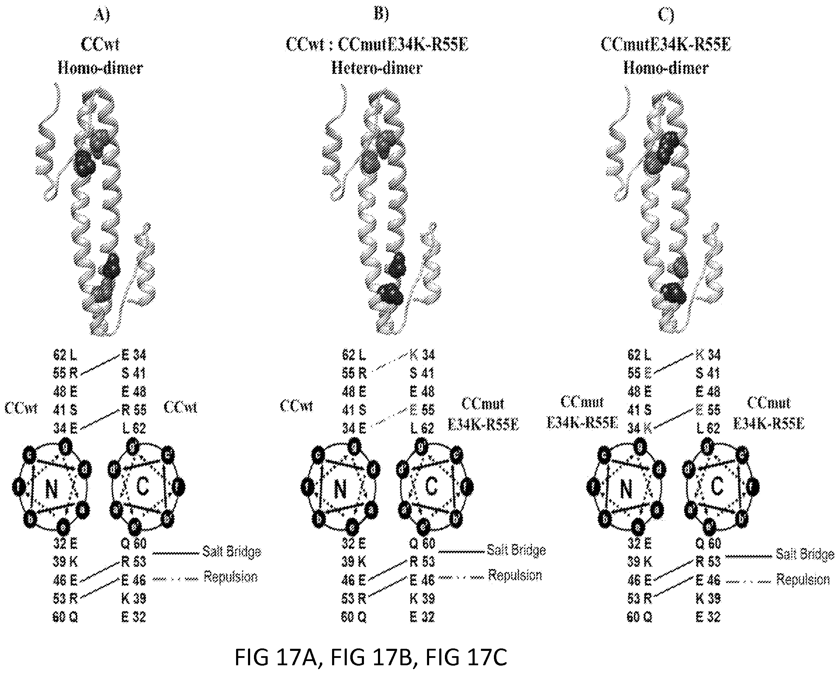

Figures 17A, 17B, and 17C show ribbon diagrams with corresponding helical wheels of CCwt homo-dimer (A), CCwt-CCmutE34K-R55E hetero-dimer (B), and CCmutE34K-R55E homo-dimer (C). Gray ribbons represent the CCwt domain, and cyan ribbons represent the CCmutE34K-R55E domain. The side chains of key residues (Glu/Lys-34 and Arg/Glu-55) are shown. Solid lines indicate salt bridges, while the long dash double dotted line represents charge-charge repulsions.

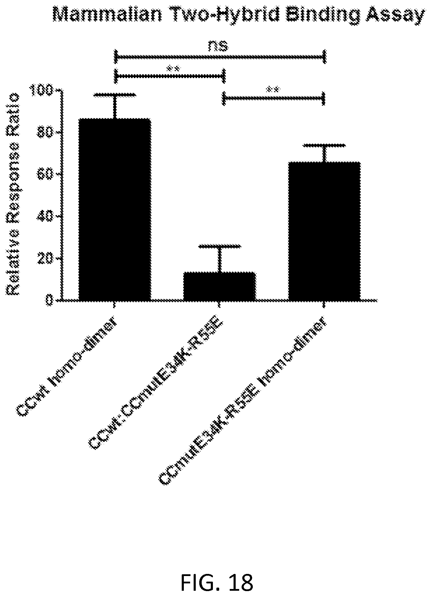

Figure 18 Binding of CCmutE34K-R55E homo- and hetero-dimers with CCwt tested using the mammalian-two hybrid assay. The assay was carried out in COS-7 cells 24 h post transfection. Both CCwt and CCmutE34K-R55E have similar binding as indicated by the first and third bar, respectively. The mammalian-two hybrid assay revealed weak binding of CCmutE34K-R55E hetero-dimerization with CCwt. Statistical analysis was performed using one-way ANOVA with Bonferroni's post test; ** p < 0.01, ns =not significant. Error bars represent standard deviations (n=3).

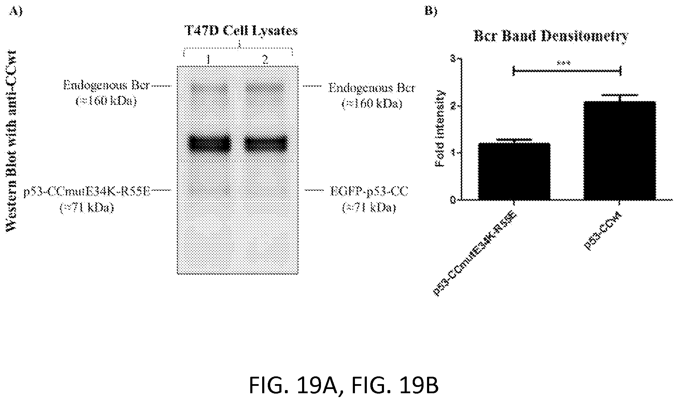

Figures 19A and 19B show the interaction ofp53-CCmutE34K-R55E and p53-CC with endogenous Bcr was investigated in T47D cells via co-IP. A) A representative cropped western blot of protein complexes co-immunoprecipitated using anti-GFP antibody is shown.

Left lane, endogenous Bcr (160 kDa) co-immunoprecipitates with p53-CCmutE34K-R55E (71 kDa) to a lesser extent compared to that with p53-CC (71 kDa) in the right lane. B) Semi-quantitative densitometric analyses was carried out as described before to evaluate Bcr interaction with p53-CCmutE34K-R55E and p53-CC (Serrano, Jap. J. Cancer Res. 93, 559-566 (2002)). Mean values were analyzed using one-way ANOVA with Bonferroni's post test; *** p < 0.001. Error bars represent standard deviations (n=3).

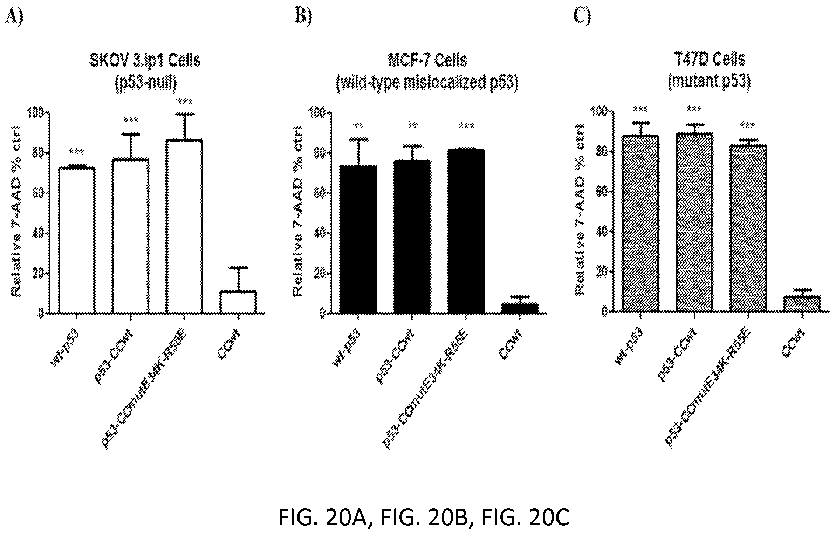

Figures 20A, 20B, and 20C show 7-AAD assays conducted in three different cell lines with varying p53 status (A) SKOV 3.ip1, (B) MCF-7, and (C) T47D cells. In all three cases, p53-CCmutE34K-R55E was capable of inducing cell death in a similar fashion compared to p53-CC and wt-p53, regardless of the endogenous p53 status or the cancer cell line used. Statistical analysis was performed using one-way ANOVA with Bonferroni's post test; ** p < 0.01 and *** p < 0.001.

### DETAILED DESCRIPTION

The disclosed method and compositions may be understood more readily by reference to the following detailed description of particular embodiments and the Example included therein and to the Figures and their previous and following description.

It is to be understood that the disclosed method and compositions are not limited to specific synthetic methods, specific analytical techniques, or to particular reagents unless otherwise specified, and, as such, may vary. It is also to be understood that the terminology used herein is for the purpose of describing particular embodiments only and is not intended to be limiting.

### A. Definitions

It must be noted that as used herein and in the appended claims, the singular forms "a", "an", and "the" include plural reference unless the context clearly dictates otherwise. Thus, for example, reference to "a peptide" includes a plurality of such peptides, reference to "the nucleic acid sequence" is a reference to one or more nucleic acid sequences and equivalents thereof known to those skilled in the art, and so forth.

As used herein, the term "amino acid sequence" refers to a list of abbreviations, letters, characters or words representing amino acid residues.

The amino acid abbreviations used herein are conventional one letter codes for the amino acids and are expressed as follows: A, alanine; B, asparagine or aspartic acid; C, cysteine; D aspartic acid; E, glutamate, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine; Z, glutamine or glutamic acid.

By an "effective amount" of a compound as provided herein is meant a sufficient amount of the compound to provide the desired effect. The exact amount required will vary from subject to subject, depending on the species, age, and general condition of the subject, the severity of disease (or underlying genetic defect) that is being treated, the particular compound used, its mode of administration, and the like. Thus, it is not possible to specify an exact "effective amount." However, an appropriate "effective amount" may be determined by one of ordinary skill in the art using only routine experimentation.

"Native Bcr" refers to the Bcr protein found in nature. For example, native Bcr refers to the Bcr found naturally in a subject.

The phrase "nucleic acid" as used herein refers to a naturally occurring or synthetic oligonucleotide or polynucleotide, whether DNA or RNA or DNA-RNA hybrid, single-stranded or double-stranded, sense or antisense, which is capable of hybridization to a complementary nucleic acid by Watson-Crick base-pairing. Nucleic acids of the invention can also include nucleotide analogs (e.g., BrdU), and non-phosphodiester internucleoside linkages (e.g., peptide nucleic acid (PNA) or thiodiester linkages). In particular, nucleic acids can include, without limitation, DNA, RNA, eDNA, gDNA, ssDNA, dsDNA or any combination thereof.

"Optional" or "optionally" means that the subsequently described event, circumstance, or material may or may not occur or be present, and that the description includes instances where the event, circumstance, or material occurs or is present and instances where it does not occur or is not present.

A "partial p53 peptide" refers to a peptide comprising a wild type p53 (wt p53) peptide sequence, wherein the wt p53 peptide sequence lacks a functional p53 tetramerization domain (TD). In some embodiments, a partial p53 peptide can comprise a wt p53 peptide sequence without the TD. In some embodiments, a partial p53 peptide can comprise a wt p53 peptide sequence with a partial TD, wherein the partial TD is not functional. In some embodiments, a partial p53 peptide comprises a full length p53 peptide, wherein the p53 peptide has at least one mutation in the TD rendering the TD non- functional. A partial p53 peptide is not able to form tetramers with wt p53 or naturally occurring mutant p53 found in cancer cells. A partial p53 peptide can only form tetramers with other partial p53 peptides. In other words, partial p53 peptides homo-oligomerize and do not hetero-oligomerize.

The term "percent sequence identity" refers to the percent sequence identity between any two sequences, such as peptide or nucleic acid sequences, that can be calculated using sequence alignment programs and parameters described elsewhere herein. The percent sequence identity between any two sequences can be at least about 40%, 45%, 50%, 55%, 60%, 65%, 70%,75%,80%, 85%,90%,91%,92%,93%,94%,95%,96%,97%,98%,99%, 99.3%, 99.5% or more sequence identity.

A "wild-type Bcr coiled-coil" (w.t. Bcr coiled-coil) refers to the coiled-coil domain present in wild-type or native Bcr. W.t. Bcr coiled-coil refers to the sequence of SEQ ID N0:4. The sequence of SEQ ID N0:4 is MVDPVGFAEAWKAQFPDSEPPRMELRSVGDIEQELERCKASIRRLEQEVNQERFRMIYLQTLLAKEKKSYDR.

A "mutant Bcr coiled-coil" refers to the w.t. Bcr coiled-coil sequence of SEQ ID N0:4 having at least one amino acid mutation. For example, the mutated Bcr coiled-coil domain can have a lysine at position 34 and a glutamic acid at position 55 of SEQ ID N0:4.

A "partial tetramerization domain" refers to a peptide comprising a portion of the p53 TD but not the entire TD. A partial TD is lacking one or more sequences responsible for tetramerization activity and therefore a partial TD is non-functional.

"Peptide" as used herein refers to any polypeptide, oligopeptide, gene product, expression product, or protein. A peptide is comprised of consecutive amino acids. The term "peptide" encompasses recombinant, naturally occurring and synthetic molecules.

In addition, as used herein, the term "peptide" refers to amino acids joined to each other by peptide bonds or modified peptide bonds, e.g., peptide isosteres, etc. and may contain modified amino acids other than the 20 gene-encoded amino acids. The peptides can be modified by either natural processes, such as post-translational processing, or by chemical modification techniques which are well known in the art. Modifications can occur anywhere in the polypeptide, including the peptide backbone, the amino acid side-chains and the amino or carboxyl termini. The same type of modification can be present in the same or varying degrees at several sites in a given peptide. Also, a given peptide can have many types of modifications. Modifications include, without limitation, acetylation, acylation, ADP-ribosylation, amidation, covalent cross-linking or cyclization, covalent attachment of flavin, covalent attachment of a heme moiety, covalent attachment of a nucleotide or nucleotide derivative, covalent attachment of a lipid or lipid derivative, covalent attachment of a phosphytidylinositol, disulfide bond formation, demethylation, formation of cysteine or pyroglutamate, formylation, gamma-carboxylation, glycosylation, GPI anchor formation, hydroxylation, iodination, methylation, myristolyation, oxidation, pergylation, proteolytic processing, phosphorylation, prenylation, racemization, selenoylation, sulfation, and transfer-RNA mediated addition of amino acids to protein such as arginylation. (See Proteins- Structure and Molecular Properties 2nd Ed., T.E. Creighton, W.H. Freeman and Company, New York (1993); Posttranslational Covalent Modification of Proteins, B.C. Johnson, Ed., Academic Press, New York, pp. 1-12 (1983)).

As used herein, the term "subject" or "patient" refers to any organism to which a composition of this invention may be administered, e.g., for experimental, diagnostic, and/or therapeutic purposes. Typical subjects include animals (e.g., mammals such as non-human primates, and humans; avians; domestic household or farm animals such as cats, dogs, sheep, goats, cattle, horses and pigs; laboratory animals such as mice, rats and guinea pigs; rabbits; fish; reptiles; zoo and wild animals) and/or plants. Typically, "subjects" are animals, including mammals such as humans and primates and the like.

The "tetramerization domain (TD)" refers to amino acids 323-356 of SEQ ID NO: 1.

By "treat" is meant to administer a compound or molecule of the invention to a subject, such as a human or other mammal (for example, an animal model), that has an increased susceptibility for developing a hyperproliferative disorder, or that has a hyperproliferative disorder, in order to prevent or delay a worsening of the effects of the disease or condition, or to partially or fully reverse the effects of the disease. For example, the hyperproliferative disorder can be cancer.

The term "wild type p53 (wt p53) peptide sequence" or "wt p53" refers to the p53 sequence of SEQ ID NO: 1. The sequence of SEQ ID NO: 1 is

Ranges may be expressed herein as from "about" one particular value, and/or to "about" another particular value. When such a range is expressed, also specifically contemplated and considered disclosed is the range^{∼} from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Throughout the description and claims of this specification, the word "comprise" and variations of the word, such as "comprising" and "comprises," means "including but not limited to," and is not intended to exclude, for example, other additives, components, integers or steps. In particular, in methods stated as comprising one or more steps or operations it is specifically contemplated that each step comprises what is listed (unless that step includes a limiting term such as "consisting of'), meaning that each step is not intended to exclude, for example, other additives, components, integers or steps that are not listed in the step.

Disclosed are materials, compositions, and components that can be used for, can be used in conjunction with, can be used in preparation for, or are products of the disclosed method and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. Thus, if a class of molecules A, B, and C are disclosed as well as a class of molecules D, E, and F and an example of a combination molecule, A-D is disclosed, then even if each is not individually recited, each is individually and collectively contemplated. Thus, is this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### B. Peptides

Disclosed are peptides comprising a partial p53 peptide and a mutated Bcr coiled-coil domain, wherein the partial p53 peptide does not have a functional tetramerization domain but retains tumor suppressor activity and the mutated Bcr coiled-coil domain comprises amino acid substitutions and deletions but retains tetramerization activity, wherein the mutated Bcr coiled-coil domain comprises mutations at residues 34 and 55 of SEQ ID NO:4 and has a lysine at position 34 and a glutamic acid at position 55 of SEQ ID NO:4 or comprises the sequence of SEQ ID NO:5, wherein the mutated Bcr coiled-coil domains comprise at least 50% sequence identity to SEQ ID NO:4.

Disclosed are peptides comprising a partial p53 peptide and a mutated Bcr coiled-coil domain, wherein the mutated Bcr coiled-coil domain is linked to the C-terminus of the DNA binding domain of the partial p53 peptide or is located between the DNA binding domain of the partial p53 peptide and the C-terminus of the peptide.

SEQ ID N0:3 is a p53-coiled coil (p53-cc) peptide construct referred to as p53-CCmutE34K-R55E peptide.

The amino acid sequence of SEQ ID N0:3 is

Disclosed are peptides comprising a partial p53 peptide and a mutated Bcr coiled-coil domain, wherein dimer or tetramer binding is strengthened.

Disclosed are peptides comprising a partial p53 peptide and a mutated Bcr coiled-coil domain, wherein interactions with native Bcr are eliminated.

The peptides disclosed herein, wherein the peptides retain functional transcriptional activity. In some embodiments, the transcriptional activity is similar to wt p53 transcriptional activity. Regardless of whether the full p53 TD is absent in the partial p53 peptide sequence or whether a portion of the p53 TD is absent, the disclosed peptides can still retain transcriptional activity. In some embodiments the transcriptional activity is at least 25%, 30%, 35%, 40%, 45%, 50%, 55%, 60%, 65%, 70%, 75%, 80%, 85%, 90%, 95%, or 100% of wt p53 transcriptional activity.

Disclosed are peptides comprising a partial p53 peptide and a mutated Bcr coiled-coil domain, wherein the peptides are capable of triggering apoptosis.

Disclosed are peptides comprising a partial p53 peptide and a mutated Bcr coiled-coil domain, wherein the peptide is capable of triggering p53-dependent apoptosis.

Also disclosed are peptides described herein for use in the treatment of cancer or hyperproliferative disorders.

### 1. Partial p53 peptide

Partial p53 peptides comprise a wt p53 peptide sequence, wherein the wt p53 peptide sequence lacks a functional p53 TD. In some embodiments, a partial p53 peptide can comprise a wt p53 peptide sequence without the TD. The entire TD can be removed from the wt p53 peptide sequence. In some embodiments, just the TD is removed from the wt p53 peptide sequence. In some embodiments, the TD is removed as well as other wt p53 peptide sequences as long as the remaining partial p53 peptide retains transcriptional activity and the ability to localize to the nucleus. For example, a partial p53 peptide can comprise a wt p53 peptide sequence lacking the TD and at least one nuclear localization signal as long as at least one nuclear localization signal is retained. In some embodiments, a partial p53 peptide comprises a wt p53 peptide, wherein the wt p53 peptide has at least one mutation in the TD rendering the TD non- functional. The mutation in the TD can be a substitution or a deletion.

In some embodiments, partial p53 peptides comprises a full length p53 peptide, wherein the p53 peptide has at least one mutation in the TD rendering the TD non- functional. Mutations can be amino acid substitutions or deletions. Amino acid mutations in the TD can be conservative or non-conservative amino acid substitutions.

In some embodiments, partial p53 peptides can comprise a wt p53 peptide sequence with a partial TD, wherein the partial TD is not functional. Thus, one or more amino acids of the TD can be substituted or deleted resulting in a partial TD that is not functional. A partial TD that is not functional means that the TD does not have tetramerization activity.

Partial p53 peptides are not able to form tetramers with wt p53 or naturally occurring mutant p53 found in cancer cells. Partial p53 peptides can only form tetramers with other partial p53 peptides. In other words, partial p53 peptides homo-oligomerize and do not hetero-oligomerize. This ability to homo-oligomerize prevents mutant p53 found in cancer cells from oligomerizing with the disclosed peptides and inhibiting their function due to transdominant inhibition.

The wt p53 peptide sequence is

The TD of the wt p53 peptide sequence is amino acids 323-356 of SEQ ID NO: 1. The amino acid sequence ofp53 TD is LDGEYFTLQIRGRERFEMFRELNEALELKDAQAG (SEQ ID N0:2).

### 2. Bcr coiled-coil domain

Like the TD of wt p53, the Bcr coiled-coil domain folds into an antiparallel dimer of dimers. Thus, the TD of wt p53, or a portion thereof, can be replaced with the Bcr coiled-coil domain. Mutating the Bcr coiled-coil domain can strengthen dimer/tetramer binding and minimize interactions with endogenous Bcr.

Disclosed are peptides comprising a partial p53 peptide and a mutated Bcr coiled-coil domain, wherein the mutated Bcr coiled-coil domain is linked to the C-terminus of the DNA binding domain of the partial p53 peptide. For example, all amino acids after the C-terminus end of the DNA binding domain can be removed from p53 and replaced with a mutated Bcr coiled-coil.

Disclosed are peptides comprising a partial p53 peptide and a mutated Bcr coiled-coil domain, wherein the mutated Bcr coiled-coil domain is located between the DNA binding domain of the partial p53 peptide and the C-terminus of the peptide. For example, the peptide can comprise a partial p53 peptide linked to a mutated Bcr coiled-coil domain linked to one or more of the C-terminal nuclear localization signals of a p53 peptide.

Mutated Bcr coiled-coil domains can comprise at least two mutated amino acids. For example, disclosed are peptides comprising a partial p53 peptide and a mutated Bcr coiled-coil domain, wherein the mutated Bcr coiled-coil domain comprises mutations at residues 34 and 55 of SEQ ID N0:4. SEQ ID N0:4 represent wt Bcr coiled-coil and has the amino acid sequence MVDPVGFAEAWKAQFPDSEPPRMELRSVGDIEQELERCKASIRRLEQEVNQERFRMIYLQTLLAKEKKSYDR (SEQ ID N0:4). For example, the mutated Bcr coiled-coil domain can have a lysine at position 34 and a glutamic acid at position 55 of SEQ ID N0:4. In other words, disclosed are peptides comprising a partial p53 peptide and a mutant Bcr coiled-coil, wherein the mutated Bcr coiled-coil domain comprises the amino acid sequence of MVDPVGFAEAWKAQFPDSEPPRMELRSVGDIEQKLERCKASIRRLEQEVNQERFEMIYL QTLLAKEKKSYDR (SEQ ID N0:5), or active fragments thereof. The underlined amino acids are the positions mutated compared to wt Bcr coiled coil (SEQ ID N0:4). In some instances, the mutated Bcr coiled-coil domain consists of the sequence of SEQ ID N0:5, or active fragments thereof. Active fragments of SEQ ID N0:5 comprise Bcr coiled-coil sequences that retain the ability to help form homo-oligomers of the peptides comprising a partial p53 peptide and a mutated Bcr coiled-coil domain.

Mutated Bcr coiled-coil domains can comprise 50%, 55%, 60%, 65%, 70%, 75%, 80%, 85%, 90%, 91%, 92%, 93%, 94%, 95%, 96%, or 97% sequence identity to wt Bcr coiled coil (SEQ ID N0:4). For example, SEQ ID N0:5 is a mutated Bcr coiled coil that comprises two amino acid mutations and has about 97% sequence identity to SEQ ID N0:4. The mutations can be amino acid substitutions or deletions. The mutations can be conservative or non-conservative amino acid substitutions.

### C. Nucleic Acid Sequences

Disclosed are nucleic acid sequences capable of encoding the peptides disclosed herein. Also disclosed are isolated nucleic acid sequences capable of encoding one or more of the peptides described herein. Nucleic acid sequences can comprise DNA, RNA, and/or eDNA.

Disclosed are nucleic acid sequences capable of encoding a peptide comprising a partial p53 peptide and a mutated Bcr coiled-coil domain.

Also disclosed are nucleic acid sequences capable of encoding a peptide comprising a partial p53 peptide and a mutated Bcr coiled-coil domain, wherein the nucleic acid sequences comprise the sequence of SEQ ID N0:8. SEQ ID N0:8 is a p53-cc nucleic acid construct referred to as p53-CCmutE34K-R55E nucleic acid sequence. The nucleic acid sequence of SEQ ID N0:8 is

Disclosed are nucleic acid sequences capable of encoding a peptide comprising a partial p53 peptide and a mutated Bcr coiled-coil domain, wherein the peptide is rationally designed to exclude interaction with native Bcr, maintain transcriptional activity of p53, and overcome dominant negative effect of endogenous p53. The ability of the disclosed peptides to avoid hetero-oligomerization and to form homo-oligomers avoids the dominant negative effect of endogenous p53. Maintaining transcriptional activity means the disclosed peptides have at least 25%, 30%, 35%, 40%, 45%, 50%, 55%, 60%, 65%, 70%, 75%, 80%, 85%, 90%, 95%, or 100% of wt p53 transcriptional activity.

Also disclosed are the uses of the nucleic acid sequences described herein for use in the treatment of cancer.

### 1. Partial p53 peptide

Disclosed are the nucleic acid sequences that encode the partial p53 peptides disclosed herein. For example, disclosed are partial p53 nucleic acid sequences. Partial p53 nucleic acid sequences can comprise a wt p53 nucleic acid sequence, wherein the wt p53 nucleic acid sequence encodes a wt p53 peptide sequence that lacks a functional p53 TD.

Wt p53 nucleic acid sequence is

Therefore, disclosed are partial p53 nucleic acid sequences comprising a partial nucleic acid sequence of SEQ ID N0:6. For example, a partial p53 nucleic acid sequence can comprise SEQ ID N0:6 lacking the nucleic acid sequence encoding the p53 TD. In some embodiments a partial p53 TD nucleic acid sequence is present in the disclosed nucleic acid sequences. A partial p53 TD nucleic acid sequence encodes a non-functional partial p53 TD peptide.

The nucleic acid sequence of p53 TD domain is

Disclosed are nucleic acid sequences that encode a partial p53 peptide, wherein the partial p53 peptide comprises a wt p53 peptide, wherein the wt p53 peptide has at least one mutation in the TD rendering the TD non- functional. The mutation in the TD can be a substitution or a deletion.

### 2. Bcr coiled-coil

Disclosed are nucleic acid sequences capable of encoding a peptide comprising a partial p53 peptide and a mutated Bcr coiled-coil domain, wherein the mutated Bcr coiled-coil domain is linked to the C-terminus of the DNA binding domain of a partial p53 peptide. For example, all amino acids after the C-terminus end of the DNA binding domain can be removed from p53 and replaced with a mutated Bcr coiled-coil. Thus, all of the nucleic acids after the C-terminus of the nucleic acid sequence that is capable of encoding the DNA binding domain can be removed from the wt p53 nucleic acid sequence and replaced with a nucleic acid sequence that encodes a mutated Bcr coiled-coil.

Disclosed are nucleic acid sequences capable of encoding a peptide comprising a partial p53 peptide and a mutated Bcr coiled-coil domain, wherein the mutated Bcr coiled-coil domain is located between the DNA binding domain of the partial p53 peptide and the C'-terminus of the peptide. For example, the nucleic acid sequence can encode a peptide that comprises a partial p53 peptide linked to a mutated Bcr coiled-coil domain linked to one or more of the C-terminal nuclear localization signals of a p53 peptide.

Also disclosed are nucleic acid sequences capable of encoding a peptide comprising a partial p53 peptide and a mutated Bcr coiled-coil domain, wherein the mutated Bcr coiled-coil domain comprises mutations at residues 34 and 55 of SEQ ID N0:4. For example, the mutated Bcr coiled-coil domain can have a lysine at position 34 and a glutamic acid at position 55 of SEQ ID N0:4. In other words, disclosed are nucleic acid sequences capable of encoding peptides that comprise a partial p53 peptide and a mutant Bcr coiled-coil, wherein the mutated Bcr coiled-coil domain comprises the amino acid sequence of SEQ ID N0:5. Thus, disclosed are nucleic acid sequences that encode mutated Bcr coiled-coil domain (SEQ ID N0:5), wherein the nucleic acid sequences can comprise the sequence of SEQ ID NO: 10, or active fragments thereof. In some instances the nucleic acid sequence can consist of SEQ ID NO: 10, or active fragments thereof. SEQ ID NO: 10 is the mutant Bcr coiled-coil domain having the nucleic acid sequence of atggtggacccggtgggcttcgcggaggcgtggaaggcgcagttcccggactcagagcccccgcgcatggagctgcgctcagtgggc gacatcgagcagAagctggagcgctgcaaggcctccattcggcgcctggagcaggaggtgaaccaggagcgcttcGAGatgatcta cctgcagacgttgctggccaaggaaaagaagagctatgaccg. SEQ ID NO: 10 is capable of encoding a mutant Bcr coiled-coil domain comprising E34K and R55E mutations. These mutations are mutations from the wt Bcr coiled-coil domain which is capable of being encoded by the nucleic acid sequence of SEQ ID N0:9. SEQ ID N0:9 is the wt Bcr coiled-coil domain nucleic acid sequence of

### D. Vectors

Disclosed are vectors comprising the nucleic acids disclosed herein. For example, disclosed are vectors comprising a nucleic acid sequence, wherein the nucleic acid sequence is capable of encoding a peptide comprising a partial p53 peptide and a mutated Bcr coiled-coil domain.

Also disclosed are vectors comprising a nucleic acid sequence, wherein the nucleic acid sequence is capable of encoding a peptide comprising a partial p53 peptide and a mutated Bcr coiled-coil domain, wherein the mutated Bcr coiled-coil domain is linked to the C-terminus of the DNA binding domain of the partial p53 peptide. For example, all amino acids after the C-terminus end of the DNA binding domain can be removed from p53 and replaced with a mutated Bcr coiled-coil. Thus, all of the nucleic acids after the C-terminus of the nucleic acid sequence that is capable of encoding the DNA binding domain can be removed from the wt p53 nucleic acid sequence and replaced with a nucleic acid sequence that encodes a mutated Bcr coiled-coil.

Disclosed are vectors comprising a nucleic acid sequence, wherein the nucleic acid sequence is capable of encoding a peptide comprising a partial p53 peptide and a mutated Bcr coiled-coil domain, wherein the mutated Bcr coiled-coil domain is located between the DNA binding domain of the partial p53 peptide and the C-terminus of the peptide. For example, the nucleic acid sequence can encode a peptide that comprises a partial p53 peptide linked to a mutated Bcr coiled-coil domain linked to one or more of the C' terminal nuclear localization signals of a p53 peptide.

Disclosed are vectors comprising a nucleic acid sequence, wherein the nucleic acid sequence is capable of encoding a peptide comprising a partial p53 peptide and a mutated Bcr coiled-coil domain, wherein the mutated Bcr coiled-coil domain comprises mutations at residues 34 and 55 of SEQ ID N0:4. For example, the mutated Bcr coiled-coil domain can have a lysine at position 34 and a glutamic acid at position 55 of SEQ ID N0:4. In other words, disclosed are vectors comprising nucleic acid sequences capable of encoding peptides that comprise a partial p53 peptide and a mutant Bcr coiled-coil, wherein the mutated Bcr coiled-coil domain comprises the amino acid sequence of SEQ ID N0:5, or active fragments thereof. In some instances, the mutated Bcr coiled-coil domain consists of the amino acid sequence of SEQ ID N0:5, or active fragments thereof.

Disclosed are vectors comprising a nucleic acid sequence, wherein the nucleic acid sequence is capable of encoding a peptide comprising a partial p53 peptide and a mutated Bcr coiled-coil domain, wherein the nucleic acid sequence comprises SEQ ID N0:8. Thus, these vectors comprise a nucleic acid sequence capable of encoding the p53-CCmutE34K-R55E peptide.

### 1. Viral and Non-Viral Vectors

The vectors disclosed herein can be viral or non-viral vectors. For example, the disclosed vectors can be viral vectors. Specifically, the disclosed vectors can be adenoviral vectors.

There are a number of compositions and methods which can be used to deliver nucleic acids to cells, either in vitro or in vivo. These methods and compositions can largely be broken down into two classes: viral based delivery systems and non-viral based delivery systems. For example, the nucleic acids can be delivered through a number of direct delivery systems such as, electroporation, lipofection, calcium phosphate precipitation, plasmids, viral vectors, viral nucleic acids, phage nucleic acids, phages, cosmids, or via transfer of genetic material in cells or carriers such as cationic liposomes. Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991). Such methods are well known in the art and readily adaptable for use with the compositions and methods described herein. In certain cases, the methods will be modified to specifically function with large DNA molecules. Further, these methods can be used to target certain diseases and cell populations by using the targeting characteristics of the carrier.

Expression vectors can be any nucleotide construction used to deliver genes or gene fragments into cells (e.g., a plasmid), or as part of a general strategy to deliver genes or gene fragments, e.g., as part of recombinant retrovirus or adenovirus (Ram et al. Cancer Res. 53:83-88, (1993)). For example, disclosed herein are expression vectors comprising a nucleic acid sequence capable of encoding one or more of the disclosed peptides operably linked to a control element.

The "control elements" present in an expression vector are those non-translated regions of the vector--enhancers, promoters, 5' and 3' untranslated regions--which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid IacZ promoter of the pBLUESCRIPT phagemid (Stratagene, La Jolla, Calif.) or pSPORTI plasmid (Gibco BRL, Gaithersburg, Md.) and the like may be used. In mammalian cell systems, promoters from mammalian genes or from mammalian viruses are generally preferred. If it is necessary to generate a cell line that contains multiple copies of the sequence encoding a polypeptide, vectors based on SV40 or EBV may be advantageously used with an appropriate selectable marker.

Preferred promoters controlling transcription from vectors in mammalian host cells may be obtained from various sources, for example, the genomes of viruses such as polyoma, Simian Virus 40 (SV40), adenovirus, retroviruses, hepatitis-B virus and most preferably cytomegalovirus, or from heterologous mammalian promoters (e.g., beta actin promoter). The early and late promoters of the SV 40 virus are conveniently obtained as an SV 40 restriction fragment, which also contains the SV40 viral origin of replication (Piers et al., Nature, 273: 113 (1978)). The immediate early promoter of the human cytomegalovirus is conveniently obtained as a Hindiii E restriction fragment (Greenway, P.J. et al., Gene 18: 355-360 (1982)). Additionally, promoters from the host cell or related species can also be used.

Enhancer generally refers to a sequence of DNA that functions at no fixed distance from the transcription start site and can be either 5' (Laimins, L. et al., Proc. Natl. Acad. Sci. 78:993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, a-fetoprotein and insulin), typically one will use an enhancer from a eukaryotic cell virus for general expression. Preferred examples are the SV 40 enhancer on the late side of the replication origin (bp 100-270), the cytomegalovirus early promoter enhancer, the polyoma enhancer on the late side of the replication origin, and adenovirus enhancers.

The promoter or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

Optionally, the promoter or enhancer region can act as a constitutive promoter or enhancer to maximize expression of the polynucleotides of the invention. In certain constructs the promoter or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTR.

Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contains a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV 40 early polyadenylation signal and consists of about 400 bases.

The expression vectors can include a nucleic acid sequence encoding a marker product. This marker product is used to determine if the gene has been delivered to the cell and once delivered is being expressed. Preferred marker genes are the E. coli lacZ gene, which encodes B-galactosidase, and the gene encoding the green fluorescent protein.

In some embodiments the marker may be a selectable marker. Examples of suitable selectable markers for mammalian cells are dihydrofolate reductase (DHFR), thymidine kinase, neomycin, neomycin analog G418, hygromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are CHO DHFR-cells and mouse LTK-cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Bioi. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

As used herein, plasmid or viral vectors are agents that transport the disclosed nucleic acids, such as a nucleic acid sequence capable of encoding one or more of the disclosed peptides into the cell without degradation and include a promoter yielding expression of the gene in the cells into which it is delivered. In some embodiments the nucleic acid sequences disclosed herein are derived from either a virus or a retrovirus. Viral vectors are, for example, Adenovirus, Adena-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Retroviruses include Murine Maloney Leukemia virus, MML V, and retroviruses that express the desirable properties of MML V as a vector. Retroviral vectors are able to carry a larger genetic payload, i.e., a transgene or marker gene, than other viral vectors, and for this reason are a commonly used vector. However, they are not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. A preferred embodiment is a viral vector which has been engineered so as to suppress the immune response of the host organism, elicited by the viral antigens. Preferred vectors of this type will carry coding regions for Interleukin 8 or 10.

Viral vectors can have higher transaction abilities (i.e., ability to introduce genes) than chemical or physical methods of introducing genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

Retroviral vectors, in general, are described by Verma, I.M., Retroviral vectors for gene transfer in Microbiology, Amer. Soc. for Microbiology, pp. 229-232, Washington, (1985) . Examples of methods for using retroviral vectors for gene therapy are described in U.S. Patent Nos. 4,868,116 and 4,980,286; PCT applications WO 90/02806 and WO 89/07136; and Mulligan, (Science 260:926-932 (1993)) .

A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serves as the priming site for the synthesis of the second strand of DNA synthesis, and specific sequences near the ends of the L TRs that enable the insertion of the DNA state of the retrovirus to insert into the host genome. This amount of nucleic acid is sufficient for the delivery of a one to many genes depending on the size of each transcript. It is preferable to include either positive or negative selectable markers along with other genes in the insert.

Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., I. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell but are unable to form new infectious viral particles. Recombinant adenoviruses have been shown to achieve high efficiency gene transfer after direct, in vivo delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Bioi. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)) . Recombinant adenoviruses achieve gene transduction by binding to specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol., 4:1528-1533 (1984); Varga et al., J. Virology 65:6061-6070 (1991); Wickham et al., Cell 73:309-319 (1993)).

A viral vector can be one based on an adenovirus which has had the E1 gene removed and these virons are generated in a cell line such as the human 293 cell line. Optionally, both the E1 and E3 genes are removed from the adenovirus genome.

Another type of viral vector that can be used to introduce the polynucleotides of the invention into a cell is based on an adena-associated virus (AA V). This defective parvovirus is a preferred vector because it can infect many cell types and is nonpathogenic to humans. AAV type vectors can transport about 4 to 5 kb and wild type AA V is known to stably insert into chromosome 19. Vectors which contain this site specific integration property are preferred. An especially preferred embodiment of this type of vector is the P4.1 C vector produced by A vigen, San Francisco, CA, which can contain the herpes simplex virus thymidine kinase gene, HSV -tk, or a marker gene, such as the gene encoding the green fluorescent protein, GFP.

In another type of AA V virus, the AA V contains a pair of inverted terminal repeats (ITRs) which flank at least one cassette containing a promoter which directs cell-specific expression operably linked to a heterologous gene. Heterologous in this context refers to any nucleotide sequence or gene which is not native to the AA V or B 19 parvovirus. Typically the AA V and B 19 coding regions have been deleted, resulting in a safe, noncytotoxic vector. The AA V ITRs, or modifications thereof, confer infectivity and site-specific integration, but not cytotoxicity, and the promoter directs cell-specific expression. United States Patent No. 6,261,834 refers to material related to the AAV vector.

The inserted genes in viral and retroviral vectors usually contain promoters, or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

Other useful systems include, for example, replicating and host-restricted nonreplicating vaccinia virus vectors. In addition, the disclosed nucleic acid sequences can be delivered to a target cell in a non-nucleic acid based system. For example, the disclosed polynucleotides can be delivered through electroporation, or through lipofection, or through calcium phosphate precipitation. The delivery mechanism chosen will depend in part on the type of cell targeted and whether the delivery is occurring for example in vivo or in vitro.

Thus, the compositions can comprise, in addition to the disclosed expression vectors, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a peptide and a cationic liposome can be administered to the blood, to a target organ, or inhaled into the respiratory tract to target cells of the respiratory tract. For example, a composition comprising a peptide or nucleic acid sequence described herein and a cationic liposome can be administered to a subjects lung cells. Regarding liposomes, see, e.g., Brigham et al. Am. J. Resp. Cell. Mol. Biol. 1:95-100 (1989); Felgner et al. Proc. Natl. Acad. Sci USA 84:7413-7417 (1987); U.S. Patent No. 4,897,355. Furthermore, the compound can be administered as a component of a microcapsule that can be targeted to specific cell types, such as macrophages, or where the diffusion of the compound or delivery of the compound from the microcapsule is designed for a specific rate or dosage.

### E. Compositions

Disclosed are compositions comprising one or more of the peptides or nucleic acid sequences described herein.

### 1. Compositions comprising peptides

Disclosed are compositions comprising a peptide comprising a partial p53 peptide and a mutated Bcr coiled-coil domain.

Disclosed are compositions comprising a peptide comprising a partial p53 peptide and a mutated Bcr coiled-coil domain, wherein the mutated Bcr coiled-coil domain is linked to the C-terminus of the DNA binding domain of the partial p53 peptide. For example, all amino acids after the C-terminus end of the DNA binding domain can be removed from p53 and replaced with a mutated Bcr coiled-coil.

Disclosed are compositions comprising a peptide comprising a partial p53 peptide and a mutated Bcr coiled-coil domain, wherein the mutated Bcr coiled-coil domain is located between the DNA binding domain of the partial p53 peptide and the C-terminus of the peptide. For example, the peptide can comprise a partial p53 peptide linked to a mutated Bcr coiled-coil domain linked to one or more of the C-terminal nuclear localization signals of a p53 peptide.

Disclosed are compositions comprising a peptide comprising a partial p53 peptide and a mutated Bcr coiled-coil domain, wherein the mutated Bcr coiled-coil domain comprises the sequence of SEQ ID N0:5, or active fragments thereof. In some instances, the mutated Bcr coiled-coil domain consists of the sequence of SEQ ID N0:5, or active fragments thereof. Active fragments of SEQ ID N0:5 comprise Bcr coiled-coil sequences that retain the ability to help form homo-oligomers of the peptides comprising a partial p53 peptide and a mutated Bcr coiled-coil domain.

Disclosed are compositions comprising a peptide comprising a partial p53 peptide and a mutated Bcr coiled-coil domain, wherein the peptide comprises the sequence of SEQ ID N0:3. SEQ ID N0:3 is a p53-coiled coil (p53-cc) peptide construct referred to as p53-CCmutE34K-R55E peptide.

Disclosed are compositions comprising a peptide comprising a partial p53 peptide and a mutated Bcr coiled-coil domain, further comprising an anti-cancer agent. For example, the anti-cancer agent can comprise paclitaxel. In some instances, the composition can further comprise carboplatin. Anti-cancer agents can include, but are not limited to, paclitaxel, carboplatin or a combination thereof. Anti-cancer agents are compounds useful in the treatment of cancer. Examples of anti-cancer agents include alkylating agents such as thiotepa and CYTOXAN(R) cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL(R)); beta-lapachone; lapachol; colchicines; betulinic acid; a camptothecin (including the synthetic analogue topotecan (HYCAMTIN(R)), CPT -II (irinotecan, CAMPTOSAR(R)), acetylcamptothecin, scopolectin, and 9-aminocamptothecin); bryostatin; callystatin; CC- 1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosf amide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gammall and calicheamicin omegall (see, e.g., Agnew, Chem Inti. Ed. Engl., 33: 183-186 (1994)); dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN(R), morpholino-doxorubicin, cyanomorpholino-doxorubicin, 2-pyrrolino- doxorubicin, doxorubicin HCI liposome injection (DOXIL(R)) and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR(R)), tegafur (UFTORAL(R)), capecitabine (XELODA(R)), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK(R) polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T -2 toxin, verracurin A, roridin A and anguidine); urethane; vindesine (ELDISEME(R), FILDESIN(R)); dacarbazine; mannomustine; mitobronitol; mitolactol; pipobroman; gacytosine; arabinoside ("Ara-C"); thiotepa; taxoids, e.g., paclitaxel (TAXOL(R)), albumin-engineered nanoparticle formulation of paclitaxel (ABRAXANE(TM)), and doxetaxel (TAXOTERE(R)); chloranbucil; 6-thioguanine; mercaptopurine; methotrexate; platinum analogs such as cisplatin and carboplatin; vinblastine (VELB AN(R)); platinum; etoposide (VP- 16); ifosfamide; mitoxantrone; vincristine (ONCOVIN(R)); oxaliplatin; leucovovin; vinorelbine (NA VELBINE(R)); novantrone; edatrexate; daunomycin; aminopterin; ibandronate; topoisomerase inhibitor RFS 2000; difluorometlhylornithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN(TM)) combined with 5-FU and leucovovin.

### 2. Compositions comprising nucleic acid sequences

Disclosed are compositions comprising a nucleic acid sequence, wherein the nucleic acid sequence is capable of encoding a peptide comprising a partial p53 peptide and a mutated Bcr coiled-coil domain.

Disclosed are compositions comprising a nucleic acid sequence, wherein the nucleic acid sequence is capable of encoding a peptide comprising a partial p53 peptide and a mutated Bcr coiled-coil domain, wherein the mutated Bcr coiled-coil domain is linked to the C-terminus of the DNA binding domain of the partial p53 peptide. For example, all amino acids after the C-terminus end of the DNA binding domain can be removed from p53 and replaced with a mutated Bcr coiled-coil. Thus, all of the nucleic acids after the C-terminus of the nucleic acid sequence that is capable of encoding the DNA binding domain can be removed from the wt p53 nucleic acid sequence and replaced with a nucleic acid sequence that encodes a mutated Bcr coiled-coil.

Disclosed are compositions comprising a nucleic acid sequence, wherein the nucleic acid sequence is capable of encoding a peptide comprising a partial p53 peptide and a mutated Bcr coiled-coil domain, wherein the mutated Bcr coiled-coil domain is located between the DNA binding domain of the partial p53 peptide and the C-terminus of the peptide. For example, the peptide can comprise a partial p53 peptide linked to a mutated Bcr coiled-coil domain linked to one or more of the C-terminal nuclear localization signals of a p53 peptide.

Disclosed are compositions comprising a nucleic acid sequence, wherein the nucleic acid sequence is capable of encoding a peptide comprising a partial p53 peptide and a mutated Bcr coiled-coil domain, wherein mutated Bcr coiled-coil domain consists of the sequence of SEQ ID N0:5, or active fragments thereof. In some instances the Bcr coiled-coil domain comprises the sequence of SEQ ID N0:5, or active fragments thereof. Active fragments of SEQ ID N0:5 comprise Bcr coiled-coil sequences that retain the ability to help form homo-oligomers of the peptides comprising a partial p53 peptide and a mutated Bcr coiled-coil domain.

Disclosed are compositions comprising a nucleic acid sequence, wherein the nucleic acid sequence is capable of encoding a peptide comprising a partial p53 peptide and a mutated Bcr coiled-coil domain, wherein the peptide is rationally designed to exclude interaction with native Bcr, maintain transcriptional activity ofp53, and overcome dominant negative effect of endogenous p53.

Disclosed are compositions comprising a nucleic acid sequence, wherein the nucleic acid sequence comprises SEQ ID N0:8. Thus, these compositions comprise a nucleic acid sequence capable of encoding the p53-CCmutE34K-R55E peptide.

Also disclosed are compositions comprising one or more of the vectors described herein. For example, disclosed are compositions comprising a vector comprising a nucleic acid sequence, wherein the nucleic acid sequence is capable of encoding peptide comprising a partial p53 peptide and a mutated Bcr coiled-coil domain.

Also disclosed are compositions comprising one or more of the peptides or nucleic acid disclosed herein and a pharmaceutically acceptable carrier.

### 3. Delivery of Compositions

In the methods described herein, delivery of the compositions to cells can be via a variety of mechanisms. As defined above, disclosed herein are compositions comprising any one or more of the peptides, nucleic acids, vectors and/or antibodies described herein can be used to produce a composition which can also include a carrier such as a pharmaceutically acceptable carrier. For example, disclosed are pharmaceutical compositions, comprising the peptides disclosed herein, and a pharmaceutically acceptable carrier.

For example, the compositions described herein can comprise a pharmaceutically acceptable carrier. By "pharmaceutically acceptable" is meant a material or carrier that would be selected to minimize any degradation of the active ingredient and to minimize any adverse side effects in the subject, as would be well known to one of skill in the art. Examples of carriers include dimyristoylphosphatidyl (DMPC), phosphate buffered saline or a multivesicular liposome. For example, PG:PC:Cholesterol:peptide or PC:peptide can be used as carriers in this invention. Other suitable pharmaceutically acceptable carriers and their formulations are described in Remington: The Science and Practice of Pharmacy (19th ed.) ed. A.R. Gennaro, Mack Publishing Company, Easton, PA 1995. Typically, an appropriate amount of pharmaceutically-acceptable salt is used in the formulation to render the formulation isotonic. Other examples of the pharmaceutically-acceptable carrier include, but are not limited to, saline, Ringer's solution and dextrose solution. The pH of the solution can be from about 5 to about 8, or from about 7 to about 7.5. Further carriers include sustained release preparations such as semi-permeable matrices of solid hydrophobic polymers containing the composition, which matrices are in the form of shaped articles, e.g., films, stents (which are implanted in vessels during an angioplasty procedure), liposomes or microparticles. It will be apparent to those persons skilled in the art that certain carriers may be more preferable depending upon, for instance, the route of administration and concentration of composition being administered. These most typically would be standard carriers for administration of drugs to humans, including solutions such as sterile water, saline, and buffered solutions at physiological pH.

Pharmaceutical compositions can also include carriers, thickeners, diluents, buffers, preservatives and the like, as long as the intended activity of the polypeptide, peptide, nucleic acid, vector of the invention is not compromised. Pharmaceutical compositions may also include one or more active ingredients (in addition to the composition of the invention) such as antimicrobial agents, anti-inflammatory agents, anesthetics, and the like. The pharmaceutical composition may be administered in a number of ways depending on whether local or systemic treatment is desired, and on the area to be treated.

Preparations of parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, antioxidants, chelating agents, and inert gases and the like.

Formulations for optical administration may include ointments, lotions, creams, gels, drops, suppositories, sprays, liquids and powders. Conventional pharmaceutical carriers, aqueous, powder or oily bases, thickeners and the like may be necessary or desirable.

Compositions for oral administration include powders or granules, suspensions or solutions in water or non-aqueous media, capsules, sachets, or tablets. Thickeners, flavorings, diluents, emulsifiers, dispersing aids, or binders may be desirable. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mon-, di-, trialkyl and aryl amines and substituted ethanolamines.

### F. Compositions for Inducing Apoptosis

Disclosed are compositions comprising a peptide according to the invention for use in inducing apoptosis in the treatment of a patient, wherein the peptide comprises a partial p53 peptide and a mutated Bcr coiled-coil domain.

The compositions comprising a peptide according to the invention for inducing apoptosis in the treatment of a patient,comprising a peptide comprising a partial p53 peptide and a mutated Bcr coiled-coil domain, wherein the mutated Bcr coiled-coil domain is linked to the C-terminus of the DNA binding domain of the partial p53 peptide. For example, all amino acids after the C-terminus end of the DNA binding domain can be removed from p53 and replaced with a mutated Bcr coiled-coil.

The compositions comprising a peptide according to the invention for inducing apoptosis in the treatment of a patient comprise
a peptide comprising a partial p53 peptide and a mutated Bcr coiled-coil domain, wherein the mutated Bcr coiled-coil domain is located between the DNA binding domain of the partial p53 peptide and the C-terminus of the peptide. For example, the peptide can comprise a partial p53 peptide linked to a mutated Bcr coiled-coil domain linked to one or more of the C-terminal nuclear localization signals of a p53 peptide.

The compositions comprising a peptide according to the invention for inducing apoptosis in the treatment of a patient comprise a peptide comprising a partial p53 peptide and a mutated Bcr coiled-coil domain, wherein the mutated Bcr coiled-coil domain is located between the DNA binding domain of the partial p53 peptide and the C' terminus of the peptide.

Disclosed are compositions comprising a peptide according to the invention for use in inducing apoptosis comprising a peptide comprising a partial p53 peptide and a mutated Bcr coiled-coil domain, wherein the mutated Bcr coiled-coil domain comprises the sequence of SEQ ID N0:5, or active fragments thereof.

Disclosed are compositions comprising a peptide according to the invention for use in inducing apoptosis wherein the peptide comprises a partial p53 peptide and a mutated Bcr coiled-coil domain, wherein the peptide comprises the sequence of SEQ ID N0:3. Thus, the peptide can comprise the p53-CCmutE34K-R55E peptide.

Disclosed are compositions comprising a peptide according to the invention for use in inducing apoptosis wherein the composition comprises a peptide comprising a partial p53 peptide and a mutated Bcr coiled-coil domain, wherein the mutated Bcr coiled-coil domain comprises the sequence of SEQ ID N0:5, or active fragments thereof.

### G. Compositions for use in Suppressing Tumor Activity

Disclosed are compositions comprising a peptide disclosed herein for use in suppressing tumor activity in a patient,
wherein the peptide comprises a partial p53 peptide and a mutated Bcr coiled-coil domain, wherein the mutated Bcr coiled-coil domain is linked to the C' terminus of the DNA binding domain of the partial p53 peptide. For example, all amino acids after the C' terminus end of the DNA binding domain can be removed from p53 and replaced with a mutated Bcr coiled-coil.

Disclosed are compositions comprising a peptide disclosed herein for use in suppressing tumor activity in a patient, wherein the peptide comprises a partial p53 peptide and a mutated Bcr coiled-coil domain, wherein the mutated Bcr coiled-coil domain is located between the DNA binding domain of the partial p53 peptide and the C-terminus of the peptide. For example, the peptide can comprise a partial p53 peptide linked to a mutated Bcr coiled-coil domain linked to one or more of the C-terminal nuclear localization signals of a p53 peptide.

Disclosed are compositions comprising a peptide disclosed herein for use in suppressing tumor activity in a patient, wherein the peptide comprises a partial p53 peptide and a mutated Bcr coiled-coil domain, wherein the mutated Bcr coiled-coil comprises the sequence of SEQ ID N0:5, or active fragments thereof. In some instances, the mutated Bcr coiled-coil domain consists of the sequence of SEQ ID N0:5, or active fragments thereof.

Disclosed are compositions comprising a peptide disclosed herein for use in suppressing tumor activity in a patient, wherein the peptide comprises a partial p53 peptide and a mutated Bcr coiled-coil domain, wherein the peptide comprises the sequence of SEQ ID N0:3. Thus, the peptide can comprise the p53-CCmutE34K-R55E peptide.

Disclosed are compositions comprising a peptide disclosed herein for use in suppressing tumor activity in a patient, wherein the peptide comprises a partial p53 peptide and a mutated Bcr coiled-coil domain, wherein the peptide is rationally designed to exclude interaction with native Bcr, maintain transcriptional activity ofp53, and overcome dominant negative effect of endogenous p53.

Disclosed are compositions comprising a peptide disclosed herein for use in suppressing tumor activity in a patient, wherein the peptide comprises a partial p53 peptide and a mutated Bcr coiled-coil domain, wherein tumor activity is measured by apoptosis, proliferation, transformative ability, gene expression profiling, and dominant negative effect.

Disclosed are compositions comprising a peptide disclosed herein for use in suppressing tumor activity in a patient, wherein the peptide comprises a partial p53 peptide and a mutated Bcr coiled-coil domain, wherein the tumor comprises breast cancer, triple negative breast cancer, ovarian cancer or any blood cancer. For example, blood cancers can be any blood cancer with a mutated p53, such as but not limited to, Acute Myeloid Leukemia (AML), Chronic Lymphocytic Leukemia (CLL), Chronic Myeloid Leukemia (CML), B-Cell Chronic Lymphocytic Leukemia (B-CELL).

### H. Peptides for use in the Treatment of Hyperproliferative Disorders

Disclosed are peptides for use in the treatment of a hyperproliferative disorder in a patient wherein the peptide comprises a partial p53 peptide and a mutated Bcr coiled-coil domain.

Disclosed are peptides for use in the treatment of a hyperproliferative disorder in a patient, wherein the peptide comprises a partial p53 peptide and a mutated Bcr coiled-coil domain, wherein the mutated Bcr coiled-coil domain is linked to the C' terminus of the DNA binding domain of the partial p53 peptide. For example, all amino acids after the C' terminus end of the DNA binding domain can be removed from p53 and replaced with a mutated Bcr coiled-coil.

Disclosed are peptides for use in the treatment of a hyperproliferative disorder in a patient, wherein the peptide comprises a partial p53 peptide and a mutated Bcr coiled-coil domain, wherein the mutated Bcr coiled-coil domain is located between the DNA binding domain of the partial p53 peptide and the C-terminus of the peptide. For example, the peptide can comprise a partial p53 peptide linked to a mutated Bcr coiled-coil domain linked to one or more of the C-terminal nuclear localization signals of a p53 peptide.

Disclosed are peptides for use in the treatment of a hyperproliferative disorder in a patient, wherein the peptide comprises a partial p53 peptide and a mutated Bcr coiled-coil domain, wherein the mutated Bcr coiled-coil domain comprises the sequence of SEQ ID N0:5, or active fragments thereof. In some instances, the mutated Bcr coiled-coil domain consists of the sequence of SEQ ID N0:5, or active fragments thereof.

Disclosed are peptides for use in the treatment of a hyperproliferative disorder in a patient, wherein the peptide comprises a partial p53 peptide and a mutated Bcr coiled-coil domain, wherein the peptide comprises the sequence of SEQ ID N0:3. Thus, the peptide can comprise the p53-CCmutE34KR55E peptide.

Disclosed are peptides for use in the treatment of a hyperproliferative disorder in a patient, wherein the peptide comprises a partial p53 peptide and a mutated Bcr coiled-coil domain, wherein the peptide is rationally designed to exclude interaction with native Bcr, maintain transcriptional activity of p53, and overcome dominant negative effect of endogenous p53.

Disclosed are peptides for use in the treatment of a hyperproliferative disorder in a patient, wherein the peptide comprises a partial p53 peptide and a mutated Bcr coiled-coil domain, wherein the hyperproliferative disorder is characterized by apoptosis, proliferation, transformative ability, gene expression profiling, and dominant negative effect.

Hyperproliferative disorders can include cancer and non-cancer hyperproliferative disorders. Cancers include, but are not limited to brain, lung, squamous cell, bladder, gastric, pancreatic, breast, head, neck, renal, kidney, ovarian, prostate, colorectal, endometrial, esophageal, testicular, gynecological and thyroid cancer. Non-cancer hyperproliferative disorders include, but are not limited to, benign hyperplasia of the skin (e.g., psoriasis), restenosis, or prostate (e.g., benign prostatic hypertrophy (BPH)), age-related macular degeneration, Crohn's disease, cirrhosis, chronic inflammatory-related disorders, proliferative diabetic retinopathy, proliferative vitreoretinopathy, retinopathy of prematurity, granulomatosis, immune hyperproliferation associated with organ or tissue transplantation, an immunoproliferative disease or disorder, e.g., inflammatory bowel disease, rheumatoid arthritis, systemic lupus erythematosus (SLE), vascular hyperproliferation secondary to retinal hypoxia, Li Fraumeni, or vasculitis.

Disclosed are peptides for use in the treatment of a hyperproliferative disorder in a patient wherein the hyperproliferative disorder comprises cancer, wherein cancer comprises breast cancer, triple negative breast cancer, ovarian cancer, or any blood cancer.. For example, blood cancers can be any blood cancer with a mutated p53, such as but not limited to, Acute Myeloid Leukemia (AML), Chronic Lymphocytic Leukemia (CLL), Chronic Myeloid Leukemia (CML), B-Cell Chronic Lymphocytic Leukemia (B-CELL).

### I. Peptides for use in the Treatment of Cancer

Disclosed are peptides for use in the treatment of cancer wherein the peptide comprises a partial p53 peptide and a mutated Bcr coiled-coil domain.

Disclosed are peptides for use in the treatment of cancer wherein the peptide comprises a partial p53 peptide and a mutated Bcr coiled-coil domain, wherein the mutated Bcr coiled-coil domain is linked to the C-terminus of the DNA binding domain of the partial p53 peptide. For example, all amino acids after the C-terminus end of the DNA binding domain can be removed from p53 and replaced with a mutated Bcr coiled-coil.

Disclosed are peptides for use in the treatment of cancer wherein the peptide comprises a partial p53 peptide and a mutated Bcr coiled-coil domain, wherein the mutated coiled-coil domain is located between the DNA binding domain of the partial p53 peptide and the C-terminus of the peptide.

Disclosed are peptides for use in the treatment of cancer wherein the peptide comprises a partial p53 peptide and a mutated Bcr coiled-coil domain, wherein the mutated Bcr coiled-coil domain comprises the sequence of SEQ ID NO:5.

Disclosed are peptides for use in the treatment of cancer peptide comprising a partial p53 peptide and a mutated Bcr coiled-coil domain, wherein the peptide comprises the sequence of SEQ ID N0:3. Thus, the peptide can comprise the p53-CCmutE34K-R55E peptide.

Disclosed are peptides for use in the treatment of cancer wherein the peptide comprises a partial p53 peptide and a mutated Bcr coiled-coil domain, wherein the mutated Bcr coiled-coil domain consists of the sequence of SEQ ID N0:5, or active fragments thereof.

Disclosed are peptides for use in the treatment of cancer wherein the peptide comprises a partial p53 peptide and a mutated Bcr coiled-coil domain, wherein cancer comprises breast cancer, triple negative breast cancer, ovarian cancer, or any blood cancer.

Disclosed are peptides for use in the treatment of cancer wherein the peptide comprisesa partial p53 peptide and a mutated Bcr coiled-coil domain, wherein the composition further comprises an anti-cancer agent. For example, the anti-cancer agent can comprise paclitaxel, carboplatin or a combination thereof. Anti-cancer agents are compounds useful in the treatment of cancer. Examples of anti-cancer agents include alkylating agents such as thiotepa and CYTOXAN(R) cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL(R)); beta-lapachone; lapachol; colchicines; betulinic acid; a camptothecin (including the synthetic analogue topotecan (HYCAMTIN(R)), CPT-II (irinotecan, CAMPTOSAR(R)), acetylcamptothecin, scopolectin, and 9-aminocamptothecin); bryostatin; callystatin; CC- 1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosf amide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gammall and calicheamicin omegall (see, e.g., Agnew, Chern Inti. Ed. Engl., 33: 183-186 (1994)); dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN(R), morpholino-doxorubicin, cyanomorpholino-doxorubicin, 2-pyrrolino-doxorubicin, doxorubicin HClliposome injection (DOXIL(R)) and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR(R)), tegafur (UFTORAL(R)), capecitabine (XELODA(R)), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK(R) polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T -2 toxin, verracurin A, roridin A and anguidine); urethane; vindesine (ELDISEME(R), FILDESIN(R)); dacarbazine; mannomustine; mitobronitol; mitolactol; pipobroman; gacytosine; arabinoside ("Ara-C"); thiotepa; taxoids, e.g., paclitaxel (TAXOL(R)), albumin-engineered nanoparticle formulation ofpaclitaxel (ABRAXANE(TM)), and doxetaxel (TAXOTERE(R)); chloranbucil; 6-thioguanine; mercaptopurine; methotrexate; platinum analogs such as cisplatin and carboplatin; vinblastine (VELB AN(R)); platinum; etoposide (VP-16); ifosfamide; mitoxantrone; vincristine (ONCOVIN(R)); oxaliplatin; leucovovin; vinorelbine (NAVELBINE(R)); novantrone; edatrexate; daunomycin; aminopterin; ibandronate; topoisomerase inhibitor RFS 2000; difluorometlhylomithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN(TM)) combined with 5-FU and leucovovin.

In some instances, the peptide comprising a partial p53 peptide and a mutated Bcr coiled-coil domain is in a separate composition from an anti-cancer agent. For example, disclosed are methods of treating cancer comprising administering to a patient a first composition comprising a peptide comprising a partial p53 peptide and a mutated Bcr coiled-coil domain and a second composition comprising an anti-cancer agent. The first composition can be one or more of the compositions disclosed herein. The first and second compositions can be administered together or consecutively. Administering the compositions together includes mixing the two compositions just prior to administration. Administering together also includes administering the separate compositions within one, two, three, four, five, six, seven, eight, nine or ten minutes of each other. Consecutive administration refers to administering the compositions at separate times greater than 10 minutes apart. For example, consecutive administration includes administering one composition at least 10, 15, 20, 25, 30, 60, 120 minutes after the administration of the other composition. In some instances, one composition can be administered 1, 2, 3, 4, 5, 6, 7, 8, 9, 10, 15, 20, 24 hours after administration of the other composition. In some instances, one composition can be administered 1, 2, 3, 4, 5, 6, 7, 8, 9, 10, 11, 12, 13, 14, 21, 28, 29, 30, or 31 days after administration of the other composition. In some instances, one composition can be administered 1, 2, 3, 4, 5, 6, 7, 8, 9, 10, 11, or 12 months after administration of the other composition.

### J. Cells

Also disclosed herein are host cells transformed or transfected with a vector comprising the nucleic acid sequences described elsewhere herein. Also disclosed are host cells comprising the vectors described herein. For example, disclosed is a host cell comprising a vector comprising the nucleic acid sequences described elsewhere herein, operably linked to a control element. Host cells can be eukaryotic or prokaryotic cells. For example, a host cell can be a mammalian cell. Also disclosed are recombinant cells comprising the disclosed nucleic acid sequences or peptides. Further disclosed are recombinant cells producing the disclosed peptides.

Disclosed are recombinant cells comprising one or more of the nucleic acid sequences disclosed herein.

Disclosed are recombinant cells comprising one or more of the nucleic acid sequences capable of producing any of the peptides disclosed herein.

### Examples

### N. Example 1: A Chimeric p53 Evades Mutant p53 Transdominant Inhibition in Cancer Cells

The tumor suppressor p53, a 393 amino acid sequence-specific transcription factor, stimulates a wide network of signals including cell cycle arrest, DNA repair, and apoptosis. p53-dependent apoptosis is achieved through two distinct apoptotic signaling pathways; the extrinsic pathway through death receptors and the intrinsic pathway through the mitochondria. While p53 is able to induce apoptosis when targeted to the mitochondria, its tumor suppressor function mainly depends on localization to the nucleus and formation of p53 tetramers leading to its function as a transcription factor of several target genes. The p53 protein is commonly divided into three regions: an acidic N-terminal region (codons 1-101), a DNA binding domain (DBD, codons 102-292), and a basic C-terminal region (codons 293-393). The C-terminus contains three nuclear localization signals (NLSs), a nuclear export signal (E), and a tetramerization domain (TD) (Figure 1A). In response to cellular stimuli such as DNA damage and oncogene activation, the MDM2-p53 degradation pathway is inactivated leading to increased concentration of p53 followed by rapid accumulation in the nucleus, which is essential for regulating cell cycle arrest, DNA repair, senescence, and apoptosis. Current strategies to enhance the anti-cancer/tumor suppressor function of p53 are focused on introducing additional wt-p53 to the affected cells or tumor. This treatment modality introduces wt-p53 as a gene into cancer cells using various delivery vehicles. Wild-type p53 is a currently approved gene therapeutic for head and neck cancer in China. While a promising approach, there are significant limitations to the efficacy of this method, namely the presence of mutations in the endogenous p53 molecule. The tumor suppressor p53 is inactivated in more than half of all human tumors. Acquisition of missense mutations in the TP53 gene results in aberrant p53 that is transcriptionally inactive. Mutant p53 can also contribute to cancer drug resistance due to its inhibition of wild-type (wt) p53 via a dominant negative effect and the acquisition of gain of function properties. Since p53 binds DNA as a tetramer consisting of a dimer of dimers, when endogenous mutant p53 oligomerizes with exogenous wt-p53 the resulting tetramer is inactive. Such heterotetramerization can occur as the TD retains functionality in mutant p53. This dominant negative effect, wherein mutant p53 inactivates therapeutic wt-p53, represents a key problem with using wt-p53 for gene therapy. The dominant negative effect of p53 has shown to be operative in vivo using knock-in mice expressing mutant p53 20. Because sequestration of wt-p53 into inactive hetero-tetramers with mutant p53 forms a critical barrier to the efficacy of utilizing p53 for cancer therapy, improvements to advance the efficacy of this therapy even in the presence of p53 mutants is needed. This study bypasses the dominant negative effect of tumor-derived p53 is to engineer a p53 variant that relies on a different oligomerization motif to prevent hetero-oligomer formation. Only one attempt has been made to eliminate the dominant negative effect of mutant p53 in hetero-tetramers via substituting its TD, with marginal success. Whereas the native TD of p53 drives the formation of antiparallel tetramers, this previous work utilized an oligomerization domain that led to parallel tetramer formation which resulted in a significant reduction in p53 function. The oligomerization domain from breakpoint cluster region (Bcr) protein, a 72 amino acid coiled-coil (CC), tetramerizes as two dimers of two antiparallel-oriented monomers, in a similar fashion to the TD of wt-p53. This is a candidate for TD substitution, forming a chimeric p53-Bcr fusion. Table 1 depicts the oligomerization domains for p53 (TD) and the CC domain from Bcr. This report demonstrates that the p53 variant, namely p53-CC, shows higher levels of transcriptional activity in reporter gene assays, and exhibits similar tumor suppressor activity compared to wt-p53 in cell lines with varying p53 status. Lastly, the ability of p53-CC to circumvent the dominant negative effect in cancer cells harboring a strong transdominant mutant p53 was shown.

**Table 1. Comparison of the native TD from wt-p53 to the CC domain from Bcr. Snapshots were taken with molecular visualization software PyMOL (PyMOL Molecular Graphics System Version 1.5.0.4 Schrödinger, LLC.) initiated from the 1C26 for p53 TD 22 and 1K1F for Bcr CC 25 PDB structures.**

| | **TD** | **CC** |
|---|---|---|
| **Sequence** | 31 amino acids | 72 amino acids |
| **Secondary Structure** | Consists of a β strand, a tight turn followed by an α helix | Consists of two α helices |
| **Orientation** | Antipavallel | Antiparallel |
| **Tetramer Formation** | Dimer of dimers | Dimer of dimers |
| **Structure** | 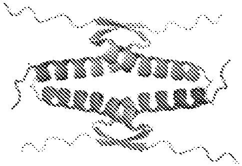 | 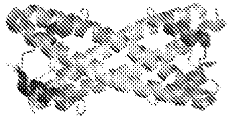 |

### 1. MATERIALS AND METHODS

### i. Construction of Plasmids (Figure 1A)

To construct pEGFP-p53-CC (p53-CC), a truncated version of wt-p53 that lacks the tetramerization domain (amino acids 1-322) was amplified via PCR with primers 5'-GCGCGCGCGCTCCGGAATGGAGGAGCCGCAGTCA-3' and 5'-GCGCGCGCGCTCCGGATGGTTTCTTCTTTGGCTGGGGAGA-3' using the previously cloned pEGFP-p53 (wt-p53) as the template DNA 4. The PCR product was then subcloned into the BspEI site of pEGFP-CC (CC)

To create pEGFP-p53-ΔTDC (p53-ΔTDC), the same truncated version of wt-p53 (amino acids 1-322) was amplified via PCR with primers 5'-GCGCGCGCGCTCCGGAATGGAGGAGCCGCAGTCA-3' and 5'-GCGCGCGCGCGGTACCTCATGGTTTCTTCTTTGGCTGGGG-3' using pEGFP-p53 as the template DNA 4. The PCR product (insert) was then subcloned into the digested pEGFP-C1 vector (Clontech, Mountain View, CA) at the BspEI and KpnI sites.

To design pTagBFP-mut-p53, wt-p53 was amplified via PCR with primers 5'-GCGCGCGCGCTCCGGAGCCATGGAGGAGCCGCAGT-3'(SEQ ID NO:1), and 5'-GCGCGCGCGCGGTACCTCAGTCTGAGTCAGGCCCTTCTGTC-3' (SEQ ID NO:2) using pEGFP-p53 as a template. This insert was then subcloned into the digested pTagBFP-C vector (Evrogen, Moscow, Russia) at the BspEI and KpnI sites. Three hot spot mutations (R175H, R248W, and R273H) were then introduced into pTagBFP-p53 via QuikChange II XL Site-Directed Mutagenesis Kit (Agilent, Santa Clara, CA). The following primers were used: for the R175H mutation, 5'-TGACGGAGGTTGTGAGGCACTGCCCCCACCATGAGCGC-3' (SEQ ID NO:23) and 5'-GCGCTCATGGTGGGGGCAGTGCCTCACAACCTCCGTCA-3'(SEQ ID NO:24); for R248W, 5'- CTGCATGGGCGGCATGAACTGGAGGCCCATCCTCACCA-3' (SEQ ID NO:25) and 5'-TGGTGAGGATGGGCCTCCAGTTCATGCCGCCCATGCAG-3'(SEQ ID NO:26); and for R273H, 5'-GGAACAGCTTTGAGGTGCATGTTTGTGCCTGTCCTGGG-3' (SEQ ID NO:27) and 5'-CCCAGGACAGGCACAAACATGCACCTCAAAGCTGTTCC-3' (SEQ ID NO:28).

### ii. Cell Lines and Transient Transfection

T47D human ductal breast epithelial tumor cells (ATCC, Manassas, VA), MCF-7 human breast adenocarcinoma cells (ATCC), HeLa human epithelial cervical adenocarcinoma cells (ATCC), H1373 human non-small cell lung carcinoma cells, and MDA-MB-231 human breast adenocarcinoma cells (ATCC) were grown as monolayers in RPMI (Invitrogen, Carlsbad, CA) supplemented with 10% fetal bovine serum (Invitrogen), 1% penicillin-streptomycin-glutamine (Invitrogen), 0.1% gentamicin (Invitrogen). T47D and MCF-7 were also supplemented with 4 mg/L insulin (Sigma, St. Louis, MO). 1471.1 murine breast adenocarcinoma cells, HEK293 human embryonic kidney (ATCC), MDA-MB-468 human breast adenocarcinoma cells (ATCC), and 4T1 murine breast carcinoma cells were grown as monolayers in DMEM (Invitrogen) supplemented with 10% fetal bovine serum, 1% penicillin-streptomycin-glutamine, and 0.1% gentamicin. MDA-MB-468 cells were also supplemented with 1% MEM non-essential amino acids (Invitrogen). All cells were incubated in 5% CO2 at 37°C. The cells were seeded at a density of 7.5 × 10⁴ cells (for 1471.1, MDA-MB-231, HeLa, and 4T1 cells) and 3.0 × 10⁵ cells (for MCF-7, T47D, HEK293, MDA-MB-468 and H1373 cells) in 6-well plates (Greiner Bio-One, Monroe, NC). Transfections of 1 pmol DNA were carried out 24 h after seeding using Lipofectamine 2000 (Invitrogen) following the manufacturer's recommendations.

### iii. Microscopy

All microscopy was performed using 1471.1 cells due to their ideal microscopic morphology. 24 h post transfection, media in 2-well live-cell chambers (Nalgene Nunc, Rochester, NY) was replaced with phenol red-free DMEM (Invitrogen). Cells were then incubated with 2 µg/mL Hoechst 33342 nuclear stain (Invitrogen) for 30 minutes at 37°C. Images were taken using an Olympus IX71F fluorescence microscope (Scientific Instrument Company, Aurora, CO) with high-quality narrow band GFP filter (excitation, HQ480/20 nm; emission, HQ510/ 20 nm) to detect EGFP and cyan GFP v2 filter (excitation HQ436/20 nm, emission HQ480/40 nm, with beam splitter 455dclp) to detect H33342.

### iv. RT-PCR

24 h following transfection of T47D cells, mRNA from cell lysates was isolated using RNeasy® Mini Kit (Qiagen, Valencia, CA). cDNA was then obtained using RT2® First Strand Kit (Qiagen) and mixed with RT SYBR® Green qPCR Mastermix (Qiagen). Equal volumes were then aliquoted into a 384-well p53 Signaling Pathway PCR Array® (Qiagen). Roche LightCycler 480 was used for real-time PCR cycling. Analysis of the PCR array was performed using the manufacturer's web-based analysis software (pcrdataanalysis.sabiosciences.com/pcr/arrayanalysis.php). Genes outside of a 2-fold range were considered to be statistically different per the manufacturer.

### v. Western Blotting

24 h following transfection of T47D cells, EGFP-positive cells were sorted using the FACSAria-II (BD-BioSciences). 3 x 10⁵ cells were pelleted and resuspended in 200 µL lysis buffer (62.5 mM Tris-HCl, 2% w/v SDS, 10% glycerol, 1 % protease inhibitor). Standard western blotting procedures were followed using primary antibodies to detect p21/WAF1, Bax, and actin as a loading control. The primary antibodies anti-p21 (ab16767, Abcam, Cambridge, MA), anti-Bax (ab7977, Abcam), anti-actin (mouse, ab3280, Abcam), and anti-actin (rabbit, ab1801, Abcam) were detected with anti-rabbit (#7074S, Cell Signaling Technology, Danvers, MA) or anti-mouse (ab6814, Abcam) HRP-conjugated antibodies before the addition of SuperSignal West Pico chemiluminescent substrate (Thermo Scientific, Waltham, MA). Signals were detected using a FluorChem FC2 imager and software (Alpha Innotech, Sanata Clara, CA).

### vi. TUNEL Assay

T47D cells were prepared 48 h after transfection using In Situ Death Detection Kit, TMR red (Roche, Mannheim, Germany). Cells were EGFP gated and analyzed using FACSAria-II (BD-BioSciences, University of Utah Core Facility) and FACSDiva software. EGFP and TMR red were excited at 488 nm and 563 nm wavelengths and detected at 507 nm and 580 nm, respectively. The TUNEL assay was repeated three times (n=3) and analyzed using one-way ANOVA with Bonferroni's post hoc test.

### vii. Annexin-V Assay

Briefly, 48 h post transfection, T47D cells were suspended in 400 µl annexin binding buffer (Invitrogen) and incubated with 5 µl annexin-APC (annexin-V conjugated to allophycocyanin, Invitrogen) for 15 minutes. The incubated cells were EGFP gated and analyzed using FACSCanto-II. EGFP and APC were excited at 488 nm and 635 nm wavelengths and detected at 507 nm and 660 nm, respectively. Each construct was tested three times (n=3) and analyzed using one-way ANOVA with Bonferroni's post hoc test.

### viii. 7-AAD Assay

Following manufacturer's instructions, T47D, MCF-7, H1373, and MDA-MB-468 cells were stained with 7-aminoactinomycin D (7-AAD, Invitrogen) 48 h after transfection. Since HeLa, MDA-MB-231, and 4T1 cells are highly proliferating cells, these cell lines were assayed 24 h post transfection. Cells were analyzed and gated for EGFP (with same fluorescence intensity to ensure equal expression of proteins) using the FACSCanto-II (BD-BioSciences,) and FACSDiva software. Excitation was set at 488 nm and detected at 507 nm and 660 nm for EGFP and 7-AAD, respectively. The means from three separate experiments (n=3) were analyzed using one-way ANOVA with Bonferroni's post hoc test.

### ix. Colony Forming Assay (CFA)

CFA was carried out using the Cytoselect® 96-well cell transformation assay (Cell Biolabs, San Diego, CA). A base agar layer was prepared per manufacturer's directions, and 50 µL was transferred to each well of a clear-bottom 96-well plate. T47D cells were transfected as described above with wt-p53, p53-CC, or CC and harvested 24 h post transfection. The cells were resuspended in RPMI medium (Invitrogen) at a concentration of 3.0 x 10⁵ cells/mL per the manufacturer's instructions. A cell agar layer was then prepared as recommended, and 75 µL of the mixture was transferred to each well of the 96-well plate containing the base agar layer. 100 µL of complete culture medium was added to each well; plates were then incubated at 37°C and 5% CO2 for 7 days. The culture medium was removed, solubilized, and lysed. 10 µL of cell lysates were then transferred to a new black-bottom 96-well plate. 90 µL of CyQuant GR dye working solution (1:400 in PBS) was added to each well and incubated for 10 min at RT. A Spectra Max M2 plate reader (Molecular Devices, Sunnyvale, CA) was used to detect fluorescence using a 485/520 nm filter set. Independent transfections of each construct were tested three times (n=3) and analyzed using one-way ANOVA with Bonferroni's post hoc test.

### x. Reporter Gene Assay

3.5 µg of construct (wt-p53, p53-CC, CC, or EGFP) was co-transfected with 0.35 µg of pRL-SV40 plasmid encoding for Renilla luciferase (Promega, Madison, WI) to normalize for transfection efficiency in T47D cells. In addition to Renilla luciferase, constructs were co-transfected with 3.5 µg of p53-Luc Cis-Reporter (Agilent Technologies, Santa Clara, CA), p21/WAF1 reporter (a generous gift from Dr. Bert Vogelstein, Addgene plasmid 16451), or PUMA reporter (Addgene plasmid 16591); all 3 reporters encode the firefly luciferase gene. The Dual-Glo Luciferase Assay System (Promega) was used to determine firefly luciferase activity and Renilla luciferase per manufacturer's instructions. Luminescence from active luciferase was then detected using PlateLumino (Stratec Biomedical Systems, Birkenfeld, Germany). Renilla luciferase activity was used to normalize the firefly luciferase values. The highest relative luminescence value was set at 100% and untreated cells were set at 0%. The means from triplicate samples were taken from 3 independent experiments and analyzed using one-way ANOVA with Bonferroni's post hoc test.

### xi. Co-Immunoprecipitation (co-IP)

The co-IP was performed using Dynabeads co-IP Kit (Invitrogen). T47D cells transfected with either EGFP-wt-p53 or EGFP-p53-CC were collected and weighed out (0.05 g) 20 h post transfection. Anti-GFP antibody (ab290, Abcam) was coupled to magnetic beads using Dynabeads Antibody Coupling Kit (Invitrogen). Approximately 0.2 g of cell pellet was lysed in 1.8 ml extraction buffer B (1x IP, 100 mM NaCl, 2 mM MgC12, 1 mM DTT, 1 % protease inhibitor). The lysate was incubated for 30 min at 4°C with 1.5 mg of the dynabeads coupled with anti-GFP antibody. The immune complexes were then collected by a magnet and washed three times with extraction buffer B and one time with last wash buffer (1x LWB, 0.02% Tween 20). Immune complexes were then eluted using 60 µl elution buffer. Finally, the eluted complexes were denatured and blotted using anti-p53 antibody HRP-conjugated (sc-126 HRP, Santa Cruz Biotechnology, Santa Cruz, CA).

### xii. Overexpression of Mutant p53

H1373 cells were cotransfected with 1 pmol of the transdominant mutant pTagBFP-mut-p53 (R175H, R248W, and R273H) and 1 pmol of wt-p53, p53-CC, or CC fused to EGFP. 48 h post transfection, cells were stained as in the 7-AAD assay above and gated for EGFP and BFP using the FACSCanto-II (BD-BioSciences) and FACSDiva software. Excitation for BFP was set at 405 nm and detected at 457 nm. The means from three separate experiments (n=3) were analyzed using one-way with Bonferroni's post hoc test and unpaired t test.

### xiii. Recombinant Adenovirus Production

Replication-deficient recombinant adenovirus serotype 5 (Ad) constructs were generated using the Adeno-X® Adenoviral Expression System 3 (Clontech). Either wt-p53 or p53-CC was inserted into a cassette under the control of the CMV promoter. A separate CMV promoter controls the expression of ZsGreenl for visualization. The empty virus (vector) was used as a negative control. Wt-p53 and p53-CC were PCR amplified with primers containing 15 base pair homology with a linearized pAdenoX vector (Clontech) based on an In-Fusion® HD Cloning Kit (Clontech). Stellar® competent cells (Clontech) were transformed with the adenoviral vector plasmids containing the constructs. Viral DNA was then purified, linearized and transfected into HEK293 cells for packaging and amplification. Viral particles were isolated from HEK293 cells by freeze-thawing, purified using Adeno-X® Mega Purification Kit (Clontech), and dialyzed against storage and proper tonicity buffer (2.5% glycerol (w/v), 25 mM NaCl, and 20 mM Tris-HCl, pH 7.4). The viral titer was determined using flow cytometry per the manufacturer's recommendation.

### 2. RESULTS

### i. p53-CC localizes to the nucleus.

Because the nuclear localization of p53 is important for anti-apoptotic function, the study first examined if p53-CC also localized to the nucleus. Full length wt-p53 contains three NLSs encoded by amino acids 305-322, 370-376, and 380-386 (Figure 1A, top). Given that p53-CC (illustrated in Figure 1A) lacks most of the C-terminal domain (amino acids 323-393), which contains two NLSs, nuclear accumulation of p53-CC was verified using fluorescence microscopy (Figure IB). Both wt-p53 and p53-CC were fused to EGFP to enable visualization of the subcellular localization of each protein. Figure 1B shows similar nuclear accumulation of p53-CC and wt-p53 in 1471.1 murine adenocarcinoma cells. CC alone fused to EGFP showed mostly cytoplasmic localization. Similar results were obtained in T47D and MCF-7 breast cancer cells.

### ii. Wt-p53 and p53-CC show similar gene expression profiles.

After verifying the nuclear localization of p53-CC via fluorescence microscopy, the activity of p53-CC was investigated next. The Human p53 Signaling Pathway RT² Profiler™ PCR Array (QIAGEN, Valencia, CA) was used to compare the transcription profiles between wt-p53 and p53-CC in T47D human breast cancer cells. T47D cells contain mutant p53 (a L194F mutation) that does not exhibit a strong transdominant effect. Exogenously added wt-p53 has been shown to be functional in this cell line 4,30, and hence these cells can be used for comparing wt-p53 activity with p53-CC. The PCR array uses real-time PCR to measure the expression profiles of 84 genes directly related to p53-mediated signal transduction, including genes involved in apoptosis, cell cycle, DNA repair, cell proliferation, and differentiation. Analysis of the PCR array indicated that p53-CC showed a similar expression profile of 83 out of 84 genes compared to wt-p53 (Figure 2A), with the exception of the p53AIPl gene (circled in black), whose protein product is one of many involved in the intrinsic apoptotic pathway. A tetramerization-deficient form of p53 (p53-ΔTDC) was included as a negative control in these assays to validate that the activity of p53-CC is due to proper tetramer formation, along with CC (also a negative control). As expected, both p53-ΔTDC and CC had significantly different expression profiles from wt-p53 (Supplementary Figures 1 and 2) in the p53 signaling pathway PCR array.

To verify the array results, the protein expression of two typical genes involved in two different pathways that are directly regulated by p53, Bax and p21/WAF1, were examined by western blotting. Bax is involved in the p53-dependent intrinsic apoptosis pathway, while p21/WAF1 is involved in cell cycle arrest 35. Figure 2B shows that T47D cells transfected with wt-p53 (first lane) or p53-CC (second lane) demonstrated overexpression of Bax and p21, while the monomeric form of p53 (p53-ΔTDC, third lane) and the CC (fourth lane) negative controls did not significantly induce expression of the Bax and p21/WAF1. Faint p21/WAF1 bands are observed with negative controls and represent background levels of this protein. Due to its inactivity, p53-ΔTDC was not included in the remaining apoptotic assays.

### iii. p53-CC exhibits tumor suppressor activity.

To determine if the similar gene expression profiles between p53-CC and wt-p53 correlate with comparable tumor suppressor activity, the apoptotic potential (TUNEL, annexin V, 7-AAD) and transformative ability (colony formation) were tested in T47D cells. As mentioned before, T47D cells were chosen to compare the activity of p53-CC and wt-p53, since it was shown before that wt-p53 is active in these cells.

The TUNEL (terminal deoxynucleotidyl transferase dUTP nick end labeling) assay, which measures DNA fragmentation into nucleosomal segments, is a hallmark of apoptosis 36. Figure 3A shows that p53-CC has a similar ability to induce DNA fragmentation as wt-p53 compared to CC control. Next, the apoptotic potential of p53-CC was also validated in the annexin-V assay, which evaluates the externalization of phosphatidylserine on the cell surface of apoptotic cells. Similar levels of annexin-V positive staining were detected between cells transfected with p53-CC and wt-p53 (Figure 3B), and were significantly higher than positive staining in cells transfected with CC negative control. The last apoptotic assay tested was the 7-AAD viability assay. In apoptotic or necrotic cells, the plasma membrane is disrupted allowing intercalation of the 7-AAD stain into DNA in the nucleus of these damaged cells. In this assay, p53-CC maintains the same level of apoptotic activity as wt-p53, and is able to induce higher levels of cell death compared to the control (CC). Finally, the decrease in transformative ability (or oncogenic potential) of cells treated with p53-CC or wt-p53 were tested via a colony forming assay. In this assay, treatment with a tumor suppressor is expected to reduce the number of cell colonies formed in an agar matrix. Indeed, as shown in Figure 3D, both p53-CC (second bar) and wt-p53 (first bar) significantly reduced the number of colonies formed compared to the negative controls (CC and untreated cells, third and fourth bars, respectively). Overall, these results indicate that p53-CC shows similar ability to induce statistically significant levels of apoptosis and reduce oncogenic potential as wt-p53.

To ensure that the potential for p53-CC to induce apoptosis is neither dependent on endogenous p53 status nor cancer cell line specific, p53-CC was tested in several different cell lines. Human epithelial cervical adenocarcinoma cells (HeLa), which express endogenous wt-p53 41, MDA-MB-231 metastatic triple-negative breast cancer cells harboring mutant p53 42, MCF-7 breast cancer cells with wild type but mislocalized p53 43, and H1373 non-small cell lung carcinoma cells that are p53 null 44 (Table 2), were tested in the 7-AAD assay. In all four cell lines, p53-CC and wt-p53 were able to induce similar levels of apoptosis, and were higher than the negative control (CC), as shown in Figures 4A-D.

**Table 2. Comparison of the four different cell lines (HeLa, MDA-MB-231, MCF-7 and H1373) in terms of p53 status and cancer type.**

| **Cell Line** | **p53 Status** | **Cancer Type** | **Ref** |
|---|---|---|---|
| **HeLa** | Wild-type | Cervical adenocarcinoma | [40] |
| **MDA-MB-231** | Mutated (R280K) | Triple-negative breast cancer | [41] |
| **MCF-7** | Mislocalized to cytoplasm | Breast cancer | [42] |
| **H1373** | Null | Non-small cell lung carcinoma | [43] |

### iv. p53-CC maintains transcriptional activity of p53 target genes.

While p53-CC exhibited similar apoptotic activity as wt-p53, the study examined whether p53-CC was capable of activating promoters of p53-dependent target genes. Tetramerization of p53 is a prerequisite to transcriptional activity, thus transcriptional activation indicates tetramerization ability (of both wt-p53 and p53-CC) 45. The transcriptional activity of p53-CC was tested in T47D cells using three different reporter gene assays. The first was the p53 cis-reporter system, a common reporter for measuring p53 activity, which relies on a synthetic promoter consisting of repeats of the transcription recognition consensus for p53 (TGCCTGGACTTGCCTGG)14 46. The second and third reporter systems utilized the binding consensus sequences from p21/WAF1 and PUMA promoters, respectively. p21/WAF1 is a cyclin-dependent kinase inhibitor that mediates p53-dependent G1 cell cycle arrest 31,35, while PUMA translocates to the mitochondria, deactivates antiapoptotic Bcl-2 and Bcl-XL proteins and induces p53-dependent apoptosis 47. In all three reporter gene assays, p53-CC showed higher transcriptional activity compared to wt-p53, and both were higher than the negative controls CC and EGFP (Figures 5A-C) in T47D cells.

### v. p53-CC avoids interaction with endogenous p53.

The higher level of transcriptional activity of p53-CC over wt-p53 was due to the hetero-oligomerization of wt-p53 with endogenous mutant p53 in this cell line. Therefore, a co-IP assay was performed to determine if exogenously added wt-p53 interacts with mutant p53 present in these cells. To this end, mutant p53 in T47D cells are not expected to co-immunoprecipitate with p53-CC. Cell lysates transfected with either EGFP-wt-p53 or EGFP-p53-CC were incubated with anti-GFP antibody to selectively immunoprecipitate the fusion EGFP proteins (Figure 5D). Endogenous p53 that can potentially co-immunoprecipitate with either exogenous EGFP-wt-p53 or EGFP-p53-CC was probed using anti-p53 antibody. Figure 5D shows that endogenous p53 (53 kDa) co-immunoprecipitates with exogenous wt-p53 (left lane, 70 kDa) but fails to immunoprecipitate with p53-CC (right lane, 71 kDa). These findings indicate that endogenous p53 interacts directly with exogenous wt-p53, which is presumably due to hetero-oligomerization via their TDs. As expected, p53-CC, which lacks the native TD, evaded binding to endogenous p53. A prominent secondary band is normally detected by this anti-p53 antibody at about 69 kDa (per Santa Cruz Biotechnology).

### vi. Bypassing the dominant negative effect.

Since p53-CC did not interact with endogenous wt-p53 in the co-IP assay, the ability of p53-CC to bypass the dominant negative effect was tested, first using overexpression of a dominant negative mutant p53 in H1373 cells (p53 null), and second, in MDA-MB-468 cells that harbor a strong dominant negative p53 mutant. The ability of p53-CC to "rescue" the loss of apoptotic activity induced by an inactive mutant p53 in H1373 (p53 null) cells was tested; Figure 6A shows that in the absence of the inactive mutant p53 (first 3 sets of bars), both p53-CC and wt-p53 can similarly induce apoptosis (measured by 7-AAD) compared to the negative CC control. However, when a transdominant mutant p53 is added (bars 4-6), only p53-CC is able to rescue apoptotic activity, while wt-p53 cannot. This transdominant mutant p53 was engineered by combining three hotspot mutations (R175H, R248W, and R273H) that are known to exhibit a dominant negative effect 27, 28. This supports the notion that p53-CC can bypass the dominant negative effect of a transdominant mutant p53. To further investigate this, the ability of p53-CC to induce apoptosis was tested in a cell line known to contain an endogenous strong transdominant mutant form of p53, MDA-MB-468. The endogenous p53 in MDA-MB-468 contains the R273H point mutation that is known to exhibit transdominant inhibition of wt-p53, so exogenous wt-p53 in this case is expected to have limited activity. MDA-MB-468 cells are resistant to transient transfection with lipofectamine (used in the majority of these studies), so instead, they were transduced with adenovirus (Ad) constructs carrying the wt-p53 or p53-CC as genetic cargo. Figure 6B shows that indeed, only Ad-p53-CC (second bar) is able to significantly induce apoptotic activity measured by 7-AAD compared to wt-p53 and empty Ad vector (bars 1 and 3). This indicates that the transdominant effect of endogenous mutant p53 found in MDA-MB-468 cells can be circumvented by using an oligomerization variant of p53, namely p53-CC. Finally, adenovirally delivered p53-CC was tested in a p53 null cell line (Figure 6C), where both wt-p53 and p53-CC is active. Indeed, as shown in Figure 6C, both constructs are active in this cell line. p53-CC is more active than wt-p53 in this particular cell line.

### 3. DISCUSSION

To summarize, a version of p53 with an alternative tetramerization domain localizes to the correct subcellular compartment (the nucleus, Figure IB), and shows a similar gene expression profile as wt-p53 (Figure 2A). Two genes regulated by p53, Bax and p21/WAF1, also showed similar protein expression levels when induced by p53-CC or wt-p53, as demonstrated by western blotting (Figure 2B). Tumor suppressor activity, measuring apoptotic activity (by TUNEL, annexin V, and 7-AAD) and reduced oncogenic potential (reduced number of colonies), Figure 3A-D, was similar between p53-CC and wt-p53. Importantly, p53-CC was found to induce statistically significant levels of apoptosis in 4 different cell lines (Figure 4A-D), regardless of p53 status, indicating that p53-CC activity is not dependent on p53 status, nor is it cell-line specific (see Table 2). The transcriptional activity of p53-CC was tested using 3 reporter gene assays in Figures 5A -C (a standard p53 reporter gene, a p21/WAFl reporter involved in cell cycle arrest, and a PUMA reporter involved in apoptosis), and in all 3 cases, was higher than wt-p53. In T47D cells, the transcriptional activity of p53-CC was higher than wt-p53 in these reporter gene assays (Figures 5A-C), while the apoptotic activity of p53-CC was similar to wt-p53 (Figures 3A-C). This is not unexpected, since transcriptional activity does not necessarily linearly correlate with apoptotic activity. Transcriptional activity of target genes is a prerequisite step prior to the apoptotic cascade; if a threshold of transcriptional activity is met, the downstream measure of apoptosis cannot change significantly. The negative controls (p53ΔTDC and CC) did not have activity in binding, nor was able to express apoptotic or cell cycle arrest genes.

A co-IP was performed and showed that p53-CC did not interact with endogenous p53 (Figure 5D). Since there was no interaction between p53-CC and endogenous p53, the ability of p53-CC to bypass the dominant negative effect was tested, first with transdominant mutant p53 overexpression (Figure 6A), and second, in MDA-MB-468 cells that harbor a tumor-derived endogenous transdominant negative p53 mutant (Figure 6B). In both cases, p53-CC appears to not be effected by this endogenous transdominant inhibition. Finally, adenovirally delivered p53-CC was also tested in a p53 null cell line, and was active, as expected (Figure 6C).

Mutant p53 retains its tetramerization capability since its TD remains intact, and can form inactive p53 tetramers upon the introduction of exogenous wt-p53 in cancer cells (Figure 7, left side). These hetero-tetramers have a significantly reduced transcriptional activity compared to homo-tetramers of p53-CC. Such a phenomenon gives rise to a great barrier that limits the utility of p53 for cancer therapy. As an approach to prevent hetero-oligomerization, swapping the TD with an alternative oligomerization domain was examined (Table 1). The CC from Bcr tetramerizes in a similar fashion as the TD; both form dimers of two antiparallel-oriented monomers. Only one attempt at substituting the TD of p53 to eliminate the dominant negative effect of mutant p53 in hetero-tetramers has been made, with marginal success. This previous work utilized an oligomerization domain that leads to parallel tetramer formation, whereas the native TD of p53 drives the formation of antiparallel tetramers. This may offer an explanation to the significant reduction in p53 function observed in their published activity assays compared to wt-p53. On the other hand, these results show that p53-CC evades hetero-oligomerization with mutant p53, allowing it to retain the full tumor suppressor function of wt-p53. Figure 7 illustrates bypassing the dominant negative effect with p53-CC (right side), which maintains functional tumor suppressor activity.

Bcr, from which the CC was obtained, is a ubiquitous eukaryotic phosphotransferase protein that can have a role in general cell metabolism. p53-CC can interact with Bcr via its CC domain. This is unlikely due to the compartmentation of Bcr (found in the cytoplasm) vs. p53-CC (found in the nucleus, shown in Figure IB). Bcr-knockout mice still survive; the major defect in these mice was reduced intimal proliferation in low-flow carotid arteries compared to wt mice. Bcr has mostly been studied in the context of chronic myeloid leukemia (CML) where a reciprocal chromosomal translocation with Abl results in the fusion protein Bcr-Abl, the causative agent of CML. The activity of Bcr-Abl is largely due to the constitutive activation of the Abl portion of the molecule. Generally, Bcr can be involved in inflammatory pathways and cell proliferation. The isolated Bcr coiled-coil does not in itself induce apoptosis. Nevertheless, potential inadvertent interaction with the CC oligomerization domain of Bcr via any introduced p53-CC is currently being addressed by introducing mutations in the CC domain of p53-CC that disfavor interactions with Bcr-CC.

Besides not interacting with endogenous p53, the elevation in p53-CC transcriptional activity can also be due to a higher stability of the p53-CC tetramer compared to wt-p53 tetramer. Melting temperature (Tm) for CC is about 83°C 26, which is slightly higher than the Tm for TD around 75°C at physiological pH. In fact, CC forms homo-dimers in thermal denaturation studies. However, further experiments are needed to definitively prove the biochemical tetramerization of p53-CC.

Unlike wt-p53, p53-CC can circumvent transdominant inhibition of mutant p53, illustrating the potential of using p53-CC as an alternative to wt-p53 for cancer gene therapy. Since the dominant negative effect of mutant p53 in cancer cells is currently one of the barriers limiting the use of p53 in cancer gene therapy, this study offers an alternative to overcome this barrier by swapping the TD of p53 with an alternative oligomerization domain while maintaining the tumor suppressor activity. This designed p53-CC can cause apoptosis in many types of cancers, especially in tumors with transdominant mutant p53, where wt-p53 has proven to be ineffective. Ultimately, the p53-CC construct was utilized as a gene therapeutic delivered using an adenoviral vector that can replace the current limited utility of wild-type p53 as a cancer therapeutic.

### O. Example 2: Re-engineered p53 Activates Apoptosis In Vivo and Causes Primary Tumor Regression in A Dominant Negative Breast Cancer Xenograft Model

The ability of p53 to achieve tumor suppressor function depends on formation of p53 tetramers to act as a transcription factor of several target genes. Once activated, p53 stimulates a wide network of signals including DNA repair, cell cycle arrest, and apoptosis. The significance of p53 function is highlighted by the correlation of its inactivity and malignant development. Inactivation of p53 pathway is reported in more than half of all human tumors and can be achieved via several mechanisms including nuclear exclusion and hyperactivation of MDM2, the main regulator of p53 function. However, acquisition of missense mutations in one or both alleles of the TP53 gene remains the most common mechanism of p53 inactivation. The majority of these mutations take place in the DNA binding domain (DBD) which is responsible for p53 interaction with DNA. Although mutant p53 in cancer cells may have impaired tumor suppressor function and transcriptional activity, it retains its ability to oligomerize with other mutant or wild-type (wt) p53 via the tetramerization domain (TD). When mutant p53 oligomerizes with wt-p53 through hetero-oligomerization, the resulting tetramer has impaired function in most cases due to transdominant inhibition by mutant p53 (Figure 8). The outcome of this transdominant inhibition varies significantly based on the type of mutant p53 present in cells. This phenomenon is known as the dominant negative effect of mutant p53 and gives rise to a critical barrier to utilizing wt-p53 for cancer gene therapy.

A new, chimeric superactive p53 with the following activity was designed: wt-p53 like functional transcriptional activity; promotion of improved, highly potent p53-dependent apoptosis; and circumvention of the dominant negative inactivating effect of endogenous mutant p53 in cancer cells. To this end, a chimeric p53 (p53-CC) was engineered that has an alternative tetramerization domain and showed its ability to escape transdominant inhibition by mutant p53 in vitro. The Bcr coiled-coil (CC) alternative oligomerization domain of p53-CC evades hetero-oligomerization with endogenous mutant p53, and hence, bypasses the dominant negative effect reported in cancer cells. The CC domain itself was tested as a control previously, and was found to be non-toxic. p53-CC activity was found to retain similar tumor suppressor activity compared to exogenous wt-p53 in several cancer cell lines harboring different p53 statuses (null, wt, wt mislocalized, and mutant non-dominant). Finally, potential transdominant inhibition of p53-CC and wt-p53 via co-expression of a potent dominant negative mutant p53 was investigated. p53-CC retained the same levels of activity regardless of the presence of transdominant mutant p53, while wt-p53 showed loss of activity.

In this report, the superior tumor suppressor activity of p53-CC in vitro and in vivo in MDA-MB-468, an aggressive p53-dominant negative breast cancer cell line was shown. Furthermore, the underlying differential mechanisms of activity for p53-CC and wt-p53 in the MDA-MB-468 tumor model were demonstrated. The viral-mediated gene therapy approach succeeds in demonstrating the effects of the transdominant effect of endogenous mutant p53 over p53-CC and wt-p53.

### 1. RESULTS

### i. p53-CC induces higher levels of cell death compared to wt-p53

The MDA-MB-468 human breast adenocarcinoma cell line serves as a suitable dominant negative mutant p53 model for testing the effect of p53-CC and wt-p53. The endogenous p53 in MDA-MB-468 contains the R273H point mutation, which is known to exhibit a dominant negative effect over wt-p53. p53-CC is capable of inducing cell death in this as well as other cancer cell lines, regardless of endogenous p53 status. Figure 8 illustrates the superior tumor suppressor function of p53-CC over wt-p53 in a 7-AAD viability assay which stains apoptotic and necrotic cells (compare Figure 9A vs. 9B). Wt-p53 activity is not significantly different from that achieved by the negative control Ad-ZsGreen1 (Figure 9B vs. 9C). This observation illustrates the dominant negative effect of endogenous mutant p53 over wt-p53 in cancer cells. These results are summarized in Figure 9D.

### ii. p53-CC caused cell death via the apoptotic pathway

Figure 9 indicates that the MDA-MB-468 cell line is a suitable tumor model to test the impact of the dominant negative effect of mutant p53 in vivo. Preceding animal studies, the mechanism of cell death were explored. Thus, three different apoptosis assays, the TMRE assay (analogous to the JC-1 assay), activated caspase-3/7 assay, and annexin-V staining were carried out.

Mitochondrial depolarization as measured by loss in TMRE intensity correlates with an increase in mitochondrial outer membrane permeabilization (MOMP). TMRE is a cationic, cell-permeant, and fluorescent dye that rapidly accumulates in mitochondria of living cells due to the negative mitochondrial membrane potential (ΔΨm) of intact mitochondria compared to cytosol. Mitochondrial depolarization results in loss of TMRE from mitochondria and a decrease in mitochondrial fluorescence intensity (FI), illustrated as %MOMP induction in Figure 10A. Figure 10A demonstrates that p53-CC induced significantly higher levels of mitochondrial membrane permeabilization, a hallmark of intrinsic apoptosis, compared to wt-p53. Wt-p53 also induced mitochondrial membrane permeabilization, although not to the same extent as p53-CC. MOMP indicates that cells are transitioning to an apoptotic state.

To further investigate the apoptotic activity of p53-CC and wt-p53, a flow cytometry-based assay to detect the levels of activated caspase-3/7 in MDA-MB-468 cells was performed (Figure 10B). Caspase-3/7 activation is downstream from mitochondrial outer membrane permeabilization in the intrinsic apoptotic pathway and plays a central role at the execution-phase of cell apoptosis. Figure 10B shows that cells treated with p53-CC display increased levels of active caspase-3/7 compared to those treated with wt-p53 or the negative control Ad-ZsGreen1.

Finally, annexin-V staining was performed, which measures externalization of phosphatidylserine on the cell surface of apoptotic cells specifically. Figure 9C shows higher levels of annexin-V positive staining in cells treated with Ad-p53-CC compared to Ad-wt-p53; wt-p53 apoptotic activity was not significantly different from the negative control Ad-ZsGreenl. Cellular apoptosis as indicated in Figure 9C parallel the results from the 7-AAD staining in Figure 8.

To summarize, Figures 9A, B, and C show MDA-MB-468 cells treated with Ad-p53-CC undergo significant apoptosis, validating p53-CC as a potent candidate for gene therapy. The levels of apoptosis induced by Ad-p53-CC are statistically significant compared to that of Ad-wt-p53 or Ad-ZsGreenl in all three apoptosis assays (Figure 9) in addition to the 7-AAD assay (Figure 8).

### iii. In vivo efficacy in a dominant negative breast cancer animal model

MDA-MB-468 human breast adenocarcinoma represents an aggressive breast cancer cell line characterized as triple negative due to the absence of molecular targets including estrogen receptor, progesterone receptor, and human epidermal growth factor receptor 2. In addition, MDA-MB-468 cells harbor a dominant negative mutant p53 capable of impairing the function of wt-p53. This cell line was used to induce orthotopic breast tumors in mice to compare the impact of the dominant negative effect of mutant p53 on the biological activity of p53-CC and wt-p53 in viral-mediated gene therapy. Because of the presence of the coxsackievirus and adenovirus receptor (CAR), MDA-MB-468 cells can be transfected by adenovirus.

Induced in the mammary fat pad of female athymic nu/nu mice, MDA-MB-468 tumors orthotopic engraftment fosters tumorigenesis to occur in the appropriate macro- as well as microenvironment mimicking the environment of human MDA-MB-468 tumors. Due to this, MDA-MB-468 is a commonly used xenograft model for triple negative breast cancer. Tumors were allowed to grow to approximately 50 mm3 prior to randomization of treatment groups which received intratumoral injections of Ad-p53-CC or Ad-wt-p53. The empty viral vector (Ad-ZsGreenl) served as a negative control in addition to an untreated control. Injections were made on days 0-4 and 7-11 for optimal efficacy and consisted of 5.0x108 PFU of the viral constructs in a 50 µl volume.

Figure 10A shows a representative image of a tumor bearing mouse with the mammary tumor located in the right inguinal area, while Figure 10B shows images of representative excised tumors from each treatment group. The tumor size reduction expected with these treatments served as a direct measure of the tumor suppressor function of the p53 variants. The Ad-p53-CC treatment group achieved statistically significant (p < 0.001) reduction in mean tumor size compared to Ad-wt-p53, Ad-ZsGreenl, and untreated groups (Figure 10C). Although tumor reduction induced by Ad-wt-p53 is not statistically significant compared to the Ad-ZsGreenl or untreated groups, Ad-wt-p53 treatment resulted in stable disease, halting tumor progression. The findings from Figure 10C reveal that p53-CC can achieve tumor regression of an aggressive p53-dominant negative breast cancer model in vivo, while wt-p53 is only capable of halting tumor progression. In addition, the excised tumors from the Ad-ZsGreen1 negative control and untreated groups appeared to be more vascularized compared to tumors derived from the treatment groups Ad-p53-CC and Ad-wt-p53 (Figure 10B), indicating an additional anti-angiogenic effect of p53-CC and wt-p53 in vivo.

Animal body weights were regularly monitored throughout the study and no significant weight loss in animals was observed in any of the groups (Figure 10D) rendering the treatment as well as the viral carrier safe. Throughout the study, the Ad-p53-CC treatment group maintained the smallest mean tumor size compared to all other groups. Both control groups (Ad-ZsGreenl and untreated) exhibited the largest mean tumor size compared to all other groups throughout the entire study.

### iv. Histopathological evaluation of tumor tissues and evidence for tumor suppressor activity

The tumor size reduction observed in Figure 10C indicates tumor suppressor functionality of Ad-p53-CC as well as Ad-wt-p53 in vivo. To verify if this activity is indeed p53-dependent, immunohistochemical staining of p21 was performed as it is one of the best characterized bona fide p53 target genes. 32 Photomicrographs of representative sections from harvested tumor tissues from each group are displayed in Figure 11A. The left column in Figure 5A exhibits hematoxylin and eosin (H&E) staining, while the middle column represents p21 immunohistochemical staining for each group. In addition, the right column in Figure 11A shows the intratumoral expression of the gene load (i.e. p53-CC or wt-p53) as a function of the ZsGreenl fluorescent protein co-expressed with the genes of interest. Microscopic examination of H&E staining revealed higher levels of necrosis (solid arrows, necrosis; open arrows, non-necrotic areas) in all tumors harvested from mice injected with Ad-p53-CC compared to the Ad-wt-p53, Ad-ZsGreenl, or untreated groups (Figure 11A). This implies the detected necrosis may be due to the tumor suppressor activity of p53-CC since the tumors did not reach a large enough size to develop a necrotic core, and as such the observed necrosis was due to treatment, not hypoxia.

3,3' diaminobenzidine (DAP) stains the nuclei of p21-positive cells brown, as shown in photomicrographs (middle column, Figure 11A). Unexpectedly, p21 immunohistochemistry staining revealed higher levels of p21 induction in the Ad-wt-p53 treatment group compared to the Ad-p53-CC treatment group. p21 is one of the key factors by which p53 enforces cell cycle arrest. The induction of cell cycle arrest by p21 converges with findings from Figure 4C where tumors from the Ad-wt-p53 treatment group show a halt (arrest) in tumor growth. As expected, p21 expression was not detected in the Ad-ZsGreenl negative control or untreated groups, which validates that p21 expression is linked to direct p53 activation. Similar expression of ZsGreenl across the different groups (Ad-p53-CC, Ad-wt-p53, and Ad-ZsGreenl) in the right column of Figure 11A indicates comparable intratumoral expression of the viral constructs. In addition, p53 immunohistochemistry staining was performed and equal levels of total (i.e. endogenous and exogenous) p53 expression were detected across all groups, including the untreated group, which relates to the known presence of endogenous p53 in MDA-MB-468 cells (data not shown). Figures 11B and C represent semi-quantitative histoscore analyses of tumor necrosis and p21 up-regulation in the excised tumors from all groups.

Tissues from additional organs (liver, kidney, spleen, heart, and lungs) harvested from animals of all treatment groups showed normal physiology and no abnormalities or signs of pathology. However, metastases of tumor cells to the gastrointestinal region were noted in two out of six mice in the Ad-ZsGreenl group and three out of six mice in the untreated group (data not shown). Thus, p53-CC can have an anti-metastatic function as well as wt-p53 in this tumor model.

### v. Detection of pathway-specific markers for cell cycle arrest and apoptosis

p53-CC is capable of causing tumor size reduction in vivo by favoring the apoptotic pathway, while wt-p53 activity is biased towards inducing cell cycle arrest. Immunoblotting of cleaved (activated) caspase-3 and p21 on samples from in vitro and in vivo was performed. It is well known that all apoptotic pathways converge on caspase-3 (the main executioner caspase) whereas p21 induction by p53 causes cells to undergo cell cycle G1 phase arrest. Therefore, detection of activated caspase-3 and p21 are acceptable biomarkers for apoptosis and cell cycle arrest, respectively. Figure 12A shows a representative western blot analyses of p21 (middle band) and caspase-3 (bottom band) of MDA-MB-468 cells in vitro. MDA-MB-468 cells treated with Ad-p53-CC express lower levels of p21 compared to cells treated with Ad-wt-p53 (Figure 12B), a clear indication of a cell cycle arrest activity of wt-p53. However, higher levels of activated caspase-3 are detected in cells treated with Ad-p53-CC compared to Ad-wt-p53 (Figure 12C, a hallmark of apoptosis induction).

Part of the excised tumor tissues from each animal was homogenized and lysed for western blotting. Figure 12D shows representative western blotting of MDA-MB-468 in vivo tumor tissue lysates. Similar to the findings obtained from in vitro western blotting (Figures 12A-C), tumor tissues from the Ad-p53-CC treatment group showed lower p21 expression (Figure 12E) but higher caspase-3 induction (Figure 12F).

Results from Figure 12 confirm that p53-CC favors induction of apoptosis in vitro and in vivo, while wt-p53 is biased towards inducing cell cycle arrest.

### 2. DISCUSSION

This data confirms that chimeric p53-CC has superior tumor suppressor function compared to wt-p53 in vitro and in vivo using a dominant negative mutant p53 model. Although the concept of a "superactive" p53 was known, there are no known reports of constructing a p53 capable of bypassing the dominant negative effect of mutant p53 in cancer cells and increases apoptosis (over wt-p53). p53-CC induces higher levels of cell death in vitro compared to wt-p53 in the 7-AAD assay (Figure 8) as well as in the apoptosis assays: TMRE, caspase-3/7, and annexin-V (Figure 9). To validate if the superior activity of p53-CC in vitro translates in vivo, animal studies were performed using an orthotopic MDA-MB-468 xenograft breast cancer model in mouse mammary fat pads. Indeed, intratumoral injections with Ad-p53-CC achieved substantial tumor regression that is statistically significant compared to the Ad-wt-p53, Ad-ZsGreenl, and untreated groups (Figure 10C), without any sign of treatment toxicity (Figure 10D). H&E staining of tumor tissues revealed higher levels of necrosis in all tumor tissues from mice injected with Ad-p53-CC compared to the Ad-wt-p53, Ad-ZsGreenl, or untreated groups. To test if the observed tumor suppressor activity of p53-CC in vivo is p53-dependent, immunohistochemistry staining of p21, the most well studied p53 target gene,32 was conducted. As expected, p21-positive staining was observed only in the Ad-p53-CC and Ad-wt-p53 treatment groups (Figure 5) with higher p21 staining with Ad-wt-p53 treatment.

Since p53-CC was able to induce apoptosis (including caspase 3/7), and wt-p53 increased p21 expression, a differential mechanism of p53-CC (favoring apoptosis) and wt-p53 (favoring cell cycle arrest) in MDA-MB-468 cells was investigated. To test this premise, immunoblotting was carried out on samples from in vitro (Figures 12A-C) and in vivo (Figures 12D-F) to detect expression levels of p21, which induces cell cycle arrest, and caspase-3, a major executer of apoptosis. Figure 6 revealed that tumor tissues treated with Ad-p53-CC expressed low levels of p21 (reduced cell cycle arrest) but high levels of active caspase-3 (increased apoptosis). In contrast, tumor tissues injected with Ad-wt-p53 expressed high levels of p21 (increased cell cycle arrest) but low levels of caspase-3 (decreased apoptosis).

The transdominant mutant p53 found endogenously in MDA-MB-468 cells retains the ability to hetero-oligomerize with exogenous wt-p53, since its tetramerization domain remains intact. Upon hetero-oligomer formation, the activity of exogenous wt-p53 is impaired due to the dominant negative effect of mutant p53 in cancer cells. The chimeric p53-CC was designed to overcome this barrier with a use of an alternative oligomerization domain, a coiled-coil from Bcr (CC). This CC is known to tetramerize as an antiparallel dimer of dimers, similar to the tetramerization domain of wt-p53.

The use of this alternative oligomerization domain allows p53-CC to escape hetero-oligomerization with mutant p53 and consequent transdominant inhibition. Indeed, previous work validated the ability of p53-CC to exclusively form homo-oligomers. From a gene therapy point of view, the ability of p53-CC to evade transdominant inhibition gives it an advantage over wt-p53 in dominant mutant p53 cancer cells such as MDA-MB-468.

The viral-mediated gene therapy in vivo studies show that p53-CC has superior tumor suppressor activity compared to wt-p53 in the MDA-MB-468 aggressive p53-dominant negative breast cancer model. In fact, p53-CC was capable of achieving significant tumor regression, while wt-p53 is only capable of halting tumor progression (Figure 10C). The difference in outcome of the tumor size reduction was due to the ability of p53-CC to activate the apoptotic pathway, whereas wt-p53 activates cell cycle arrest via p21 induction. These findings are supported by western blot analyses from in vitro (Figure 12A-C) and in vivo (Figure 12D-F) MDA-MB-468 cells/tumors.

Analysis of p53-regulated gene expression patterns explains the differential pathway activation between p53-CC and wt-p53 (apoptosis vs cell cycle arrest). It has been shown that p53-responsive gene expression patterns are highly variable, depending on the p53 protein levels in the cell.40 It is also known that higher levels of active p53 lead to activation of apoptotic genes, while lower levels of p53 activate cell cycle regulator genes. In cells treated with p53-CC vs wt-p53, higher levels of the chimeric p53-CC protein exist compared to levels of active wt-p53 protein in cells, due to the ability of p53-CC to escape sequestration by mutant p53. Unlike p53-CC, substantial amounts of the wt-p53 protein are forced into inactive hetero-oligomers with endogenous mutant p53 (the dominant negative effect). This reduction in 'available' active wt-p53 could lead to failure in binding promoters of apoptotic genes that require higher active p53 protein levels in the cell. Cell cycle regulator genes, such as p21, would be activated instead, since wt-p53 possess higher binding affinities to these promoters (i.e. requires less p53 to bind and activate). In contrast, abundance in active chimeric p53-CC protein levels is found in cells treated with p53-CC, which would lead to binding and activation of apoptotic genes promoters. The variability of pathway activation (i.e. p53-CC, apoptosis vs. wt-p53, cell cycle arrest) may be specific to this tumor model due to the dominant negative mutant p53 endogenously found in MDA-MB-468 cells/tumors. This is because it has been shown12 that p53-CC and wt-p53 induce similar levels of apoptosis in four different non-p53-dominant negative breast cancer cell lines with varying endogenous p53 statuses (H1373 cells: p53 null, HeLa cells: wt-p53, T47D cells: wt-p53 mislocalized, and MDA-MB-231 cells: mutant p53). Furthermore, RT-PCR analyses and western blotting showed that p53-CC and wt-p53 induced similar levels of p21 gene expression in T47D breast carcinoma cells.

A chimeric superactive p53 has been described as the 'ultimate cancer therapeutic'. The p53-CC demonstrates comparable functional transcriptional activity to wt p53,12 shows significantly improved apoptosis (Figures 8 and 9), and successfully circumvents the dominant negative inactivating effect of endogenous mutant p53 in vitro. The in vivo data (Figure 10) demonstrates that p53-CC is more effective than wt p53, and can serve as a more potent and reliable novel anticancer therapeutic.

### 3. MATERIALS AND METHODS

### i. Recombinant Adenovirus Production

Replication-deficient recombinant adenovirus serotype 5 (Ad) constructs were generated using the Adeno-X® Adenoviral Expression System 3 (Clontech, Mountain View, CA). Either wt-p53 or p53-CC was inserted into a cassette under the control of the CMV promoter. A separate CMV promoter controls the expression of ZsGreenl fluorescent protein for visualization. The empty virus (vector) was used as a negative control. Wt-p53 and p53-CC were PCR amplified with primers containing 15 base pair homology with a linearized pAdenoX vector (Clontech) based on an In-Fusion® HD Cloning Kit (Clontech). Stellar® competent cells (Clontech) were transformed with the adenoviral vector plasmids containing the constructs. Viral DNA was then purified, linearized and transfected into HEK293 cells for packaging and amplification. Viral particles were isolated from HEK293 cells by freeze-thawing, purified using Adeno-X® Mega Purification Kit (Clontech), and dialyzed against storage and proper tonicity buffer (2.5% glycerol (w/v), 25 mM NaCl, and 20 mM Tris-HCl, pH 7.4). The viral titer was determined using flow cytometry per the manufacturer's recommendation.

### ii. Cell Lines and Viral Transductions

HEK293 human embryonic kidney cells (ATCC, Manassas, VA) were used for viral production and MDA-MB-468 human breast adenocarcinoma cells (ATCC) harboring a dominant negative mutant p53 were grown as monolayers in DMEM (Invitrogen, Carlsbad, CA) supplemented with 10% fetal bovine serum, 1% penicillin-streptomycin-glutamine, and 0.1% gentamicin. MDA-MB-468 cells were also supplemented with 1% MEM non-essential amino acids (Invitrogen). All cells were incubated in 5% CO2 at 37°C. The cells were seeded at a density of 3.0 × 10⁵ cells in 6-well plates (Greiner Bio-One, Monroe, NC). Viral transductions were carried out immediately after seeding the cells at multiplicity of infection (MOI) of 200.

### iii. 7-AAD Assay

Following manufacturer's instructions and as previously described, 43 MDA-MB-468 cells were stained with 7-aminoactinomycin D (7-AAD, Invitrogen) 48 h after transfection. Cells were analyzed and gated for ZsGreenl (with same fluorescence intensity to ensure equal expression of proteins) using the FACSCanto-II (BD-BioSciences, University of Utah Core Facility) and FACSDiva software. Excitation was set at 488 nm and detected at 507 nm and 780 nm for ZsGreen1 and 7-AAD, respectively. The means from three separate experiments (n=3) were analyzed using one-way ANOVA with Bonferroni's post hoc test.

### iv. TMRE Assay

MDA-MB-468 cells were incubated with 100 nM tetramethylrhodamine ethyl ester (TMRE) (Invitrogen) for 30 min at 37 °C 36 h after infection. 44 The time point was determined to be 36 h as a result of several optimization pilot studies for the TMRE assay, and since mitochondrial outer membrane permeabilization occurs prior to caspase-3/7, annexin-V, and 7-AAD detection (48 h). MDA-MB-468 cells were pelleted and resuspended in 300 µL of annexin-V binding buffer (Invitrogen). Only ZsGreenl positive cells were analyzed by using the FACS Canto-II (BD BioSciences, University of Utah Core Facility) with FACS Diva software. ZsGreenl was excited with the 488 nm laser with emission filter 530/35, and TMRE was excited with the 561 nm laser with the emission filter 585/15. Mitochondrial depolarization (loss in TMRE intensity) correlates with an increase in MOMP. Independent transfections of each construct were tested three times (n = 3).

### v. Caspase-3/7 Assay

MDA-MB-468 cells were probed 48 h after treatment using FLICA® 660 Caspase-3/7 Assay Kit (Immunochemistry Technologies, Bloomington, MN). Cells were pelleted, resuspended in 300 µL of 1× wash buffer (Immunochemistry Technologies), and incubated with FLICA® 660 Caspase-3/7 reagent for 45 min per the manufacturer's recommendations. Only ZsGreenl positive cells were analyzed using the FACS Canto-II (BD BioSciences) with FACS Diva software. ZsGreenl and FLICA® 660 were excited with the 488 nm (emission filter 530/35) and the 635 laser (emission filter 670/30), respectively. Independent transfections of each construct were tested three times (n = 3).

### vi. Annexin-V Assay

The annexin-V assay was performed as before. Briefly, 48 h post infection, MDA-MB-468 cells were suspended in 400 µl annexin binding buffer (Invitrogen) and incubated with 5 µl annexin-APC (annexin-V conjugated to allophycocyanin, Invitrogen) for 15 minutes. The incubated cells were ZsGreenl gated and analyzed using FACSCanto-II. ZsGreenl and APC were excited at 488 nm and 635 nm wavelengths and detected at 507 nm and 660 nm, respectively. Each construct was tested three times (n=3) and analyzed using one-way ANOVA with Bonferroni's post hoc test.

### vii. In Vivo Study

All experiments were performed in Female nu/nu athymic mice (6-8 weeks old, Jackson Laboratories, Bar Harbor, ME). Human MDA-MB-468 cells (1x10⁷ cells/mouse in 100 µl of serum-free RPMI-1640 medium) were injected subcutaneously into the mammary fat pad located in the right inguinal area. When tumors reached a mean size of 50mm³, animals were randomized into 4 treatment groups and received single peritumoral injections of adenoviral constructs (5.0x10⁸ pfu) in a 50 µl volume prepared fresh on days 0-4 and 7-11. Twenty four hours after the last injection the mice were sacrificed and the tumors as well as the organs were harvested for analyses. Tumor volumes were measured daily using Vernier calipers along the longest width (W) and the corresponding perpendicular length (L). The tumor volume was calculated using V= (L x W (0.5W)).

### viii. Histology

Animal tumor tissue samples and organs were fixed in 10% formalin for 24 h followed by tissue preparation and embedded in paraffin. Embedded tissues were then sectioned to cut at 4 µm thick sections and mounted on plus slides. Slides from each tumor tissue from all mice in the three treatment groups as well as the untreated group were stained using hematoxylin and eosin and p21 immunohistochemistry stain. Tissue and histological slide preparation was conducted in collaboration with ARUP Laboratories (Salt Lake City, Utah).

### ix. Western Blotting

In vivo: fresh tumor tissue samples from animals of each treatment group were collected, snap-frozen in liquid nitrogen, ground with mortar and pestle, re-suspended in 200 mL lysis buffer (62.5 mM Tris-HCl, 2% w/v SDS, 10% glycerol, 1 % protease inhibitor) followed by sonication on ice. Recovered tissue lysates were then centrifuged for 45 min at 14,000 rpm and the supernatants were used for immunoblotting. Standard western blotting procedures were followed using primary antibodies to detect p21/WAF1, cleaved caspase-3, and actin as a loading control. The primary antibodies anti-p21 (ab 16767, Abcam, Cambridge, MA), anti-cleaved caspase-3 (#9665P, Cell Signaling Technology, Danvers, MA), anti-actin (mouse, ab3280, Abcam), and anti-actin (rabbit, ab1801, Abcam) were detected with anti-rabbit (#7074S, Cell Signaling Technology) or anti-mouse (ab6814, Abcam) HRP-conjugated antibodies before the addition of SuperSignal West Pico chemiluminescent substrate (Thermo Scientific, Waltham, MA). Signals were detected using a FluorChem FC2 imager and software (Alpha Innotech, Sanata Clara, CA). All experiments were conducted in triplicates.

In vitro: 24 h following infection of MDA-MB-468 cells, 3x10⁵ cells were pelleted and resuspended in 200 µL lysis buffer (62.5 mM Tris-HCl, 2% w/v SDS, 10% glycerol, 1 % protease inhibitor), sonicated on ice, and centrifuged for 15 min at 14,000 rpm. The supernatants were used for immunoblotting as described above.

### x. Statistical Analysis

One-way ANOVA with Bonferroni's post test was used to compare the different treatment groups and controls. A value of p < 0.05 was considered statistically significant. Error bars represent standard deviations from at least three independent experiments (n = 3) except for the animal study (n=6).

Those skilled in the art will recognize, or be able to ascertain using no more than routine experimentation, many equivalents to the specific embodiments of the method and compositions described herein. Such equivalents are intended to be encompassed by the following claims.

### P. Example 3: A Re-Engineered p53 Chimera with Enhanced Homo-Oligomerization that Maintains tumor Suppressor Activity

The tumor suppressor p53 is the most commonly mutated gene of all human cancers, making it an ideal therapeutic target. However, the diversity of p53 mutations precludes finding a single drug that hits all possible variants of the protein. In cancer cells, mutant p53 may not only impair tumor suppressor function and transcriptional activity, but effectively deplete wild type p53 (wt-p53) since mutant p53 retains its ability to oligomerize with other p53 via the tetramerization domain (TD). Upon hetero-oligomerization of mutant and wt-p53 in cancer cells, mutant p53 exerts a dominant negative effect over wt-p53 and leads to its inactivation as a therapeutic. To overcome these issues, an alternative approach has been to engineer a chimeric version of p53 for cancer gene therapy that can be used universally, regardless of p53 mutational status in cancer. To create this chimeric, transcriptionally active version p53 that can only form homo-tetramers, domain swapping motifs were searched, and the 31 amino acid TD of p53 was replaced with the 72 amino acid coiled-coil (CC) of Bcr (breakpoint cluster region protein). Superficially, these motifs can appear structurally dissimilar, but both the TD and CC contain a main α-helix that orients in an anti-parallel fashion and forms a dimer of dimers. Due to their similar orientation and ability to form tetramers, the CC motif from Bcr was a reasonable starting point for domain swapping. Swapping the tetramerization domain of p53 with the CC domain enhances the utility of p53 for cancer gene therapy in p53-dominant negative breast cancer cells. This alteration of the oligomerization motif of the tumor suppressor allowed for the chimeric p53, namely p53-CC, to evade hetero-oligomerization with endogenous mutant p53 commonly found in cancer cells while retaining the tumor suppressor function of p53. This proves to be critical since mutant p53 has a transdominant inhibitory effect over wild-type p53 upon hetero-oligomerization.

Bcr, from which the CC was obtained, is a ubiquitous eukaryotic phosphotransferase, and has mostly been studied in the context of chronic myeloid leukemia (CML) where a reciprocal chromosomal translocation with Abl results in the fusion protein Bcr-Abl, the causative agent of CML. Generally, Bcr can be involved in inflammatory pathways and cell proliferation. Although it has been shown that Bcr-knockout mice still survive, one of the major defects in these mice was reduced intimal proliferation in low-flow carotid arteries compared to wild type mice. In addition, Bcr plays a role in arterial proliferative disease *in vivo* as well as differentiation and inflammatory responses of vascular smooth muscle cells. Since domain swapping to create p53-CC could result in p53-CC interacting with endogenous Bcr, modifications on the CC domain are necessary to minimize potential interactions with Bcr. Hence, this study was used to modify the CC domain in p53-CC to reduce potential interactions with endogenous Bcr.

Coiled-coil domains are characterized by heptad repeats of amino acids (denoted by letters for each residue, (**abcdefg**)ₙ, for n repeats) that control the specificity and orientation of the oligomerization motif. Distinct interaction profiles exist between the different residues based on the orientation (parallel or antiparallel) of the coiled-coil. Surface interactions between positions **e** to **e'** (where the **'** denotes a residue on the opposing coiled-coil in the dimer) and **g** to **g'** are known to be essential in antiparallel coiled-coils, whereas, interactions between positions **g** to **e'** are the most critical for parallel coiled-coils. The coiled-coil domain from Bcr is assembled as two 36-residues helices antiparallel to each other (Figure 15A). This antiparallel orientation gives rise to the aforementioned **e** to **e'** and **g** to **g'** interactions that can be utilized to modify electrostatic interactions within a dimer. The design of mutations that will form opposing charges on residues **e** to **e'** and **g** to **g'** to increase salt bridge formation (see Figure 15) in order to improve homo-dimerization of CC mutants and disfavor hetero-oligomerization with wild-type (CCwt) were investigated, with the goal of minimizing potential interactions with endogenous Bcr in cells. *In silico* examination of CCwt (Figure 15A) revealed that Bcr has uncharged Ser-41 at position **g** and Glu-48 (acidic) representing **g'** that are within proximity for salt bridge formation. Similarly, CCwt has uncharged Gln-60 at position **e** and Lys-39 (basic) at position **e'** which are also within proximity for salt bridge formation. Therefore, introducing S41R (Arg, basic) and Q60E (Glu, acidic) mutations separately, can form two extra salt bridges per mutation (Figure 15B and 15C, respectively). These two mutant candidates are referred to as CCmutS41R and CCmutQ60E.

In addition, examination of the coiled-coil inter-chain salt bridges indicates that two more compound mutants (i.e., more than one mutation per candidate) can be made to improve homo-dimerization of CC mutants. Mutation of Glu-34 to Lys and Arg-55 to Glu (CCmutE34K-R55E) can preserve all four stabilizing salt bridges found in CCwt in the case of CCmut homo-oligomerization (Figure 15D). However, in the case of CCmutE34K-R55E hetero-oligomerization with CCwt, only two stabilizing salt bridges are maintained while two destabilizing charge-charge repulsions are formed. This allows for increased specificity for CCmutE34K-R55E towards homo-oligomerization over hetero-oligomerization with CCwt. Similarly, introducing the E46K and R53E compound mutation (CCmutE46K-R53E) results in favoring homo-oligomerization (Figure 15E). Disfavoring hetero-oligomer formation with CCwt represents minimizing interactions with endogenous Bcr in cells.

The resulting designed four mutant candidates: p53-CCmutS41R, p53-CCmutQ60E, p53-CCmutE34K-R55E, and p53-CCmutE46K-R53E, are listed in Table 3 and were further assessed computationally and tested *in vitro* for their ability to retain apoptotic activity and minimize any possible interaction with endogenous Bcr.

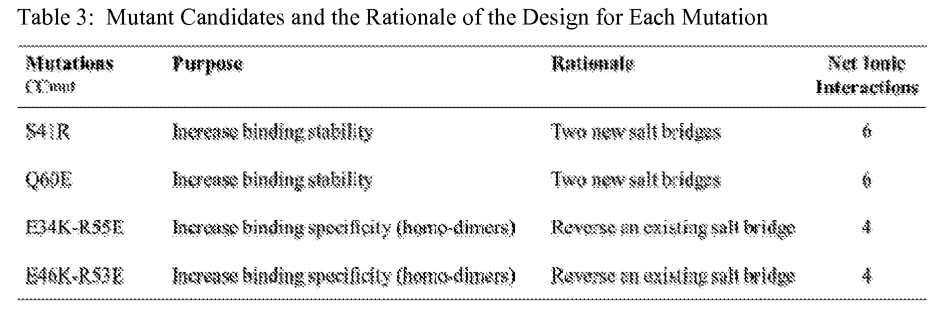

### 1. Materials and Methods

### i. Computational Modeling and Simulation

Models of the Bcr CC domain were built starting with the crystal structure of the N-terminal oligomerization domain of Bcr-Abl (Protein Data Bank code 1K1F, choosing residues 1-67 in each of chains A and B.). Using the *swapaa* tool in Chimera, selenomethionine residues were reverted back to methionine, and residue 38 was mutated back to cysteine, consistent with the wild-type structures. Models of the mutant coiled-coils were built using the *swapaa* tool which facilitates placement of modified side chains by sourcing the Dunbrack backbone-dependent rotamer library to predict the most accurate side-chain rotamers. Models were built using ff12SB force field parameters and explicitly solvated in truncated octahedron with at least a 10 Å surrounding buffer of TIP3P water. Net-neutralizing counter-ions (Na⁺/Cl⁻) were incorporated using the Joung & Cheatham ion parameters, and 52 additional Na⁺/Cl⁻ atoms were added to achieve an approximate ion concentration of 200 mM. All models were subjected to an extensive minimization and equilibration protocol to relax and steer systems towards energetically-favored conformations prior to production molecular dynamics (MD). An initial minimization was performed (steepest descent and conjugate gradient) prior to heating of the system to 300 K. 25 kcal/mol-Å² restraints were placed upon backbone C_{α} atoms throughout the initial minimization and heating step. Following the initial minimization and heating, systems were subjected to five cycles of minimization (steepest descent and conjugate gradient) and equilibration, in which restraint weights were lifted from 5 kcal/mol-Å² to 1 kcal/mol-Å² following each cycle. A final equilibration was performed at a restraint weight of 0.5 kcal/mol-Å² prior to production MD. Constant temperature and pressure were controlled throughout the minimization protocol using a Berendsen thermostat with a 0.2 coupling time. All production MD simulations were carried out with the AMBER 12.0 modeling code suite for 200 ns (using a 2 fs time step) in explicit solvent, using a Berendsen thermostat with default coupling time to control constant temperature and pressure, a 10 Å non-bonded cutoff, default particle mesh Ewald treatment of electrostatics and SHAKE applied to the hydrogens.

Analysis of the MD trajectories was performed using the ptraj and CPPTRAJ analysis tools available in the AmberTools 12.0 and 13.0 distributions: RMSD and 2D-RMS analyses were employed to monitor if the protein structure retained the expected structure, and clustering analysis of the structures sampled during the MD (using the average linkage algorithm) was used to identify the most frequently sampled protein conformations of each MD trajectory. Additionally, a DSSP analysis of secondary structure was performed to determine the percent helicity of each mutant, and α-helical specific hydrogen bonds were recorded by monitoring hydrogen bonding interactions between peptide backbone atoms of *i* and *i*+*4* residues. The atomic positional fluctuations of C_{α} backbone atoms were recorded to identify regions of flexibility in response to the induced mutations. An MM-PBSA energetic analysis was performed to assess the relative binding energies of each mutant.

### ii. Cell Lines and Transient Transfections

T47D human ductal breast epithelial tumor cells (ATCC, Manassas, VA), COS-7 monkey kidney fibroblast cells (ATCC), SKOV-3.ipl human ovarian adenocarcinoma cells (a kind gift from Dr. Margit Janát-Amsbury, University of Utah), and MCF-7 human breast adenocarcinoma cells (ATCC) were cultured in RPMI 1640 (T47D, COS-7, MCF-7) or DMEM (SKOV-3.ipl) (Invitrogen, Carlsbad, CA) supplemented with 10% FBS (Invitrogen), 1% penicillin-streptomycin (Invitrogen), 1% glutamine (Invitrogen) and 0.1% gentamycin (Invitrogen). Additionally, T47D and MCF-7 cells were supplemented with 4 mg/L insulin (Sigma, St. Louis, MO). Cells were maintained in a 5% CO₂ incubator at 37°C. For all assays, 3.0 x 10⁵ cells for T47D and MCF-7 cells, 2.0 x 10⁵ for COS-7 and SKOV-3.ipl cells were seeded in 6-well plates (Greiner Bio-One, Monroe, NC). Approximately 24 h after seeding, transfection was performed using 1 pmol of DNA per well and Lipofectamine 2000 (Invitrogen) following the manufacturer's recommendations.

### iii. Plasmid Construction

The plasmids pEGFP-wt-p53 (wt-p53), pEGFP-p53-CC (p53-CCwt), and pEGFP-CC (CCwt) were subcloned as previously. pEGFP-p53-CCmutS41R (p53-CCmutS41R), pEGFP-p53-CCmutQ60E (p53-CCmutQ60E), pEGFP-p53-CCmutE34K-R55E (p53-CCmutE34K-R55E), and pEGFP-p53-CCmutE46K-R53E (p53-CCmutE46K-R53E) were created through site directed mutagenesis using pEGFP-p53-CC as the template.

The following primers were used for the p53-CCmutS41R mutation: 5'-GGAGCGCTGCAAGGCCCGCTCCATTCGGCGCCTGG-3' (SEQ ID NO:9) and 5'-CCAGGCGCCGAATGGAGCGGGCCTTGCAGCGCTCC-3'(SEQ ID NO: 10); for the p53-CCmutQ60E mutation, 5'-TCCGCATGATCTACCTGGAGACGTTGCTGGCCAAG-3' (SEQ ID NO: 11) and 5'-CTTGGCCAGCAACGTCTCCAGGTAGATCATGCGGA-3' (SEQ ID NO: 12) primers were used.

For the p53-CCmutE34K-R55E compound mutant, sequential site directed mutagenesis was carried out using the following primers: for the E34K mutation, 5'-GTGGGCGACATCGAGCAGAAGCTGGAGCGCTGCAAGG-3'(SEQ ID NO: 13) and 5'-CCTTGCAGCGCTCCAGCTTCTGCTCGATGTCGCCCAC-3'(SEQ ID NO: 14); for the R55E mutation, 5'-AGGTGAACCAGGAGCGCTTCGAGATGATCTACCTGCAGACGTT-3' (SEQ ID NO:15) and 5'-AACGTCTGCAGGTAGATCATCTCGAAGCGCTCCTGGTTCACCT-3' (SEQ ID NO:16) primers were used.

For the p53-CCmutE46K-R53E compound mutant, sequential site directed mutagenesis was carried out using the following primers: for the E46K mutation, 5'-GCCTCCATTCGGCGCCTGAAGCAGGAGGTGAACCAGG-3'(SEQ ID NO: 17) and 5'-CCTGGTTCACCTCCTGCTTCAGGCGCCGAATGGAGGC-3' (SEQ ID NO: 18); for the R53E mutation, primers 5'-AGCAGGAGGTGAACCAGGAGTTCCGCATGATCTACCTGCA-3' (SEQ ID NO: 19) and 5'-TGCAGGTAGATCATGCGGAACTCCTGGTTCACCTCCTGCT-3' (SEQ ID NO:20) were used for deletion of R53; primers 5'-GCAGGAGGTGAACCAGGAGGAGTTCCGCATGATCTACCTGC-3' (SEQ ID NO:21) and 5'-GCAGGTAGATCATGCGGAACTCCTCCTGGTTCACCTCCTGC-3' (SEQ ID NO:22) were used for insertion of 53E.

The plasmids pBIND-p53-CCwt, pBIND-p53CCmutE34K-R55E, pACT-p53-CCwt, and pACT-p53-CCmutE34K-R55E were cloned for the mammalian two-hybrid assay. For pBIND-p53-CCwt and pBIND-p53-CCmutE34K-R55E, DNA encoding p53-CCwt and p53-CCmutE34K-R55E was digested from the pEGFP-p53-CC and pEGFP-p53-CCmutE34K-R55E vectors respectively, using the *Bam*HI and *Kpn*I restriction enzymes and subcloned into the pBIND vector (Promega, Madison, WI) at the *BamHI* and *Kpn*I sites. Similarly, to clone pACT-p53-CCwt and pACT-p53-CCmutE34K-R55E, DNA encoding p53-CCwt and p53-CCmutE34K-R55E was also digested from the pEGFP-p53-CC and pEGFP-p53-CCmutE34K-R55E vectors respectively, using the *BamHI* and *Kpn*I restriction enzymes and subcloned into the pACT vector (Promega) at the *Bam*HI and *Kpn*I sites.

### iv. 7- AAD Assay

Following manufacturer's instructions and as previously described, T47D, SKOV-3.ip1, and MCF-7 cells were pelleted and resuspended in 500 µL PBS (Invitrogen) containing 1 µM 7-aminoactinomycin D (7-AAD) (Invitrogen) for 30 min prior to analysis by flow cytometry. The assay was performed 48 h after transfection for T47D and MCF-7 and 24 h for SKOV-3.ipl. Cells were analyzed and gated for EGFP (with same fluorescence intensity to ensure equal expression of proteins) using the FACSCanto-II (BD-BioSciences, University of Utah Core Facility) and FACSDiva software. Excitation was set at 488 nm and detected at 507 nm and 660 nm, respectively. Each construct was tested three times (n=3).

### v. Mammalian Two-Hybrid Assay

The pBIND-p53-CCwt (or pBIND-p53-CCmutE34K-R55E) containing the *Renilla reniformis* luciferase gene for normalization, pACT-p53-CCwt (or pACT-p53-CCmutE34K-R55E), and pG51uc (containing firefly luciferase gene, Promega) plasmids were cotransfected using 3.5 µg of each plasmid following the manufacture's recommendations. The pBIND-Id and pACT-MyoD (Promega) plasmids were used for the positive control, and pBIND vector lacking the coiled-coil gene was used as the negative control. Approximately 24 h after transfection, the Dual-Glo Luciferase Assay (Promega) was used to detect both firefly and renilla luminescence as previously. The means from duplicate transfections were taken from three separate experiments (n=3). As per the manufacturer's protocol, a relative response ratio was calculated using the firefly luciferase values normalized to the renilla luciferase values: (sample - ctrl⁻) /(ctrl⁺ - ctrl⁻).

### vi. Co-Immunoprecipitation (Co-IP)

Co-IP was performed as we have done before. Briefly, T47D cells treated with p53-CCmutE34K-R55e or p53-CCwt were prepared using the Dynabeads Co-Immunoprecipitation Kit (Invitrogen) 24 h post transfection. Approximately 0.2 g of T47D treated cell pellet was lysed in 1.8 mL of extraction buffer B (1 x IP, 100 nM NaCl, 2 mM MgCl₂, 1 mM DTT, 1% protease inhibitor). The lysate was incubated for 30 min at 4°C with 1.5 mg of dynabeads coupled with anti-GFP antibody (ab290, Abcam). Immune complexes were then collected on a magnet, washed, and eluted using 60 µL of elution buffer. Finally, the eluted complexes were denatured and western blots were carried out as described before.⁹ The coiled-coil domain was probed using anti-Bcr (sc-885, Santa Cruz Biotechnology, Santa Cruz, CA). The primary antibody was detected with anti-rabbit HRP-conjugated (#7074S, Cell Signaling Technology, Danvers, MA) antibody before the addition of SuperSignal West Pico chemiluminescent substrate (Thermo Scientific, Waltham, MA). Signals were detected using a FluorChem FC2 imager and software (Alpha Innotech, Santa Clara, CA). Each co-IP was repeated at least three times. A semi-quantitative densitometry analysis was carried out by normalizing the detected Bcr band to either p53-CCwt or p53-CCmutE34K-R55E as described before.

### vii. Statistical Analysis

For *in vitro* experiments, one-way ANOVA with Bonferroni's post test was used to compare the different groups and controls. A value of p < 0.05 was considered statistically significant. Error bars represent standard deviations from at least three independent experiments (n = 3).

### 2. Results

### i. In silico modeling of coiled-coil structures and estimation of binding free energies

Computational modeling and atomistic biomolecular simulations were employed to facilitate the design of coiled-coil mutations which serve to enhance homo-oligomerization of the modified coils while disrupting hetero-oligomerization with the native coiled-coil region of Bcr. Initial simulations estimated differences in relative binding free energy of the modified coils to predict the most effective coiled-coil design (Table 4). All four mutants from Table 3 were rationally designed based on optimization of the electrostatic interactions and the potential for salt bridge formation identified in the helical wheel structure of the CC motif (helical wheel characterized previously by Taylor et al.). The designed mutations aimed to enhance homo-oligomerization by either enhancement of the binding interaction between modified coiled-coils (CCmutS41R and CCmutQ60E), or disruption of the interaction between mutant and wild-type coiled-coils (CCmutE34K-R55E and CCmutE46K-R53E). Production molecular dynamics were carried out on a total of nine independent simulations, in which trajectories were generated for each of the modified coils paired with either itself (homo-dimer) or CCwt (hetero-dimer). A wild-type coiled-coil homo-dimer was used as a control.

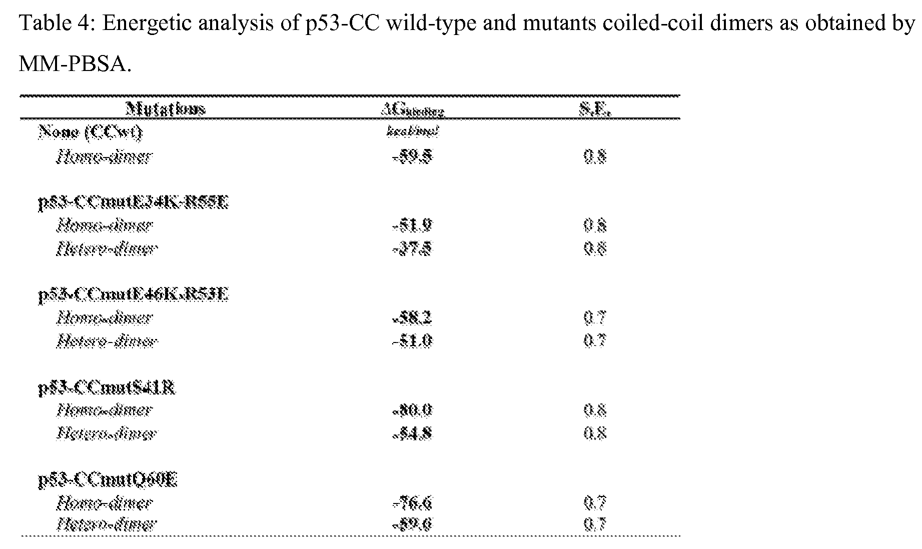

An MM-PBSA post-processing energetic analysis of the MD trajectories of the dimers was performed on each independent simulation to identify the optimal modifications to enhance self-oligomerization (see Table 4). Modified coiled-coils, which were designed to promote self-oligomerization by increasing the binding stability (p53-CCmutS41R and p53-CCmutQ60E), had relatively strong binding for their homo-dimers (Table 4, ΔG= -80.0 kcal/mol and ΔG= -76.6 kcal/mol, respectively). However, they failed to disrupt binding to the native CCwt (Table 4, ΔG= -54.8 kcal/mol and ΔG= -59.6 kcal/mol, respectively), indicating that creating additional salt bridges *will not* prevent p53-CC from binding to endogenous Bcr. Results (Table 4) indicate that the best approach to increase self-oligomerization among the modified coiled-coils while minimizing hetero-oligomerization with Bcr is to increase the binding specificity of the coiled-coil for itself through the reversing of existing salt bridges (resembled by CCmutE34K-R55E and CCmutE46K-R53E). Energetic analyses of the modified coiled-coils featuring a reversal of salt bridges (p53-CCmutE34K-R55E and p53-CCmutE46K-R53E) revealed minimal de-stabilization of the homo-dimers p53-CCmutE34K-R55E and p53-CCmutE46K-R53E (Table 4, ΔG= -51.9 kcal/mol and ΔG= -58.2 kcal/mol, respectively), and in the case of the p53-CCmutE34K-R55E mutant, a significant de-stabilization of the hetero-dimer with CCwt (Table 4, ΔG= -37.5 kcal/mol). The p53-CCmutE46K-R53E mutant hetero-dimer with CCwt was minimally destabilized (AG= -51.0 kcal/mol). Therefore, of the four rationally designed mutants, p53-CCmutE34K-R55E is the only variant which displays both of the desired characteristics of homo-dimer stabilization and disruption of CCwt binding, indicating that the CCmutE34K-R55E mutant provides the most effective strategy to promote self-oligomerization and prevent interaction with native Bcr.

### ii. Initial screening for in vitro activity

Next, *in vitro* screening of the activity of each p53-CCmut was performed to examine if the mutations abrogate the tumor suppressor function of p53-CC. Active p53-CC has been shown previously to induce significant levels of cell death in T47D breast cancer cells. Hence, the 7-AAD assay, which stains apoptotic and necrotic cells, served as a screening tool to measure tumor suppressor function of the different p53-CC mutants (Figure 16). Surprisingly, all of the designed mutations led to abolishment of p53-CC function, except for the CCmutE34K-R55E compound mutation. Figure 16 illustrates that p53-CCmutE34K-R55E (fifth bar) retains the apoptotic activity of p53-CCwt and wt-p53 (first two bars). As expected, the negative control CCwt alone shows no apoptotic activity in the 7-AAD assay (last bar). These findings indicate that the S41R, Q60E, and E46K-R53E mutations can disrupt the oligomerization of CC, lead to instability of the coiled-coil domain, or alter the conformation of p53, resulting in loss of tumor suppressor function (third, fourth and sixth bars, respectively).

Although computational design and modeling implies that S41R, Q60E, and E46K-R53E can be candidates for increasing salt bridge formation and binding stability, the data in Figure 16 illustrates that introducing any of these mutations on the CC domain leads to biological inactivation of the chimeric p53-CC. Therefore, the mutant candidate was narrowed down to p53-CCmutE34K-R55E, which favors homo-oligomer formation over hetero-dimerization with CCwt of Bcr (Table 4), while retaining the biological activity of p53-CCwt (Figure 16). Figure 17 shows ribbon diagrams with corresponding helical wheels (below) of CCwt homo-dimer (Figure 17A), CCwt:CCmutE34K-R55E hetero-dimer (Figure 17B), and CCmutE34K-R55E homo-dimer (Figure 17C). As expected from our computational design, the compound mutant CCmutE34K-R55E does not lead to formation of new additional ionic interactions (salt bridges). Instead, the same two salt bridges found in the CCwt:CCwt homo-dimer (Figure 17A) are preserved (but reversed) in the CCmutE34K-R55E:CCmutE34K-R55E homo-dimer (Figure 17C). However, Figure 17B illustrates that two charge-charge repulsions in the CCwt:CCmutE34K-R55E hetero-dimer can form, which can reduce p53-CCmutE34K-R55E interaction with Bcr (aka CCwt).

### iii. Global stability of p53-CCmutE34K-R55E

Several analyses were performed to evaluate the stability of CCmutE34K-R55E homo-dimer relative to the CCwt homo-dimer and the CCwt:CCmutE34K-R55E hetero-dimer. RMSD analyses of the MD sampled structures to the initial structures revealed that both the mutant homo-dimer and mutant hetero-dimers remained close to their initial structures, as was observed with the CCwt homo-dimer. The atomic positional fluctuations of C_{α} backbone atoms were recorded to identify regions of flexibility in response to the induced mutations, revealing an increase in flexibility of the CCmutE34K-R55E mutant when paired to CCwt, in the region of the E34K-R55E mutations. This can be attributed to the destabilization of the coiled-coils by the unfavorable electrostatic interactions occurring between the mutant and wild-type coiled-coils. A slight increase in the flexibility of the CCmutE34K-R55E homo-dimer is observed at N-termini and C-termini α-helical regions (Residues 1-10 & 124-134, respectively); however a DSSP secondary structure analysis revealed no loss in coiled-coil helicity in the CCmutE34K-R55E homo-dimer relative to the CCwt homo-dimer (Table 5, helicity= 71.8% and 71.6%, respectively), indicating that the α-helical dimerization interface remains stable. Analysis of α-helical specific hydrogen bonding interactions (between backbone atoms of *i* and *i*+*4* residues) revealed no significant difference in hydrogen bonding patterns between the CCmutE34K-R55E and CCwt homo-dimers (Table 5; *i, i* + *4* hydrogen bond = 33.1% and 32.1%, respectively) to indicate a loss of coiled-coil stability due to the observed atomic positional fluctuations. Together, these results indicate that the compound mutation E34K-R55E does not affect the stability of the coiled-coil, supporting the existing evidence (Figure 16) that p53-CCmutE34K-R55E forms biologically active oligomers, retaining transcriptional and tumor suppressor activity of p53.

### iv. Binding assay validates design

To specifically address whether the mutant compound CCmutE34K-R55E limited hetero-oligomerization with CCwt (found in endogenous Bcr), the widely accepted mammalian two-hybrid binding assay was carried out. Figure 18 demonstrates that formation of CCmutE34K-R55E homo-oligomers (third bar) is more favored than CCwt:CCmutE34K-R55E hetero-oligomerization (middle bar). While CCmutE34K-R55E homo-dimerization leads to preserving all 4 possible salt bridges that normally exist in the CCwt homo-dimer (see Figure 17, C vs A), CCmutE34K-R55E hetero-dimerization with CCwt may produce two new possible charge-charge repulsions (see Figure 17B). In addition, Figure 18 shows no significant difference in the binding between CCwt and CCmutE34K-R55E homo-dimers (first and third bars), as expected. This similarity in binding between CCwt vs CCmutE34K-R55E homo-dimers converges with the data obtained from our computational modeling of binding energies (Table 4; also illustrated in Figure 17), in which no change of the total number of salt bridges occur as a consequence of introducing the E34K-R55E mutation to the coiled-coil domain.

### v. p53-CCmutE34K-R55E interaction with endogenous Bcr

The mammalian two-hybrid assay illustrates the ability of the CCmutE34K-R55E compound mutation in limiting the interaction of p53-CCmutE34K-R55E with the CCwt domain of endogenous Bcr in cells. To substantiate the mammalian two-hybrid assay data, a coimmunoprecipitation assay was performed to determine if exogenously added p53-CCmutE34K-R55E has limited interaction with the CCwt domain of endogenous Bcr compared to p53-CCwt. Cell lysates transfected with either p53-CCmutE34K-R55E or p53-CCwt were immunoprecipitated. Endogenous Bcr that could potentially coimmunoprecipitate was probed using anti-CCwt antibody. Figure 19A shows that endogenous Bcr coimmunoprecipitates (i.e. interacts) with p53-CCmutE34K-R55E to a lesser extent compared to p53-CCwt. Furthermore, Bcr mean band densitometry analyses were carried out from three separate coimmunoprecipitation assays. Figure 19B shows that p53-CCwt hetero-oligomerization with endogenous Bcr is two-fold higher than the p53-CCmutE34K-R55E interaction with Bcr. These findings indicate that the E34K-R55E compound mutation reduces hetero-oligomerization with endogenous Bcr compared to CCwt interaction with Bcr, presumably due to the formation of charge-charge repulsions (see Figure 17B). It should be noted that prominent double secondary bands are detected by this anti-CCwt antibody even in untreated cell lysates.

### vi. p53-CCmutE34K-R55E induces apoptosis regardless of the p53 status or cancer cell type

To ensure that the ability of p53-CCmutE34K-R55E to induce cell death is neither dependent on endogenous p53 status nor cancer cell line specific, its apoptotic activity was tested in three different cancer cell lines; SKOV-3.ipl human ovarian cancer cells (p53-null), MCF-7 human breast cancer cells (wild type but mislocalized p53), and T47D human breast carcinoma cells (mutant p53). Figure 20A-C demonstrates that p53-CCmutE34K-R55E is capable of inducing cell death similarly to p53-CCwt and wt-p53, regardless of the endogenous p53 status or cancer cell line.

### 3. Discussion

Since domain swapping to create p53-CC can result in p53-CC interacting with endogenous Bcr, p53-CC was mutated to avoid this. The implications of possible binding of endogenous Bcr are unknown, but can be undesired, as Bcr is a ubiquitous protein involved in inflammatory pathways and cell proliferation. Since no other proteins in cells contain the Bcr CCwt motif, the sequence-specific interaction with Bcr CCwt is the only one to be concerned with eliminating.

In this report, mutations in the alternative oligomerization domain, the coiled-coil, were designed to avoid interaction with Bcr. Computationally designed and modeled mutations in the CC domain were developed to minimize interactions with native endogenous Bcr. Based on initial examination of the CC motif, several possible mutation sites were identified and summarized in Table 3 with the rationale behind designing each mutation. In addition, Figure 15 shows helical diagrams representing the CCwt, the modified CC domain (CCmut), and the hypothesized changes in electrostatic interactions (salt bridges). Two different modified coiled-coils with a single point mutation each were designed to enhance self-oligomerization. Residues Ser-41 and Gln-60 are arranged opposite of charged residues Glu-48 and Lys-39, such that the mutations S41R and Q60E serve to create additional salt bridges in the coiled-coil dimers. In the first mutant, Ser-41 was mutated to Arg, creating two new salt bridges via interaction with Glu-48 (Figure 15B). In the second mutant, Gln-60 was mutated to Glu, creating two new salt bridges via interaction with Lys-39 (Figure 15C).

Furthermore, two different modified coiled-coils with two point mutations each (compound mutants) were designed to increase binding specificity of the modified coiled-coil for itself by disrupting affinity for CCwt. By reversing the charge of existing salt bridges (dashed line highlighted in green in Figure 15D and E), a scenario is created in which charge repulsion disrupts the binding of the CCwt to the modified coiled-coils. In the p53-CCmutE34K-R55E mutant, the salt bridge between Glu-34 and Arg-55 is effectively reversed by introducing the mutations E34K and R55E. Similarly, the p53-CCmutE46K-R53E mutant features the mutations E46K and R53E to reverse the salt bridge between Glu-46 and Arg-53.

Molecular modeling, MD simulation, and free energy analysis revealed the ranking of the different modifications in terms of minimizing CCwt-CCmut hetero-oligomerization (Table 4). On one hand, free binding energy analysis by MM-PBSA revealed that CCmutS41R and CCmutQ60E can both have relatively strong homo-oligomer binding stability (Table 4, ΔG= -80.0 kcal/mol and ΔG= -76.6 kcal/mol, respectively). However, the same analysis revealed that both, CCmutS41R and CCmutQ60E, also have similar or increased binding stability for their hetero-dimers with CCwt (Table 4, ΔG= -54.8 kcal/mol and ΔG= -59.6 kcal/mol, respectively). In addition, there is no significant difference in binding energies between CCmutE46K-R53E homo-dimers and hetero-dimers (Table 4, ΔG= -58.2 kcal/mol and ΔG= -51.0 kcal/mol, respectively). On the other hand, free binding energy analysis showed that CCmutE34K-R55E can be a suitable candidate for minimizing interactions with CCwt, with CCmutE34K-R55E disfavoring interaction with CCwt. Significant difference in the binding free energies exist between the CCmutE34-R55E homo-dimer and hetero-dimer with CCwt (Table 4, ΔG= -51.9 kcal/mol and ΔG= -37.5 kcal/mol, respectively). This result indicates that CCmutE34K-R55E favors homo-oligomerization over hetero-oligomerization with CCwt of Bcr. Furthermore, the free binding energy for CCmutE34K-R55E hetero-dimer with CCwt is less favored (Table 4, ΔG= -37.5 kcal/mol) compared to that of CCwt homo-oligomer (Table 4, ΔG= -51.9 kcal/mol). To test if the mutations led to any abrogation in p53-CC activity, an *in vitro* cell death assay in which p53-CC has been proven previously to induce cell death (in T47D cells) was performed. Figure 16 showed that all mutants (p53-CCmutS41R, p53-CCmutQ60E, and p53-CCmutE46K-R53E) have lost the tumor suppressor activity of p53-CC except for the compound mutant p53-CCmutE34K-R55E. Thus, p53-CCmutE34K-R55E was the lead, eliminating the need to test the inactive mutants in the remaining experiments.

Both the mammalian two-hybrid assay (Figure 18) and the co-immunoprecipitation experiment (Figure 19) validate the computational modeling and strongly indicate that p53-CCmutE34K-R55E minimize interaction with CCwt of endogenous Bcr in cells, indicating that the interactions are indeed occurring. Finally, it was confirmed that the tumor suppressor activity (measured by apoptotic activity) of p53-CCmutE34K-R55E remains consistent regardless of endogenous p53 status or the type of cancer cell line as shown in Figure 20.

This study showed how *in silico* modeling can guide experimental design and that further iterations of *in vitro* design resulted in an enhanced version of the chimeric p53. The resulting rationally designed p53-CCmutE34K-R55E avoids binding to endogenous Bcr and yet retains potent apoptotic activity in a variety of cancer cell lines, regardless of p53 status. This construct can be used for gene therapy experiments for treatment of cancers characterized by p53 dysfunction, which represent over half of all human cancers.

### REFERENCES

1. Haupt, S., Berger, M., Goldberg, Z. & Haupt, Y. Apoptosis - the p53 network. J. Cell Sci. 116, 4077-4085, doi:10.1242/jcs.00739 (2003).
2. Marchenko, N. D., Zaika, A. & Moll, U. M. Death signal-induced localization of p53 protein to mitochondria. A potential role in apoptotic signaling. J. Biol. Chem. 275, 16202-16212 (2000).
3. Mihara, M. et al. p53 has a direct apoptogenic role at the mitochondria. Mol. Cell 11, 577-590, doi:S1097276503000509 (2003).
4. Mossalam, M., Matissek, K. J., Okal, A., Constance, J. E. & Lim, C. S. Direct induction of apoptosis using an optimal mitochondrially targeted p53. Mol. Pharm. 9, 1449-1458, doi:10.1021/mp3000259 (2012).
5. McLure, K. G. & Lee, P. W. How p53 binds DNA as a tetramer. EMBO J. 17, 3342-3350, doi:10.1093/emboj/17.12.3342 (1998).
6. Lacroix, M., Toillon, R. A. & Leclercq, G. p53 and breast cancer, an update. Endocr. Relat. Cancer 13, 293-325, doi:13/2/29310.1677/erc.1.01172 (2006).
7. Willis, A., Jung, E. J., Wakefield, T. & Chen, X. Mutant p53 exerts a dominant negative effect by preventing wild-type p53 from binding to the promoter of its target genes. Oncogene 23, 2330-2338 (2004).
8. Shaulsky, G., Goldfinger, N., Tosky, M. S., Levine, A. J. & Rotter, V. Nuclear localization is essential for the activity of p53 protein. Oncogene 6, 2055-2065 (1991).
9. Baptiste, N. & Prives, C. p53 in the cytoplasm: a question of overkill? Cell 116, 487-489, doi:S0092867404001643 (2004).
10. Peng, Z. Current status of gendicine in China: recombinant human Ad-p53 agent for treatment of cancers. Human gene therapy 16, 1016-1027, doi:10.1089/hum.2005.16.1016 (2005).
11. de Vries, A. et al. Targeted point mutations of p53 lead to dominant-negative inhibition of wild-type p53 function. Proc. Natl. Acad. Sci. U S A 99, 2948-2953, doi:10.1073/pnas.052713099 (2002).
12. Soussi, T. p53 alterations in human cancer: more questions than answers. Oncogene 26, 2145-2156, doi:121028010.1038/sj.onc.1210280 (2007).
13. Soussi, T., Ishioka, C., Claustres, M. & Beroud, C. Locus-specific mutation databases: pitfalls and good practice based on the p53 experience. Nat. Rev. Cancer 6, 83-90, doi:nrc178310.1038/nrc1783 (2006).
14. Soussi, T. & Wiman, K. G. Shaping genetic alterations in human cancer: the p53 mutation paradigm. Cancer Cell 12, 303-312, doi:S1535-6108(07)00270-X10.1016/j.ccr.2007.10.001 (2007).
15. Goh, A. M., Coffill, C. R. & Lane, D. P. The role of mutant p53 in human cancer. J. Path. 223, 116-126, doi:10.1002/path.2784 (2011).
16. Weinberg, R. L., Freund, S. M., Veprintsev, D. B., Bycroft, M. & Fersht, A. R. Regulation of DNA binding of p53 by its C-terminal domain. J. Mol. Biol. 342, 801-811, doi:10.1016/j.jmb.2004.07.042 (2004).
17. Milner, J. & Medcalf, E. A. Cotranslation of activated mutant p53 with wild type drives the wild-type p53 protein into the mutant conformation. Cell 65, 765-774 (1991).
18. Srivastava, S., Wang, S., Tong, Y. A., Hao, Z. M. & Chang, E. H. Dominant negative effect of a germ-line mutant p53: a step fostering tumorigenesis. Canc. Res. 53, 4452-4455 (1993).
19. Kern, S. E. et al. Oncogenic forms of p53 inhibit p53-regulated gene expression. Science 256, 827-830 (1992).
20. Lee, Ming K. et al. Cell-type, Dose, and Mutation-type Specificity Dictate Mutant p53 Functions In Vivo. Cancer Cell 22, 751-764, doi:http://dx.doi.org/10.1016/j.ccr.2012.10.022 (2012).
21. Waterman, M. J., Waterman, J. L. & Halazonetis, T. D. An engineered four-stranded coiled coil substitutes for the tetramerization domain of wild-type p53 and alleviates transdominant inhibition by tumor-derived p53 mutants. Cancer Res. 56, 158-163 (1996).
22. Jeffrey, P. D., Gorina, S. & Pavletich, N. P. Crystal structure of the tetramerization domain of the p53 tumor suppressor at 1.7 angstroms. Science 267, 1498-1502 (1995).
23. Lee, W. et al. Solution structure of the tetrameric minimum transforming domain of p53. Nat. Struct. Biol. 1, 877-890 (1994).
24. Wichmann, C. et al. Dimer-tetramer transition controls RUNX1/ETO leukemogenic activity. Blood 116, 603-613, doi:10.1182/blood-2009-10-248047 (2010).
25. Zhao, X., Ghaffari, S., Lodish, H., Malashkevich, V. N. & Kim, P. S. Structure of the Bcr-Abl oncoprotein oligomerization domain. Nat Struct Biol 9, 117-120, doi:10.1038/nsb747 (2002).
26. Dixon, A. S. et al. Disruption of Bcr-Abl coiled coil oligomerization by design. J. Biol. Chem. 286, 27751-27760, doi:10.1074/jbc.M111.264903 (2011).
27. Di Como, C. J., Gaiddon, C. & Prives, C. p73 function is inhibited by tumor-derived p53 mutants in mammalian cells. Molecular and Cellular Biology 19, 1438-1449 (1999).
28. Dong, P. et al. p53 dominant-negative mutant R273H promotes invasion and migration of human endometrial cancer HHUA cells. Clin Exp Metastasis 24, 471-483, doi:10.1007/s10585-007-9084-8 (2007).
29. Dixon, A. S. et al. Controlling subcellular localization to alter function: Sending oncogenic Bcr-Abl to the nucleus causes apoptosis. J. Control. Release 140, 245-249, doi:10.1016/j.jconrel.2009.06.026 (2009).
30. Reaz, S., Mossalam, M., Okal, A. & Lim, C. S. A Single Mutant, A276S of p53, Turns the Switch to Apoptosis. Mol Pharm, doi:10.1021/mp300598k (2013).
31. el-Deiry, W. S. et al. WAF1, a potential mediator of p53 tumor suppression. Cell 75, 817-825 (1993).
32. Yu, J., Zhang, L., Hwang, P. M., Kinzler, K. W. & Vogelstein, B. PUMA induces the rapid apoptosis of colorectal cancer cells. Mol. Cell 7, 673-682 (2001).
33. Brockschmidt, C. et al. Anti-apoptotic and growth-stimulatory functions of CK1 delta and epsilon in ductal adenocarcinoma of the pancreas are inhibited by IC261 in vitro and in vivo. Gut 57, 799-806, doi:10.1136/gut.2007.123695 (2008).
34. Tomita, Y. et al. WT p53, but not tumor-derived mutants, bind to Bcl2 via the DNA binding domain and induce mitochondrial permeabilization. The Journal of biological chemistry 281, 8600-8606, doi:10.1074/jbc.M507611200 (2006).
35. Waldman, T., Kinzler, K. W. & Vogelstein, B. p21 is necessary for the p53-mediated G1 arrest in human cancer cells. Cancer Res. 55, 5187-5190 (1995).
36. Loo, D. T. & Rillema, J. R. Measurement of cell death. Methods Cell Biol 57, 251-264 (1998).
37. Koopman, G. et al. Annexin V for flow cytometric detection of phosphatidylserine expression on B cells undergoing apoptosis. Blood 84, 1415-1420 (1994).
38. Vermes, I., Haanen, C., Steffens-Nakken, H. & Reutelingsperger, C. A novel assay for apoptosis. Flow cytometric detection of phosphatidylserine expression on early apoptotic cells using fluorescein labelled Annexin V. J. Immun. Meth. 184, 39-51 (1995).
39. Schmid, I., Krall, W. J., Uittenbogaart, C. H., Braun, J. & Giorgi, J. V. Dead cell discrimination with 7-amino-actinomycin D in combination with dual color immunofluorescence in single laser flow cytometry. Cytometry 13, 204-208, doi:10.1002/cyto.990130216 (1992).
40. Serrano, M. J. et al. Evaluation of a gemcitabine-doxorubicin-paclitaxel combination schedule through flow cytometry assessment of apoptosis extent induced in human breast cancer cell lines. Jap. J. Cancer Res. 93, 559-566 (2002).
41. Goodrum, F. D. & Ornelles, D. A. p53 status does not determine outcome of E1B 55-kilodalton mutant adenovirus lytic infection. J. Virol. 72, 9479-9490 (1998).
42. Bartek, J., Iggo, R., Gannon, J. & Lane, D. P. Genetic and immunochemical analysis of mutant p53 in human breast cancer cell lines. Oncogene 5, 893-899 (1990).
43. Mooney, L. M., Al-Sakkaf, K. A., Brown, B. L. & Dobson, P. R. Apoptotic mechanisms in T47D and MCF-7 human breast cancer cells. Br J Cancer 87, 909-917, doi:10.1038/sj.bjc.6600541 (2002).
44. Bodner, S. M. et al. Expression of mutant p53 proteins in lung cancer correlates with the class of p53 gene mutation. Oncogene 7, 743-749 (1992).
45. Kawaguchi, T. et al. The relationship among p53 oligomer formation, structure and transcriptional activity using a comprehensive missense mutation library. Oncogene 24, 6976-6981, doi:10.1038/sj.onc.1208839 (2005).
46. Yahagi, N. et al. p53 Activation in adipocytes of obese mice. J. Biol. Chem. 278, 25395-25400, doi:10.1074/jbc.M302364200 (2003).
47. Brunelle, J. K. & Letai, A. Control of mitochondrial apoptosis by the Bcl-2 family. J. Cell Sci. 122, 437-441, doi:10.1242/jcs.031682 (2009).
48. Wang, W., Cheng, B., Miao, L., Mei, Y. & Wu, M. Mutant p53-R273H gains new function in sustained activation of EGFR signaling via suppressing miR-27a expression. Cell death & disease 4, e574, doi:10.1038/cddis.2013.97 (2013).
49. Lim, L. Y., Vidnovic, N., Ellisen, L. W. & Leong, C. O. Mutant p53 mediates survival of breast cancer cells. British journal of cancer 101, 1606-1612, doi:10.1038/sj.bjc.6605335 (2009).
50. Taylor, C. M. & Keating, A. E. Orientation and oligomerization specificity of the Bcr coiled-coil oligomerization domain. Biochem. 44, 16246-16256, doi:10.1021/bi051493t (2005).
51. Maru, Y. & Witte, O. N. The BCR gene encodes a novel serine/threonine kinase activity within a single exon. Cell 67, 459-468 (1991).
52. Alexis, J. D. et al. Bcr kinase activation by angiotensin II inhibits peroxisome-proliferator-activated receptor gamma transcriptional activity in vascular smooth muscle cells. Circ Res 104, 69-78, doi:10.1161/CIRCRESAHA.108.188409 (2009).
53. Woessner, D. W., Lim, C. S. & Deininger, M. W. Development of an effective therapy for chronic myelogenous leukemia. Cancer J 17, 477-486, doi:10.1097/PPO.0b013e318237e5b7 (2011).
54. Melo, J. V. & Barnes, D. J. Chronic myeloid leukaemia as a model of disease evolution in human cancer. Nat Rev Cancer 7, 441-453, doi:10.1038/nrc2147 (2007).
55. Galea, C., Bowman, P. & Kriwacki, R. W. Disruption of an intermonomer salt bridge in the p53 tetramerization domain results in an increased propensity to form amyloid fibrils. Protein Sci. 14, 2993-3003, doi:10.1110/ps.051622005 (2005).
56. Gu W, Roeder RG. Activation of p53 Sequence-Specific DNA Binding by Acetylation of the p53 C-Terminal Domain. Cell 1997; 90(4): 595-606.
57. Vogelstein B, Kinzler KW. p53 function and dysfunction. Cell 1992; 70(4): 523-6.
58. Vogelstein B, Lane D, Levine AJ. Surfing the p53 network. Nature 2000; 408(6810): 307-10.
59. Levine AJ, Momand J, Finlay CA. The p53 tumour suppressor gene. Nature 1991; 351(6326): 453-6.
60. Moll UM, Riou G, Levine AJ. Two distinct mechanisms alter p53 in breast cancer: mutation and nuclear exclusion. PNAS 1992; 89(15): 7262-6.
61. Kussie PH, Gorina S, Marechal V, Elenbaas B, Moreau J, Levine AJ et al. Structure of the MDM2 oncoprotein bound to the p53 tumor suppressor transactivation domain. Science 1996; 274(5289): 948-53.
62. Okal A, Reaz S, Lim C. Cancer Biology: Some Causes for a Variety of Different Diseases. In: Bae YH, Mrsny RJ, Park K (eds). Cancer Targeted Drug Delivery. Springer New York, 2013, pp 121-159.
63. Waterman MJ, Waterman JL, Halazonetis TD. An engineered four-stranded coiled coil substitutes for the tetramerization domain of wild-type p53 and alleviates transdominant inhibition by tumor-derived p53 mutants. Cancer Res 1996; 56(1): 158-63.
64. Willis A, Jung EJ, Wakefield T, Chen X. Mutant p53 exerts a dominant negative effect by preventing wild-type p53 from binding to the promoter of its target genes. Oncogene 2004; 23(13): 2330-8.
65. Chan WM, Siu WY, Lau A, Poon RY. How many mutant p53 molecules are needed to inactivate a tetramer? Molecular and Cellular Biology 2004; 24(8): 3536-51.
66. Zeimet AG, Marth C. Why did p53 gene therapy fail in ovarian cancer? The Lancet Oncology 2003; 4(7): 415-22.
67. Okal A, Mossalam M, Matissek KJ, Dixon AS, Moos PJ, Lim CS. A Chimeric p53 Evades Mutant p53 Transdominant Inhibition in Cancer Cells. Mol Pharm 2013; 10(10): 3922-33.
68. Junk DJ, Vrba L, Watts GS, Oshiro MM, Martinez JD, Futscher BW. Different mutant/wild-type p53 combinations cause a spectrum of increased invasive potential in nonmalignant immortalized human mammary epithelial cells. Neoplasia 2008; 10(5): 450-61.
69. Lim LY, Vidnovic N, Ellisen LW, Leong CO. Mutant p53 mediates survival of breast cancer cells. British Journal of Cancer 2009; 101(9): 1606-12.
70. Schmid I, Krall WJ, Uittenbogaart CH, Braun J, Giorgi JV. Dead cell discrimination with 7-amino-actinomycin D in combination with dual color immunofluorescence in single laser flow cytometry. Cytometry 1992; 13(2): 204-8.
71. Serrano MJ, Sanchez-Rovira P, Algarra I, Jaen A, Lozano A, Gaforio JJ. Evaluation of a gemcitabine-doxorubicin-paclitaxel combination schedule through flow cytometry assessment of apoptosis extent induced in human breast cancer cell lines. Japanese journal of cancer research : Gann 2002; 93(5): 559-66.
72. Matissek KJ, Mossalam M, Okal A, Lim CS. The DNA binding domain of p53 is sufficient to trigger a potent apoptotic response at the mitochondria. Mol Pharm 2013; 10(10): 3592-602.
73. O'Reilly CM, Fogarty KE, Drummond RM, Tuft RA, Walsh JV, Jr. Quantitative analysis of spontaneous mitochondrial depolarizations. Biophys J 2003; 85(5): 3350-7.
74. Ricci JE, Gottlieb RA, Green DR. Caspase-mediated loss of mitochondrial function and generation of reactive oxygen species during apoptosis. J Cell Biol 2003; 160(1): 65-75.
75. Chipuk JE, Green DR. How do BCL-2 proteins induce mitochondrial outer membrane permeabilization? Trends in Cell Biology 2008; 18(4): 157-64.
76. Mossalam M, Matissek KJ, Okal A, Constance JE, Lim CS. Direct Induction of Apoptosis Using an Optimal Mitochondrially Targeted p53. Mol Pharm 2012; 9(5): 1449-58.
77. Slee EA, Adrain C, Martin SJ. Executioner caspase-3, -6, and -7 perform distinct, non-redundant roles during the demolition phase of apoptosis. The Journal of biological chemistry 2001; 276(10): 7320-6.
78. Lakhani SA, Masud A, Kuida K, Porter GA, Jr., Booth CJ, Mehal WZ et al. Caspases 3 and 7: key mediators of mitochondrial events of apoptosis. Science 2006; 311(5762): 847-51.
79. Koopman G, Reutelingsperger CP, Kuijten GA, Keehnen RM, Pals ST, van Oers MH. Annexin V for flow cytometric detection of phosphatidylserine expression on B cells undergoing apoptosis. Blood 1994; 84(5): 1415-20.
80. Vermes I, Haanen C, Steffens-Nakken H, Reutelingsperger C. A novel assay for apoptosis. Flow cytometric detection of phosphatidylserine expression on early apoptotic cells using fluorescein labelled Annexin V. J Immunol Methods 1995; 184(1): 39-51.
81. Metzger-Filho O, Tutt A, de Azambuja E, Saini KS, Viale G, Loi S et al. Dissecting the heterogeneity of triple-negative breast cancer. J Clin Oncol 2012; 30(15): 1879-87.
82. Price R, Gustafson J, Greish K, Cappello J, McGill L, Ghandehari H. Comparison of silk-elastinlike protein polymer hydrogel and poloxamer in matrix-mediated gene delivery. Int J Pharm 2012; 427(1): 97-104.
83. Killion J, Radinsky R, Fidler I. Orthotopic Models are Necessary to Predict Therapy of Transplantable Tumors in Mice. Cancer and Metastasis Reviews 1998; 17(3): 279-284.
84. Bibby MC. Orthotopic models of cancer for preclinical drug evaluation: advantages and disadvantages. Eur J Cancer 2004; 40(6): 852-7.
85. Vantyghem S, Allan A, Postenka C, Al-Katib W, Keeney M, Tuck A et al. A New Model for Lymphatic Metastasis: Development of a Variant of the MDA-MB-468 Human Breast Cancer Cell Line that Aggressively Metastasizes to Lymph Nodes. Clin Exp Metastasis 2005; 22(4): 351-361.
86. Garlich JR, De P, Dey N, Su JD, Peng X, Miller A et al. A vascular targeted pan phosphoinositide 3-kinase inhibitor prodrug, SF1126, with antitumor and antiangiogenic activity. Cancer Research 2008; 68(1): 206-15.
87. Laptenko O, Beckerman R, Freulich E, Prives C. p53 binding to nucleosomes within the p21 promoter in vivo leads to nucleosome loss and transcriptional activation. Proceedings of the National Academy of Sciences of the United States of America 2011; 108(26): 10385-90.
88. Tait SW, Green DR. Mitochondria and cell death: outer membrane permeabilization and beyond. Nature reviews. Molecular cell biology 2010; 11(9): 621-32.
89. Fulda S, Debatin KM. Extrinsic versus intrinsic apoptosis pathways in anticancer chemotherapy. Oncogene 2006; 25(34): 4798-811.
90. el-Deiry WS, Harper JW, O'Connor PM, Velculescu VE, Canman CE, Jackman J et al. WAF1/CIP1 is induced in p53-mediated G1 arrest and apoptosis. Cancer Research 1994; 54(5): 1169-74.
91. Waldman T, Kinzler KW, Vogelstein B. p21 is necessary for the p53-mediated G1 arrest in human cancer cells. Cancer Research 1995; 55(22): 5187-90.
92. Waldman T, Zhang Y, Dillehay L, Yu J, Kinzler K, Vogelstein B et al. Cell-cycle arrest versus cell death in cancer therapy. Nature medicine 1997; 3(9): 1034-6.
93. Lane DP, Cheok CF, Lain S. p53-based cancer therapy. Cold Spring Harb Perspect Biol 2010; 2(9): a001222.
94. Wichmann C, Becker Y, Chen-Wichmann L, Vogel V, Vojtkova A, Herglotz J et al. Dimer-tetramer transition controls RUNX1/ETO leukemogenic activity. Blood 2010; 116(4): 603-13.
95. Yamada T, Hiraoka Y, Ikehata M, Kimbara K, Avner BS, Das Gupta TK et al. Apoptosis or growth arrest: Modulation of tumor suppressor p53's specificity by bacterial redox protein azurin. Proceedings of the National Academy of Sciences of the United States of America 2004; 101(14): 4770-5.
96. Chen X, Ko LJ, Jayaraman L, Prives C. p53 levels, functional domains, and DNA damage determine the extent of the apoptotic response of tumor cells. Genes & development 1996; 10(19): 2438-51.
97. Lane DP, Cheok CF, Lain S. p53-based cancer therapy. Cold Spring Harb Perspect Biol 2010; 2(9): a001222.
98. Mossalam M, Matissek KJ, Okal A, Constance JE, Lim CS. Direct induction of apoptosis using an optimal mitochondrially targeted p53. Mol. Pharm. 2012; 9(5): 1449-58.
99. Krohn AJ, Wahlbrink T, Prehn JH. Mitochondrial depolarization is not required for neuronal apoptosis. The Journal of neuroscience : the official journal of the Society for Neuroscience 1999; 19(17): 7394-404.
100. Salama ME, Rajan Mariappan M, Inamdar K, Tripp SR, Perkins SL. The value of CD23 expression as an additional marker in distinguishing mediastinal (thymic) large B-cell lymphoma from Hodgkin lymphoma. Int J Surg Pathol 2010; 18(2): 121-8.
101. Reaz S, Mossalam M, Okal A, Lim CS. A Single Mutant, A276S of p53, Turns the Switch to Apoptosis. Mol Pharm 2013.
102. Woessner DW, Lim CS. Disrupting BCR-ABL in combination with secondary leukemia-specific pathways in CML cells leads to enhanced apoptosis and decreased proliferation. Mol Pharm 2013; 10(1): 270-7.
103. Levine, A. J.; Oren, M. The first 30 years of p53: growing ever more complex. Nat Rev Cancer 2009, 9, (10), 749-58.
104. Hollstein, M.; Sidransky, D.; Vogelstein, B.; Harris, C. C. p53 mutations in human cancers. Science 1991, 253, (5015), 49-53.
105. Turner, N.; Moretti, E.; Siclari, O.; Migliaccio, I.; Santarpia, L.; D'Incalci, M.; Piccolo, S.; Veronesi, A.; Zambelli, A.; Del Sal, G.; Di Leo, A. Targeting triple negative breast cancer: Is p53 the answer? Cancer Treat Rev 2013, 39, (5), 541-50.
106. Waterman, M. J.; Waterman, J. L.; Halazonetis, T. D. An engineered four-stranded coiled coil substitutes for the tetramerization domain of wild-type p53 and alleviates transdominant inhibition by tumor-derived p53 mutants. Cancer Res 1996, 56, (1), 158-63.
107. Willis, A.; Jung, E. J.; Wakefield, T.; Chen, X. Mutant p53 exerts a dominant negative effect by preventing wild-type p53 from binding to the promoter of its target genes. Oncogene 2004, 23, (13), 2330-8.
108. Kern, S.; Kinzler, K.; Bruskin, A.; Jarosz, D.; Friedman, P.; Prives, C.; Vogelstein, B. Identification of p53 as a sequence-specific DNA-binding protein. Science 1991, 252, (5013), 1708-1711.
109. Milner, J.; Medcalf, E. A. Cotranslation of activated mutant p53 with wild type drives the wild-type p53 protein into the mutant conformation. Cell 1991, 65, (5), 765-74.
110. Srivastava, S.; Wang, S.; Tong, Y. A.; Hao, Z. M.; Chang, E. H. Dominant negative effect of a germ-line mutant p53: a step fostering tumorigenesis. Cancer Research 1993, 53, (19), 4452-5.
111. Okal, A.; Mossalam, M.; Matissek, K. J.; Dixon, A. S.; Moos, P. J.; Lim, C. S. A Chimeric p53 Evades Mutant p53 Transdominant Inhibition in Cancer Cells. Mol Pharm 2013, 10, (10), 3922-33.
112. Jeffrey, P.; Gorina, S.; Pavletich, N. Crystal structure of the tetramerization domain of the p53 tumor suppressor at 1.7 angstroms. Science 1995, 267, (5203), 1498-1502.
113. Zhao, X.; Ghaffari, S.; Lodish, H.; Malashkevich, V. N.; Kim, P. S. Structure of the Bcr-Abl oncoprotein oligomerization domain. Nat Struct Biol 2002, 9, (2), 117-20.
114. Deininger, M. W.; Goldman, J. M.; Melo, J. V. The molecular biology of chronic myeloid leukemia. Blood 2000, 96, (10), 3343-56.
115. Woessner, D. W.; Lim, C. S.; Deininger, M. W. Development of an effective therapy for chronic myelogenous leukemia. Cancer J 2011, 17, (6), 477-86.
116. Alexis, J. D.; Wang, N.; Che, W.; Lerner-Marmarosh, N.; Sahni, A.; Korshunov, V. A.; Zou, Y.; Ding, B.; Yan, C.; Berk, B. C.; Abe, J. Bcr kinase activation by angiotensin II inhibits peroxisome-proliferator-activated receptor gamma transcriptional activity in vascular smooth muscle cells. Circ Res 2009, 104, (1), 69-78.
117. Yi, S. J.; Groffen, J.; Heisterkamp, N. Bcr is a substrate for Transglutaminase 2 crosslinking activity. BMC Biochem 2011, 12, 8.
118. Alexis, J. D.; Wang, N.; Che, W.; Lerner-Marmarosh, N.; Sahni, A.; Korshunov, V. A.; Zou, Y.; Ding, B.; Yan, C.; Berk, B. C.; Abe, J. Bcr kinase activation by angiotensin II inhibits peroxisome-proliferator-activated receptor gamma transcriptional activity in vascular smooth muscle cells. Circ Res 2009, 104, (1), 69-78.
119. Mason, J. M.; Arndt, K. M. Coiled coil domains: stability, specificity, and biological implications. Chembiochem 2004, 5, (2), 170-6.
120. Lupas, A. Coiled coils: new structures and new functions. Trends Biochem Sci 1996, 21, (10), 375-382.
121. Burkhard, P.; Stetefeld, J.; Strelkov, S. V. Coiled coils: a highly versatile protein folding motif. Trends in Cell Biology 2001, 11, (2), 82-88.
122. Pettersen, E. F.; Goddard, T. D.; Huang, C. C.; Couch, G. S.; Greenblatt, D. M.; Meng, E. C.; Ferrin, T. E. UCSF Chimera--a visualization system for exploratory research and analysis. Journal of Computational Chemistry 2004, 25, (13), 1605-12.
123. Shapovalov, M. V.; Dunbrack, R. L., Jr. A smoothed backbone-dependent rotamer library for proteins derived from adaptive kernel density estimates and regressions. Structure 2011, 19, (6), 844-58.
124. Hornak, V.; Abel, R.; Okur, A.; Strockbine, B.; Roitberg, A.; Simmerling, C. Comparison of multiple Amber force fields and development of improved protein backbone parameters. Proteins 2006, 65, (3), 712-25.
125. Cornell, W. D.; Cieplak, P.; Bayly, C. I.; Gould, I. R.; Merz, K. M.; Ferguson, D. M.; Spellmeyer, D. C.; Fox, T.; Caldwell, J. W.; Kollman, P. A. A Second Generation Force Field for the Simulation of Proteins, Nucleic Acids, and Organic Molecules. Journal of the American Chemical Society 1995, 117, (19), 5179-5197.
126. Jorgensen, W. L.; Chandrasekhar, J.; Madura, J. D.; Impey, R. W.; Klein, M. L. Comparison of simple potential functions for simulating liquid water. The Journal of Chemical Physics 1983, 79, (2), 926-935.
127. Joung, I. S.; Cheatham, T. E., 3rd. Determination of alkali and halide monovalent ion parameters for use in explicitly solvated biomolecular simulations. J Phys Chem B 2008, 112, (30), 9020-41.
128. Berendsen, H. J. C.; Postma, J. P. M.; van Gunsteren, W. F.; DiNola, A.; Haak, J. R. Molecular dynamics with coupling to an external bath. The Journal of Chemical Physics 1984, 81, (8), 3684-3690.
129. D.A. Case, T. A. D., T.E. Cheatham, III, C.L. Simmerling, J. Wang, R.E. Duke, R. Luo, R.C. Walker, W. Zhang, K.M. Merz, B. Roberts, S. Hayik, A. Roitberg, G. Seabra, J. Swails, A.W. Goetz, I. Kolossváry, K.F. Wong, F. Paesani, J. Vanicek, R.M. Wolf, J. Liu, X. Wu, S.R. Brozell, T. Steinbrecher, H. Gohlke, Q. Cai, X. Ye, J. Wang, M.-J. Hsieh, G. Cui, D.R. Roe, D.H. Mathews, M.G. Seetin, R. Salomon-Ferrer, C. Sagui, V. Babin, T. Luchko, S. Gusarov, A. Kovalenko, and P.A. Kollman (2012), AMBER 12, University of California, San Francisco.
130. Pearlman, D. A.; Case, D. A.; Caldwell, J. W.; Ross, W. S.; Cheatham Iii, T. E.; DeBolt, S.; Ferguson, D.; Seibel, G.; Kollman, P. AMBER, a package of computer programs for applying molecular mechanics, normal mode analysis, molecular dynamics and free energy calculations to simulate the structural and energetic properties of molecules. Computer Physics Communications 1995, 91, (1-3), 1-41.
131. Essmann, U.; Perera, L.; Berkowitz, M. L.; Darden, T.; Lee, H.; Pedersen, L. G. A smooth particle mesh Ewald method. The Journal of Chemical Physics 1995, 103, (19), 8577-8593.
132. Ryckaert, J.-P.; Ciccotti, G.; Berendsen, H. J. C. Numerical integration of the cartesian equations of motion of a system with constraints: molecular dynamics of n-alkanes. Journal of Computational Physics 1977, 23, (3), 327-341.
133. Roe, D. R.; Cheatham, T. E. PTRAJ and CPPTRAJ: Software for Processing and Analysis of Molecular Dynamics Trajectory Data. Journal of Chemical Theory and Computation 2013, 9, (7), 3084-3095.
134. Shao, J.; Tanner, S. W.; Thompson, N.; Cheatham, T. E. Clustering Molecular Dynamics Trajectories: 1. Characterizing the Performance of Different Clustering Algorithms. Journal of Chemical Theory and Computation 2007, 3, (6), 2312-2334.
135. Rost, B.; Sander, C. Prediction of protein secondary structure at better than 70% accuracy. Journal of Molecular Biology 1993, 232, (2), 584-99.
136. Miller, B. R.; McGee, T. D.; Swails, J. M.; Homeyer, N.; Gohlke, H.; Roitberg, A. E. MMPBSA.py: An Efficient Program for End-State Free Energy Calculations. Journal of Chemical Theory and Computation 2012, 8, (9), 3314-3321.
137. Kollman, P. A.; Massova, I.; Reyes, C.; Kuhn, B.; Huo, S.; Chong, L.; Lee, M.; Lee, T.; Duan, Y.; Wang, W.; Donini, O.; Cieplak, P.; Srinivasan, J.; Case, D. A.; Cheatham, T. E., 3rd. Calculating structures and free energies of complex molecules: combining molecular mechanics and continuum models. Acc Chem Res 2000, 33, (12), 889-97.
138. Dixon, A. S.; Pendley, S. S.; Bruno, B. J.; Woessner, D. W.; Shimpi, A. A.; Cheatham, T. E., 3rd; Lim, C. S. Disruption of Bcr-Abl coiled coil oligomerization by design. J Biol Chem 2011, 286, (31), 27751-60.
139. Mossalam, M.; Matissek, K. J.; Okal, A.; Constance, J. E.; Lim, C. S. Direct induction of apoptosis using an optimal mitochondrially targeted p53. Mol. Pharm. 2012, 9, (5), 1449-58.
140. Mossalam, M.; Matissek, K. J.; Okal, A.; Constance, J. E.; Lim, C. S. Direct Induction of Apoptosis Using an Optimal Mitochondrially Targeted p53. Mol Pharm 2012, 9, (5), 1449-58.
141. Dixon, A. S.; Miller, G. D.; Bruno, B. J.; Constance, J. E.; Woessner, D. W.; Fidler, T. P.; Robertson, J. C.; Cheatham, T. E.; Lim, C. S. Improved coiled-coil design enhances interaction with Bcr-Abl and induces apoptosis. Mol Pharm 2012, 9, (1), 187-95.
142. Woessner, D. W.; Lim, C. S. Disrupting BCR-ABL in combination with secondary leukemia-specific pathways in CML cells leads to enhanced apoptosis and decreased proliferation. Mol Pharm 2013, 10, (1), 270-7.
143. Taylor, C. M.; Keating, A. E. Orientation and oligomerization specificity of the Bcr coiled-coil oligomerization domain. Biochemistry 2005, 44, (49), 16246-56.
144. Schmid, I.; Krall, W. J.; Uittenbogaart, C. H.; Braun, J.; Giorgi, J. V. Dead cell discrimination with 7-amino-actinomycin D in combination with dual color immunofluorescence in single laser flow cytometry. Cytometry 1992, 13, (2), 204-8.
145. Serrano, M. J.; Sanchez-Rovira, P.; Algarra, I.; Jaen, A.; Lozano, A.; Gaforio, J. J. Evaluation of a gemcitabine-doxorubicin-paclitaxel combination schedule through flow cytometry assessment of apoptosis extent induced in human breast cancer cell lines. Japanese journal of cancer research : Gann 2002, 93, (5), 559-66.
146. Emdad, L.; Sarkar, D.; Lebedeva, I. V.; Su, Z. Z.; Gupta, P.; Mahasreshti, P. J.; Dent, P.; Curiel, D. T.; Fisher, P. B. Ionizing radiation enhances adenoviral vector expressing mda-7/IL-24-mediated apoptosis in human ovarian cancer. Journal of Cellular Physiology 2006, 208, (2), 298-306.
147. Mooney, L. M.; Al-Sakkaf, K. A.; Brown, B. L.; Dobson, P. R. Apoptotic mechanisms in T47D and MCF-7 human breast cancer cells. Br J Cancer 2002, 87, (8), 909-17.
148. Tomita, Y.; Marchenko, N.; Erster, S.; Nemajerova, A.; Dehner, A.; Klein, C.; Pan, H.; Kessler, H.; Pancoska, P.; Moll, U. M. WT p53, but not tumor-derived mutants, bind to Bcl2 via the DNA binding domain and induce mitochondrial permeabilization. J Biol Chem 2006, 281, (13), 8600-6.
149. Dixon, A. S.; Pendley, S. S.; Bruno, B. J.; Woessner, D. W.; Shimpi, A. A.; Cheatham, T. E.; Lim, C. S. Disruption of BCR-ABL coiled-coil oligomerization by design. Journal of Biological Chemistry 2011.

## Claims

1. A peptide comprising a partial p53 peptide and a mutated Bcr coiled-coil domain, wherein the partial p53 peptide does not have a functional tetramerization domain but retains tumor suppressor activity and the mutated Bcr coiled-coil domain comprises amino acid substitutions and deletions but retains tetramerization activity,
wherein the mutated Bcr coiled-coil domain comprises mutations at residues 34 and 55 of SEQ ID NO:4 and has a lysine at position 34 and a glutamic acid at position 55 of SEQ ID NO:4 or comprises the sequence of SEQ ID NO:5, wherein the mutated Bcr coiled-coil domains comprise at least 50% sequence identity to SEQ ID NO:4.

2. The peptide of claim 1 wherein the mutated Bcr coiled-coil domain is linked to the C-terminus of the DNA binding domain of the partial p53 peptide or is located between the DNA binding domain of the partial p53 peptide and the C-terminus of the peptide.

3. A nucleic acid sequence encoding the peptides of any of the preceding claims.

4. A vector comprising the nucleic acid of claims 3.

5. The vector of claim 4, wherein the vector is a viral vector, preferably an adenoviral vector.

6. Composition comprising a peptide according to any of the claims 1 or 2 for use in inducing apoptosis in the treatment of a patient.

7. Composition comprising a peptide according to any of the claims 1 or 2 for use in suppressing a tumor in a patient.

8. A composition comprising any of the peptides of claims 1 or 2 or any of the nucleic acid sequences of claim 3.

9. The composition of any of the claims 6 to 8, further comprising a pharmaceutically acceptable carrier.

10. Peptide according to any of the claims 1 or 2 for use in the treatment of cancer or hyperproliferative disorder.

11. The peptide for use according to claim 10, wherein the cancer comprises breast cancer, triple negative breast cancer, ovarian cancer, or any blood cancer.

12. The peptide for use according to claim 10, wherein the hyperproliferative disorder comprises cancer, wherein cancer comprises breast cancer, triple negative breast cancer, ovarian cancer, or any blood cancer.

13. A recombinant cell comprising any of the nucleic acids of claim 3.

## Patentansprüche

1. Peptid, das ein partielles p53-Peptid und eine mutierte Bcr-Coiled-Coil-Domäne umfasst, wobei das partielle p53-Peptid keine funktionelle Tetramerisierungsdomäne aufweist, aber die Tumorsuppressoraktivität beibehält, und die mutierte Bcr-Coiled-Coil-Domäne Aminosäuresubstitutionen und -deletionen umfasst, aber die Tetramerisierungsaktivität beibehält,
wobei die mutierte Bcr-Coiled-Coil-Domäne Mutationen an den Resten 34 und 55 von SEQ ID NO: 4 umfasst und ein Lysin an Position 34 und eine Glutaminsäure an Position 55 von SEQ ID NO: 4 aufweist oder die Sequenz von SEQ ID NO: 5 umfasst, wobei die mutierten Bcr-Coiled-Coil-Domänen mindestens 50% Sequenzidentität zu SEQ ID NO: 4 aufweisen.

2. Peptid von Anspruch 1, wobei die mutierte Bcr-Coiled-Coil-Domäne mit dem C-Terminus der DNA-Bindungsdomäne des partiellen p53-Peptids verbunden ist oder sich zwischen der DNA-Bindungsdomäne des partiellen p53-Peptids und dem C-Terminus des Peptids befindet.

3. Nukleinsäuresequenz, die die Peptide nach einem beliebigen der vorhergehenden Ansprüche kodiert.

4. Vektor, der die Nukleinsäure nach Anspruch 3 umfasst.

5. Vektor nach Anspruch 4, wobei der Vektor ein viraler Vektor, vorzugsweise ein adenoviraler Vektor ist.

6. Zusammensetzung, umfassend ein Peptid nach beliebigen der Ansprüche 1 oder 2 zur Verwendung bei der Induktion von Apoptose bei der Behandlung eines Patienten.

7. Zusammensetzung, umfassend ein Peptid nach beliebigen der Ansprüche 1 oder 2 zur Verwendung bei der Unterdrückung eines Tumors in einem Patienten.

8. Zusammensetzung, umfassend beliebige der Peptide von Anspruch 1 oder 2 oder beliebige der Nukleinsäuresequenzen von Anspruch 3.

9. Zusammensetzung nach beliebigen der Ansprüche 6 bis 8, weiterhin umfassend einen pharmazeutisch annehmbaren Träger.

10. Peptid nach beliebigen der Ansprüche 1 oder 2 zur Verwendung bei der Behandlung von Krebs oder hyperproliferativer Störung.

11. Peptid zur Verwendung nach Anspruch 10, wobei der Krebs Brustkrebs, dreifach-negativen Brustkrebs, Eierstockkrebs oder einen beliebigen Blutkrebs umfasst.

12. Peptid zur Verwendung nach Anspruch 10, wobei die hyperproliferative Störung Krebs umfasst, wobei der Krebs Brustkrebs, dreifach-negativen Brustkrebs, Eierstockkrebs oder einen beliebigen Blutkrebs umfasst.

13. Rekombinante Zelle, umfassend beliebige der Nukleinsäuren nach Anspruch 3.

## Revendications

1. Peptide comprenant un peptide p53 partiel et un domaine en superhélice Bcr muté, dans lequel le peptide p53 partiel ne possède pas de domaine de tétramérisation fonctionnel mais conserve une activité de suppresseur de tumeur et le domaine en superhélice Bcr muté comprend des substitutions et des délétions d'acides aminés mais conserve une activité de tétramérisation,
où le domaine en superhélice Bcr muté comprend des mutations au niveau des résidus 34 et 55 de SEQ ID n° 4 et possède une lysine au niveau de la position 34 et un acide glutamique au niveau de la position 55 de SEQ ID n° 4 ou comprend la séquence de SEQ ID n° 5, où les domaines en superhélice Bcr mutés comprennent au moins 50% d'identité de séquence avec SEQ ID n° 4.

2. Peptide selon la revendication 1, dans lequel le domaine en superhélice Bcr muté est lié à l'extrémité C-terminale du domaine de fixation à l'ADN du peptide p53 partiel ou est situé entre le domaine de fixation à l'ADN du peptide p53 partiel et l'extrémité C-terminale du peptide.

3. Séquence d'acide nucléique codant pour les peptides selon l'une quelconque des revendications précédentes.

4. Vecteur comprenant l'acide nucléique selon la revendication 3.

5. Vecteur selon la revendication 4, le vecteur étant un vecteur viral, préférablement un vecteur adénoviral.

6. Composition comprenant un peptide selon l'une quelconque des revendications 1 ou 2, pour une utilisation dans l'induction d'une apoptose dans le traitement d'un patient.

7. Composition comprenant un peptide selon l'une quelconque des revendications 1 ou 2, pour une utilisation dans la suppression d'une tumeur chez un patient.

8. Composition comprenant l'un quelconque parmi les peptides selon les revendications 1 ou 2 ou l'une quelconque parmi les séquences d'acide nucléique selon la revendication 3.

9. Composition selon l'une quelconque des revendications 6 à 8, comprenant en outre un véhicule pharmaceutiquement acceptable.

10. Peptide selon l'une quelconque des revendications 1 ou 2, pour une utilisation dans le traitement d'un cancer ou d'un trouble hyperprolifératif.

11. Peptide pour une utilisation selon la revendication 10, le cancer comprenant le cancer du sein, le cancer du sein triple négatif, le cancer de l'ovaire, ou un cancer quelconque du sang.

12. Peptide pour une utilisation selon la revendication 10, le trouble hyperprolifératif comprenant le cancer, le cancer comprenant le cancer du sein, le cancer du sein triple négatif, le cancer de l'ovaire, ou un cancer quelconque du sang.

13. Cellule recombinée, comprenant l'un quelconque parmi les acides nucléiques selon la revendication 3.


### SEQUENCE LISTING

<110> Lim, Carol Okal, Abood
<120> OLIGOMERIZATION DOMAIN OF p53 TO BYPASS THE DOMINANT-NEGATIVE EFFECT OF MUTANT
<130> 21101.0296P1
<150> 61/992,678
   <151> 2014-05-13
<150> 61/936,790
   <151> 2014-02-06
<160> 28
<170> PatentIn version 3.5
<210> 1
   <211> 393
   <212> PRT
   <213> Homo sapiens
<400> 1
<210> 2
   <211> 34
   <212> PRT
   <213> Artificial Sequence
<220>
   <223> synthetic construct; tetramerization domain of p53
<400> 2 Ala Gly
<210> 3
   <211> 396
   <212> PRT
   <213> Artificial Sequence
<220>
   <223> synthetic construct; mutant p53
<400> 3
   Met Glu Glu Pro Gln Ser Asp Pro Ser Val Glu Pro Pro Leu Ser Gln
   1               5                   10                  15
   Glu Thr Phe Ser Asp Leu Trp Lys Leu Leu Pro Glu Asn Asn Val Leu
               20                  25                  30
   Ser Pro Leu Pro Ser Gln Ala Met Asp Asp Leu Met Leu Ser Pro Asp
           35                  40                  45
   Asp Ile Glu Gln Trp Phe Thr Glu Asp Pro Gly Pro Asp Glu Ala Pro
       50                  55                  60
   Arg Met Pro Glu Ala Ala Pro Pro Val Ala Pro Ala Pro Ala Ala Pro
   65                  70                  75                  80
   Thr Pro Ala Ala Pro Ala Pro Ala Pro Ser Trp Pro Leu Ser Ser Ser
                   85                  90                  95
   Val Pro Ser Gln Lys Thr Tyr Gln Gly Ser Tyr Gly Phe Arg Leu Gly
               100                 105                 110
   Phe Leu His Ser Gly Thr Ala Lys Ser Val Thr Cys Thr Tyr Ser Pro
           115                 120                 125
   Ala Leu Asn Lys Met Phe Cys Gln Leu Ala Lys Thr Cys Pro Val Gln
       130                 135                 140
   Leu Trp Val Asp Ser Thr Pro Pro Pro Gly Thr Arg Val Arg Ala Met
   145                 150                 155                 160
   Ala Ile Tyr Lys Gln Ser Gln His Met Thr Glu Val Val Arg Arg Cys
                   165                 170                 175
   Pro His His Glu Arg Cys Ser Asp Ser Asp Gly Leu Ala Pro Pro Gln
               180                 185                 190
   His Leu Ile Arg Val Glu Gly Asn Leu Arg Val Glu Tyr Leu Asp Asp
           195                 200                 205
   Arg Asn Thr Phe Arg His Ser Val Val Val Pro Tyr Glu Pro Pro Glu
       210                 215                 220
   Val Gly Ser Asp Cys Thr Thr Ile His Tyr Asn Tyr Met Cys Asn Ser
   225                 230                 235                 240
   Ser Cys Met Gly Gly Met Asn Arg Arg Pro Ile Leu Thr Ile Ile Thr
                   245                 250                 255
   Leu Glu Asp Ser Ser Gly Asn Leu Leu Gly Arg Asn Ser Phe Glu Val
               260                 265                 270
   Arg Val Cys Ala Cys Pro Gly Arg Asp Arg Arg Thr Glu Glu Glu Asn
           275                 280                 285
   Leu Arg Lys Lys Gly Glu Pro His His Glu Leu Pro Pro Gly Ser Thr
       290                 295                 300
   Lys Arg Ala Leu Pro Asn Asn Thr Ser Ser Ser Pro Gln Pro Lys Lys
   305                 310                 315                 320
   Lys Pro Ser Gly Met Val Asp Pro Val Gly Phe Ala Glu Ala Trp Lys
                   325                 330                 335
   Ala Gln Phe Pro Asp Ser Glu Pro Pro Arg Met Glu Leu Arg Ser Val
               340                 345                 350
   Gly Asp Ile Glu Gln Lys Leu Glu Arg Cys Lys Ala Ser Ile Arg Arg
           355                 360                 365
   Leu Glu Gln Glu Val Asn Gln Glu Arg Phe Glu Met Ile Tyr Leu Gln
   370 375 380
   Thr Leu Leu Ala Lys Glu Lys Lys Ser Tyr Asp Arg
   385 390 395
<210> 4
   <211> 72
   <212> PRT
   <213> Artificial Sequence
<220>
   <223> synthetic construct; coiled-coil domain of Bcr
<400> 4
   Met Val Asp Pro Val Gly Phe Ala Glu Ala Trp Lys Ala Gln Phe Pro
   1 5 10 15
   Asp Ser Glu Pro Pro Arg Met Glu Leu Arg Ser Val Gly Asp Ile Glu
   20 25 30
   Gln Glu Leu Glu Arg Cys Lys Ala Ser Ile Arg Arg Leu Glu Gln Glu
   35 40 45
   Val Asn Gln Glu Arg Phe Arg Met Ile Tyr Leu Gln Thr Leu Leu Ala
   50 55 60
   Lys Glu Lys Lys Ser Tyr Asp Arg
   65 70
<210> 5
   <211> 72
   <212> PRT
   <213> Artificial Sequence
<220>
   <223> synthetic construct; mutated Bcr coiled-coil domain
<400> 5
   Met Val Asp Pro Val Gly Phe Ala Glu Ala Trp Lys Ala Gln Phe Pro
   1 5 10 15
   Asp Ser Glu Pro Pro Arg Met Glu Leu Arg Ser Val Gly Asp Ile Glu
   20 25 30
   Gln Lys Leu Glu Arg Cys Lys Ala Ser Ile Arg Arg Leu Glu Gln Glu
   35 40 45
   Val Asn Gln Glu Arg Phe Glu Met Ile Tyr Leu Gln Thr Leu Leu Ala
   50 55 60
   Lys Glu Lys Lys Ser Tyr Asp Arg
   65                  70
<210> 6
   <211> 1182
   <212> DNA
   <213> Homo sapiens
<400> 6
   atggaggagc cgcagtcaga tcctagcgtc gagccccctc tgagtcagga aacattttca      60
   gacctatgga aactacttcc tgaaaacaac gttctgtccc ccttgccgtc ccaagcaatg     120
   gatgatttga tgctgtcccc ggacgatatt gaacaatggt tcactgaaga cccaggtcca     180
   gatgaagctc ccagaatgcc agaggctgct ccccccgtgg cccctgcacc agcagctcct     240
   acaccggcgg cccctgcacc agccccctcc tggcccctgt catcttctgt cccttcccag     300
   aaaacctacc agggcagcta cggtttccgt ctgggcttct tgcattctgg gacagccaag     360
   tctgtgactt gcacgtactc ccctgccctc aacaagatgt tttgccaact ggccaagacc     420
   tgccctgtgc agctgtgggt tgattccaca cccccgcccg gcacccgcgt ccgcgccatg     480
   gccatctaca agcagtcaca gcacatgacg gaggttgtga ggcgctgccc ccaccatgag     540
   cgctgctcag atagcgatgg tctggcccct cctcagcatc ttatccgagt ggaaggaaat     600
   ttgcgtgtgg agtatttgga tgacagaaac acttttcgac atagtgtggt ggtgccctat     660
   gagccgcctg aggttggctc tgactgtacc accatccact acaactacat gtgtaacagt     720
   tcctgcatgg gcggcatgaa ccggaggccc atcctcacca tcatcacact ggaagactcc     780
   agtggtaatc tactgggacg gaacagcttt gaggtgcgtg tttgtgcctg tcctgggaga     840
   gaccggcgca cagaggaaga gaatctccgc aagaaagggg agcctcacca cgagctgccc     900
   ccagggagca ctaagcgagc actgcccaac aacaccagct cctctcccca gccaaagaag     960
   aaaccactgg atggagaata tttcaccctt cagatccgtg ggcgtgagcg cttcgagatg    1020
   ttccgagagc tgaatgaggc cttggaactc aaggatgccc aggctgggaa ggagccaggg    1080
   gggagcaggg ctcactccag ccacctgaag tccaaaaagg gtcagtctac ctcccgccat    1140
   aaaaaactca tgttcaagac agaagggcct gactcagact ga                       1182
<210> 7
   <211> 102
   <212> DNA
   <213> Artificial Sequence
<220>
   <223> synthetic construct; tetramerization domain of p53
<400> 7
   ctggatggag aatatttcac ccttcagatc cgtgggcgtg agcgcttcga gatgttccga 60
   gagctgaatg aggccttgga actcaaggat gcccaggctg gg 102
<210> 8
   <211> 1188
   <212> DNA
   <213> Artificial Sequence
<220>
   <223> synthetic construct; mutant p53
<400> 8
<210> 9
   <211> 35
   <212> DNA
   <213> Artificial Sequence
<220>
   <223> synthetic construct; primer
<400> 9
   ggagcgctgc aaggcccgct ccattcggcg cctgg 35
<210> 10
   <211> 35
   <212> DNA
   <213> Artificial Sequence
<220>
   <223> synthetic construct; primer
<400> 10
   ccaggcgccg aatggagcgg gccttgcagc gctcc 35
<210> 11
   <211> 35
   <212> DNA
   <213> Artificial Sequence
<220>
   <223> synthetic construct; primer
<400> 11
   tccgcatgat ctacctggag acgttgctgg ccaag 35
<210> 12
   <211> 35
   <212> DNA
   <213> Artificial Sequence
<220>
   <223> synthetic construct; primer
<400> 12
   cttggccagc aacgtctcca ggtagatcat gcgga 35
<210> 13
   <211> 37
   <212> DNA
   <213> Artificial Sequence
<220>
   <223> synthetic construct; primer
<400> 13
   gtgggcgaca tcgagcagaa gctggagcgc tgcaagg 37
<210> 14
   <211> 37
   <212> DNA
   <213> Artificial Sequence
<220>
   <223> synthetic construct; primer
<400> 14
   ccttgcagcg ctccagcttc tgctcgatgt cgcccac 37
<210> 15
   <211> 43
   <212> DNA
   <213> Artificial Sequence
<220>
   <223> synthetic construct; primer
<400> 15
   aggtgaacca ggagcgcttc gagatgatct acctgcagac gtt 43
<210> 16
   <211> 43
   <212> DNA
   <213> Artificial Sequence
<220>
   <223> synthetic construct; primer
<400> 16
   aacgtctgca ggtagatcat ctcgaagcgc tcctggttca cct 43
<210> 17
   <211> 37
   <212> DNA
   <213> Artificial Sequence
<220>
   <223> synthetic construct; primer
<400> 17
   gcctccattc ggcgcctgaa gcaggaggtg aaccagg 37
<210> 18
   <211> 37
   <212> DNA
   <213> Artificial Sequence
<220>
   <223> synthetic construct; primer
<400> 18
   cctggttcac ctcctgcttc aggcgccgaa tggaggc 37
<210> 19
   <211> 40
   <212> DNA
   <213> Artificial Sequence
<220>
   <223> synthetic construct; primer
<400> 19
   agcaggaggt gaaccaggag ttccgcatga tctacctgca 40
<210> 20
   <211> 40
   <212> DNA
   <213> Artificial Sequence
<220>
   <223> synthetic construct; primer
<400> 20
   tgcaggtaga tcatgcggaa ctcctggttc acctcctgct 40
<210> 21
   <211> 41
   <212> DNA
   <213> Artificial Sequence
<220>
   <223> synthetic construct; primer
<400> 21
   gcaggaggtg aaccaggagg agttccgcat gatctacctg c 41
<210> 22
   <211> 41
   <212> DNA
   <213> Artificial Sequence
<220>
   <223> synthetic construct; primer
<400> 22
   gcaggtagat catgcggaac tcctcctggt tcacctcctg c 41
<210> 23
   <211> 38
   <212> DNA
   <213> Artificial Sequence
<220>
   <223> synthetic construct; primer
<400> 23
   tgacggaggt tgtgaggcac tgcccccacc atgagcgc 38
<210> 24
   <211> 38
   <212> DNA
   <213> Artificial Sequence
<220>
   <223> synthetic construct; primer
<400> 24
   gcgctcatgg tgggggcagt gcctcacaac ctccgtca 38
<210> 25
   <211> 38
   <212> DNA
   <213> Artificial Sequence
<220>
   <223> synthetic construct; primer
<400> 25
   ctgcatgggc ggcatgaact ggaggcccat cctcacca 38
<210> 26
   <211> 38
   <212> DNA
   <213> Artificial Sequence
<220>
   <223> synthetic construct; primer
<400> 26
   tggtgaggat gggcctccag ttcatgccgc ccatgcag 38
<210> 27
   <211> 38
   <212> DNA
   <213> Artificial Sequence
<220>
   <223> synthetic construct; primer
<400> 27
   ggaacagctt tgaggtgcat gtttgtgcct gtcctggg 38
<210> 28
   <211> 38
   <212> DNA
   <213> Artificial Sequence
<220>
   <223> synthetic construct; primer
<400> 28
   cccaggacag gcacaaacat gcacctcaaa gctgttcc 38